# EUROPEAN PATENT SPECIFICATION

(11) **EP 4 463 553 B1**
(45) Date of publication and mention of the grant of the patent: **01.10.2025**
(21) Application number: 23855822.5
(22) Date of filing: 18.12.2023
(51) Int. Cl.: C12N 15/113

(54) **ADVANCED RNA TARGETING (ARNATAR)**
FORTGESCHRITTENES RNA-TARGETING (ARNATAR)
CIBLAGE D'ARN AVANCÉ (ARNATAR)

(30) Priority: 19.12.2022 US 202263433706 P; 13.06.2023 US 202363472780 P
(43) Date of publication of application: 20.11.2024
(73) Proprietor: Arnatar Therapeutics, Inc, San Diego, CA 92121 (US)
(72) Inventor: LIANG, Xuehai, San Diego, CA 92121 (US); ZHANG, Lingdi, San Diego, CA 92121 (US); WANG, Yanfeng, San Diego, CA 92121 (US)
(74) Representative: Schiener, Jens
(86) International application number: PCT/US2023/084688
(87) International publication number: WO 2024/137543

(56) References cited:
- WO-A1-2009/002944
- WO-A1-2021/119034
- WO-A1-2022/261005
- WO-A2-2018/027106
- WO-A2-2020/123508
- WO-A2-2021/234459
- FAITH CONROY: "Chemical engineering of therapeutic siRNAs for allele-specific gene silencing in vivo in CNS", BIORXIV, 2 July 2022 (2022-07-02), XP093157137, Retrieved from the Internet <URL:https://www.biorxiv.org/content/10.1101/2022.06.29.498088v1.full.pdf> DOI: 10.1101/2022.06.29.498088
- SHOWKAT AHMAD DAR ET AL: "siRNAmod: A database of experimentally validated chemically modified siRNAs", SCIENTIFIC REPORTS, vol. 6, no. 1, 28 January 2016 (2016-01-28), XP055674735, DOI: 10.1038/srep20031
- DONALD J. FOSTER ET AL: "Advanced siRNA Designs Further Improve In Vivo Performance of GalNAc-siRNA Conjugates", MOLECULAR THERAPY, vol. 26, no. 3, 4 January 2018 (2018-01-04), US, pages 708 - 717, XP055598561, ISSN: 1525-0016, DOI: 10.1016/j.ymthe.2017.12.021

## Description

### CROSS-REFERENCE TO RELATED APPLICATIONS

This PCT application claims the benefit of priority to U.S. Provisional Application Nos. 63/433,706, filed on December 19, 2022, and 63/472,780, filed on June 13, 2023.

### FIELD

Certain embodiments are directed to compounds for modulating gene expression by Advanced RNA Targeting (ARNATAR). Such compounds are useful for reducing expression of certain genes, many of which are associated with a variety of diseases and disorders.

### BACKGROUND

The use of therapeutic oligomeric compounds was first proposed over forty years ago by Stephenson and Zamecnik (Inhibition of Rous Sarcoma Viral RNA Translation by a Specific Oligodeoxyribonucleotide, PNAS, 1978, 75:285-288). However, issues with delivery, stability, specificity, safety, and potency were barriers to therapeutic efficacy and to using oligomeric compounds as therapeutics. Decades have been spent researching the mechanisms underlying the ability of oligomeric compounds to inhibit gene expression, for example, by regulating transcription and translation, in order to enhance the delivery, stability, specificity, safety and potency of oligomeric compounds.

Sequence-specific silencing of gene expression, RNA interference (RNAi), was discovered in 1998 by Fire et al. (Potent and Specific Genetic Interference by Double-Stranded RNA in Caenorhabditis elegans, Nature, 1998, 391:806-811). RNAi inhibits gene expression via the RNA-induced silencing complex (RISC).

RISC comprises a complex of multiple proteins interacting with an oligomeric compound to inhibit gene expression. The oligomeric compound acts as a template for RISC to recognize complementary messenger RNA (mRNA) transcripts to target a specific mRNA transcript for cleavage. Cleavage of the target mRNA blocks translation of the target mRNA and silences the target gene. Oligomeric compounds utilized by RISC include, but, are not limited to: single stranded oligomeric compounds such as microRNAs (miRNAs), certain oligonucleotides, and single strand siRNAs (Lima et al., Single-stranded siRNAs activate RNAi in animals. Cell. 2012, 150(5):883-94), and double stranded oligomeric compounds such as short hairpin RNAs (shRNAs) and small interfering RNAs (siRNAs).

In 2001, Elbashir et al., showed that 21-nucleotide long siRNA duplexes specifically suppress expression of endogenous and heterologous genes in mammalian cell lines and theorized that siRNA may eventually be used as a gene-specific therapeutic (Duplexes of 21-Nucleotide RNAs Mediate RNA Interference in Cultured Mammalian Cells, Nature, 2001, 411:494-498). Currently, five siRNA compounds have received U.S. Food and Drug Administration (FDA) marketing approval (patisiran, givosiran, inclisiran, lumasiran and vutrisiran) while several more are in clinical trials (Moumné et al, Oligonucleotide Therapeutics: From Discovery and Development to Patentability, Pharmaceutics, 2022, 14(2):260).

Patisiran (Onpattro^{™}) became the first FDA approved siRNA therapeutic in 2018 (Hoy, Patisiran: First Global Approval, Drugs, 2018, 78:1625-1631). Patisiran is a partially modified siRNA targeting transthyretin (TTR) for the treatment of peripheral nerve disease (polyneuropathy) caused by hereditary transthyretin-mediated amyloidosis (hATTR), where chemically modified nucleosides are scattered along the 21-nucleotide long sense and antisense strands forming the siRNA duplex. Later approved siRNAs such as givosiran introduced additional modified nucleosides, modified inter-nucleoside linkages and a N-Acetylgalactosamine (GalNAc) conjugate to aid cellular delivery. (Hu et al., Therapeutic siRNA: State of the Art, Signal Transduction and Targeted Therapy, 2020, 5:101). The latest FDA approved siRNA, vutrisiran (Amvuttra^{™}), has the same target and indication as patisiran and was developed by the same company (Alnylam Press Release in Businesswire, Alnylam Announces FDA Approval of Amvuttra^{™} (vitrusiran), an RNAi Therapeutic for the Treatment of the Polyneuropathy of Hereditary Transthyretin-Mediated Amyloidosis in Adults, June 2022). Vutrisiran is a direct competitor to patisiran and was designed with a different sequence, chemical modification pattern and delivery modality to improve its therapeutic efficacy over patisiran.

International application WO 2009/002944 A1 discloses compositions comprising oligomeric compounds that modulate gene expression. In detail, this application provides double stranded compositions wherein the first strand is modified to have a particular motif and the second strand is modified by a selected motif that differs from the claimed invention. The motifs are defined by positioning of differentially modified nucleosides wherein at least the sugar moieties are different.

International application WO 2022/261005 A1 discloses compositions comprising an oligonucleotide that targets Angiopoietin-like 4. Oligonucleotide modification patterns differing from the claimed invention are included that may be used to improve their stability or efficacy.

The field of therapeutic oligomeric compounds has advanced immensely from the discovery of RNAi in 1998, to the first regulatory approval of an siRNA therapeutic in 2018, to the latest improved siRNA therapeutic. However, the field is still constantly seeking improvements to increase therapeutic efficacy even over existing oligomeric compounds. The ideal oligomeric compound should be: 1) specifically deliverable to the cell or organ of interest, 2) stable/durable once administered to a patient e.g., slow to degradation, a long half-life, 3) specific to the target with no off-target effects, 4) safe for the patient with no activation of the immune system and nontoxic, and 5) potent with efficient and specific cleavage of its target.

Improvements in delivery, stability, specificity, safety and potency of oligomeric compounds are still being sought in order to produce a better therapeutic. Disclosed herein are improved oligomeric compounds with Advanced RNA Targeting (ARNATAR) abilities that enhance their gene silencing activity.

### SUMMARY

Several embodiments provided herein relate to the discovery of certain modifications to oligomeric compounds that can enhance their effectiveness in modulating gene expression. In several aspects, the oligomeric compound is double-stranded (e.g., shRNA and siRNA) and is modified. The double-stranded oligomeric compound comprises a sense strand and an antisense strand. The antisense strands are fully or partially complementary to a target nucleic acid.

The invention is defined by the appended claims.

In one embodiment, an oligomeric compound capable of inhibiting the expression of a target nucleic acid, comprises a sense strand having 21 linked nucleotides, wherein the sense strand sequence is represented by Formula (III): 5' M*F*MMN*MN*MFFNMN*MN*MMFM*D*D 3'. The D is a deoxyribonucleoside, M is a 2'-OMe modified nucleoside, N is a modified or unmodified nucleoside, F is a 2'-F modified nucleoside, * is a phosphorothioate (PS) linkage, and no single modification type modifies more than two consecutive nucleotides.

In one embodiment, an oligomeric compound capable of inhibiting the expression of a target nucleic acid, comprises an antisense strand having 21 linked nucleotides, wherein the antisense strand sequence is represented by Formula (VI): 5' L-M*N*MNMFNMFMMNMFMFMMN*M*M 3'. The M is a 2'-OMe modified nucleoside, N is a modified or unmodified nucleoside, F is a 2'-F modified nucleoside, * is a phosphorothioate (PS) linkage, L is a 5' OH, 5' vinyl phosphonate or a 5' phosphate (p), and no single modification type modifies more than two consecutive nucleotides.

In one embodiment, an oligomeric compound capable of inhibiting the expression of a target nucleic acid, comprises: (a) a sense strand having 21 linked nucleotides, wherein the sense strand sequence is represented by Formula (III): 5' M*F*MMN*MN*MFFNMN*MN*MMFM*D*D 3', and (b) antisense strand having 21 linked nucleotides, wherein the antisense strand sequence is represented by Formula (VI): 5' L-M*N*MNMFNMFMMNMFMFMMN*M*M 3'. A duplex is formed by the sense and antisense strands, where the duplex region is 19 nucleotide pairs in length and each strand has a 2 nucleotide overhang at the 3' end. The D is a deoxyribonucleoside, M is a 2'-OMe modified nucleoside, N is a modified or unmodified nucleoside, F is a 2'-F modified nucleoside, * is a phosphorothioate (PS) linkage, L is a 5' OH, 5' vinyl phosphonate or a 5' phosphate (p), and no single modification type modifies more than two consecutive nucleotides.

In one embodiment, an oligomeric compound capable of inhibiting the expression of a target nucleic acid, comprises an antisense strand having 21 linked nucleotides, wherein the antisense strand sequence is represented by Formula (VII): 5' L-M*D*MFMFNMFMMFMFMFMMN*M*M 3'. The D is a deoxyribonucleoside, M is a 2'-OMe modified nucleoside, N is a modified or unmodified nucleoside, F is a 2'-F modified nucleoside, * is a phosphorothioate (PS) linkage, L is a 5' OH, 5' vinyl phosphonate or a 5' phosphate (p), and no single modification type modifies more than two consecutive nucleotides.

According to the invention, an oligomeric compound capable of inhibiting the expression of a target nucleic acid, comprises a sense strand having 21 linked nucleotides, wherein the sense strand sequence is represented by Formula (X): 5' MFMMNMNMFFNMNMNMMNMDD 3'. The D is a deoxyribonucleoside, M is a 2'-OMe modified nucleoside, N is a modified or unmodified nucleoside, F is a 2'-F modified nucleoside, and no single modification type modifies more than two consecutive nucleotides.

According to the invention, the oligomeric compound capable of inhibiting the expression of a target nucleic acid, comprises an antisense strand having 21 linked nucleotides, wherein the antisense strand sequence is represented by Formula (VIII): 5' L-MNMNMFNMFMMNMFMFMMNMM 3'. The M is a 2'-OMe modified nucleoside, N is a modified or unmodified nucleoside, F is a 2'-F modified nucleoside, L is a 5' OH, 5' vinyl phosphonate or a 5' phosphate (p), and no single modification type modifies more than two consecutive nucleotides.

In one embodiment, a duplex is formed by the sense and antisense strands, where the duplex region is 19 nucleotide pairs in length and each strand has a 2 nucleotide overhang at the 3' end.

In one embodiment, an oligomeric compound capable of inhibiting the expression of a target nucleic acid, comprises a sense strand having 21 linked nucleotides, wherein the sense strand sequence is represented by Formula (XI): 5' MFMMNMNMFFMMNMNMMFMDD 3'. The D is a deoxyribonucleoside, M is a 2'-OMe modified nucleoside, N is a modified or unmodified nucleoside, F is a 2'-F modified nucleoside, and no single modification type modifies more than two consecutive nucleotides.

In one embodiment, an oligomeric compound capable of inhibiting the expression of a target nucleic acid, comprises an antisense strand having 21 linked nucleotides, wherein the antisense strand sequence is represented by Formula (XII): 5' L-MDMFMFNMFMMFMFMFMMNMM 3'. The M is a 2'-OMe modified nucleoside, N is a modified or unmodified nucleoside, F is a 2'-F modified nucleoside, L is a 5' OH, 5' vinyl phosphonate or a 5' phosphate (p), and no single modification type modifies more than two consecutive nucleotides.

In one embodiment, an oligomeric compound capable of inhibiting the expression of a target nucleic acid, comprises: (a) a sense strand having 21 linked nucleotides, wherein the sense strand sequence is represented by Formula (XI): 5' MFMMNMNMFFMMNMNMMFMDD 3', and (b) antisense strand having 21 linked nucleotides, wherein the antisense strand sequence is represented by Formula (XII): 5' L-MDMFMFNMFMMFMFMFMMNMM 3'. A duplex is formed by the sense and antisense strands, where the duplex region is 19 nucleotide pairs in length and each strand has a 2 nucleotide overhang at the 3' end. The D is a deoxyribonucleoside, M is a 2'-OMe modified nucleoside, N is a modified or unmodified nucleoside, F is a 2'-F modified nucleoside, L is a 5' OH, 5' vinyl phosphonate or a 5' phosphate (p), and no single modification type modifies more than two consecutive nucleotides.

### BRIEF DESCRIPTION OF THE DRAWINGS

**FIGURE 1****:** shows a table with LMNA antisense strand sequences with various modifications.
**FIGURE 2****:** shows a table with LMNA sense strand sequences with various modifications.
**FIGURE 3****:** shows a table with ApoC3 antisense strand sequences with various modifications.
**FIGURE 4****:** shows a table with ApoC3 sense strand sequences with various modifications.
**FIGURE 5****:** shows tables with NCL antisense strand and sense strand sequences with various modifications.
**FIGURE 6****:** shows an assay where siRNAs were incubated with human serum to determine their stability in serum. LMNA-si2, LMNA-si29 and LMNA-si33 were incubated in human serum for various lengths of time then run on a gel to visualize the amount of siRNA duplex present after exposure to serum.
**FIGURE 7****:** shows a tritosome stability assay where siRNAs are assessed for their stability. LMNA-si2, LMNA-si31, LMNA-si47, LMNA-si49 and LMNA-si51 were tested for various lengths of time then run on a gel to visualize the amount of siRNA duplex present after exposure to tritosomes.
**FIGURE 8****:** shows an assay where siRNAs were incubated with human serum to determine their stability in serum. LMNA-si2, LMNA-si31, LMNA-si47, LMNA-si49 and LMNA-si51 were incubated in human serum for various lengths of time then run on a gel to visualize the amount of siRNA duplex present after exposure to serum.
**FIGURE 9****:** shows select ARNATAR design siRNAs assessed for onset of siRNA activity. ARNATAR Design siRNA compounds showed faster onset of siRNA activity compared to benchmark.
**FIGURE 10****:** shows the Melting Temperature (Tm) of select ARNATAR designed siRNAs.
**FIGURE 11****:** shows select ARNATAR design siRNAs assessed for RISC loading. ARNATAR designed siRNAs showed faster association with Ago2.
**FIGURE 12****:** shows RNA inhibition data from an *in vivo* mouse study with select ARNATAR designed siRNAs.
**FIGURE 13****:** shows safety data from an *in vivo* mouse study with select ARNATAR designed siRNAs. Serum proteins ALB, ALT and BUN were assessed.
**FIGURE 14****:** shows safety data from an *in vivo* mouse study with select ARNATAR designed siRNAs. Liver and spleen weight were measured.
**FIGURE 15****:** shows safety data from an *in vivo* mouse study with select ARNATAR designed siRNAs. Serum analytes were assessed.
**FIGURE 16****:** shows data from an *in vivo* mouse study on the effect of select ARNATAR designed siRNAs on immunogenicity markers NFkB, IL-6 and TNF.
**FIGURE 17****:** shows RNA inhibition at Day 7 from an *in vivo* mouse study with select ARNATAR designed siRNAs.
**FIGURE 18****:** shows data at Day 7 from an *in vivo* mouse study on the effect of select ARNATAR designed siRNAs on immunogenicity markers NFkB, IL-6 and TNF.
**FIGURE 19****:** shows select siRNAs with ARNATAR platform chemistry are stable in serum and in tritosome assays.
**FIGURE 20****:** shows *in vitro* inhibition of LMNA in HeLa cells by select ARNATAR designed siRNAs.
**FIGURE 21****:** shows data from an *in vitro* study in HeLa cells on the effect of select ARNATAR designed siRNAs on immunogenicity marker NFkB.
**FIGURE 22****:** shows *in vitro* inhibition of LMNA in HEK293 cells by select ARNATAR designed siRNAs.
**FIGURE 23****:** shows mRNA inhibition data from an *in vivo* Balb/c mouse study with select ARNATAR designed siRNAs.
**FIGURE 24****:** shows a table with HAO1 antisense and sense strand sequences with various modifications.
**FIGURE 25****:** shows a graph of *in vitro* inhibition of HAO1 RNA in Hep3B cells at 24 hours by modified oligomeric compounds.
**FIGURE 26****:** shows graphs of *in vitro* inhibition of LMNA RNA in HeLa cells at 72 hours and 96 hours by modified oligomeric compounds.
**FIGURE 27****:** shows *in vivo* inhibition data of LMNA RNA in mice liver at 3 days, 1 week, or 2 weeks after treatment by modified oligomeric compounds.
**FIGURE 28****:** shows a graph of *in vitro* inhibition of LMNA RNA in HeLa cells at 20 hours by modified oligomeric compounds.
**FIGURE 29****:** shows graphs of *in vitro* inhibition of LMNA RNA in HeLa cells at 16 hours, 24 hours and 48 hours by modified oligomeric compounds.
**FIGURE 30****:** shows graphs of *in vitro* inhibition of LMNA RNA in Hepa1-6 cells at 16 hours and 48 hours by modified oligomeric compounds.
**FIGURE 31****:** shows a graph of *in vitro* inhibition of LMNA RNA in HeLa cells at 20 hours by modified oligomeric compounds.
**FIGURE 32****:** shows *in vivo* data of LMNA siRNA activity in mice liver at 10 days and 25 days after treatment by modified oligomeric compounds.
**FIGURE 33****:** shows graphs of *in vitro* inhibition by ARNATAR motif siRNAs compared to third party motifs applied to siRNAs targeting LMNA, NCL and ApoC3.
**FIGURE 34****:** shows graphs of *in vitro* inhibition of LMNA RNA in HeLa cells at 16 hours and 36 hours by modified oligomeric compounds.
**FIGURE 35****:** shows a graph of *in vitro* inhibition of NCL RNA in HeLa cells at 36 hours by modified oligomeric compounds.
**FIGURE 36****:** shows tables with AGT antisense strand sequences with various modifications.
**FIGURE 37****:** shows tables with AGT sense strand sequences with various modifications.
**FIGURE 38****:** shows graphs of *in vitro* inhibition of AGT RNA in Hep3B cells via transfection at 36 hours or in human primary hepatocytes (HPH) via free uptake at 48 hours by modified oligomeric compounds. The x-axis is siRNA concentration at nM for Hep3B cells and at µM for HPH cells. The y-axis is AGT mRNA percentage after treatment with varying siRNA concentrations.
**FIGURE 39****:** shows graphs of *in vitro* inhibition of AGT RNA in Hep3B cells via transfection at 20 hours. The x-axis is siRNA concentration at nM.
**FIGURE 40****:** shows graphs of AGT *in vitro* inhibition in human primary hepatocytes (HPH) via free uptake at 42 hours by modified oligomeric compounds. The x-axis is siRNA concentration at various µM concentrations. The y-axis is AGT mRNA percentage after treatment with varying siRNA concentrations.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-O(CH₂)₂-OCH₃) refers to an O-methoxy-ethyl modification at the 2' position of a furanose ring. A 2'-O-methoxyethyl modified sugar is a modified sugar.

"2'-MOE nucleoside" (also 2'-O-methoxyethyl nucleoside) means a nucleoside comprising a 2'-MOE modified sugar moiety. "2'-MOE nucleotide" (also 2'-O-methoxyethyl nucleotide) means a nucleotide comprising a 2'-MOE modified sugar moiety.

"2'-O-methyl" (also 2'-OCH₃ and 2'-OMe) refers to an O-methyl modification at the 2' position of a furanose ring. A 2'-O-methyl modified sugar is a modified sugar.

"2'-OMe nucleoside" (also 2'-O-methyl nucleoside) means a nucleoside comprising a 2'-OMe modified sugar moiety. "2'-OMe nucleotide" (also 2'-O-methyl nucleotide) means a nucleotide comprising a 2'-OMe modified sugar moiety.

"2'-substituted nucleoside" means a nucleoside comprising a substituent at the 2'-position of the furanosyl ring other than H or OH. In certain embodiments, 2' substituted nucleosides include nucleosides with a fluoro (2'-F), O-methyl (2'-OMe), O-methoxyethyl (2'-MOE) or bicyclic sugar modifications.

"5-methylcytosine" means a cytosine modified with a methyl group attached to the 5 position. A 5-methylcytosine is a modified nucleobase.

"About" means within ±7% of a value. For example, if it is stated, "the compounds affected at least about 70% inhibition of mRNA", it is implied that the mRNA levels are inhibited within a range of 63% and 77%.

"Animal" refers to a human or non-human animal, including, but not limited to, mice, rats, rabbits, dogs, cats, pigs, and non-human primates, including, but not limited to, monkeys and chimpanzees.

"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, F_{ab} region, and F_{c} region.

"Antisense oligonucleotide" or "ASO" means a single-stranded oligonucleotide having a nucleobase sequence that permits hybridization to a corresponding region or segment of a target nucleic acid. In certain embodiments, the antisense oligonucleotide comprises one or more ribonucleosides (RNA nucleosides) and/or deoxyribonucleosides (DNA nucleosides).

"Base complementarity" refers to the capacity for the base pairing of nucleobases of an oligonucleotide with corresponding nucleobases in a target nucleic acid (i.e., hybridization), and is mediated by Watson-Crick, Hoogsteen or reversed Hoogsteen hydrogen binding between corresponding nucleobases. Base complementarity also refers to canonical (e.g., A:U, A:T, or C:G) or non-canonical base pairings (e.g., A:G, A:U, G:U, I:U, I:A, or I:C).

"Bicyclic sugar" means a furanose ring modified by the bridging of two non-geminal carbon₌atoms. A bicyclic sugar is a modified sugar.

"Cap structure" or "terminal cap moiety" means chemical modifications, which have been incorporated at either terminus of an oligomeric compound.

"Chemical modification" means modification of molecular structure or element from naturally occurred molecules. For example, siRNA compounds are composed of linked ribonucleosides (also sometimes referred to herein as RNA), therefore, substitution of a

deoxyribonucleoside (also sometimes referred to herein as DNA nucleoside) for a ribonucleoside is considered a chemical modification of the siRNA compound.

"Chemically distinct region" refers to a region of an oligomeric compound that is in some way chemically different than another region of the same oligomeric compound. For example, a region having 2'-OMe nucleotides is chemically distinct from a region having nucleotides without 2'-OMe modifications.

"Chimeric oligomeric compounds" means oligomeric compounds that have at least 2 chemically distinct regions, each position having a plurality of subunits. For example, as disclosed herein, siRNA can comprise a peripheral region and a central region. The peripheral region comprises motifs with various modified or unmodified nucleobases so as to confer increased stability, specificity, safety and potency, while the central region comprises various modified or unmodified nucleobases to serve as substrate for RISC mediated degradation.

"Complementarity" means the capacity for pairing between nucleobases of a first nucleic acid and a second nucleic acid.

"Comply" means the adherence with a recommended therapy by an individual.

"Comprise," "comprises" and "comprising" will be understood to imply the inclusion of a stated step or element or group of steps or elements but not the exclusion of any other step or element or group of steps or elements.

"Contiguous nucleobases" means nucleobases immediately adjacent to each other.

"Deoxyribonucleoside" means a nucleoside having a hydrogen at the 2' position of the sugar portion of the nucleoside. A deoxyribonucleoside is sometimes referred to as DNA nucleoside, "D" or "d" herein. Deoxyribonucleosides may be modified with any of a variety of substituents and may be connected by covalent linkages other than naturally occurring phosphodiester such as phosphorothioate.

"Deoxyribonucleotide" means a nucleotide having a hydrogen at the 2' position of the sugar portion of the nucleotide. A deoxyribonucleotide is sometimes referred to as DNA nucleotide, "D" or "d" herein. Deoxyribonucleotides may be modified with any of a variety of substituents and may be connected by covalent linkages other than naturally occurring phosphodiester such as phosphorothioate.

"Designing" or "Design" refers to the process of designing an oligomeric compound that specifically hybridizes with a target nucleic acid molecule.

"Efficacy" means the ability to produce a desired effect.

"Expression" includes all the functions by which a gene's coded information is converted into structures present and operating in a cell. Such structures include, but are not limited to the products of transcription and translation.

"Fully complementary" or "100% complementary" means each nucleobase of a first nucleic acid has a complementary nucleobase in a second nucleic acid. In certain embodiments, a first nucleic acid is an oligomeric compound and a target nucleic acid is a second nucleic acid.

"Fully modified motif" refers to an oligomeric compound comprising a contiguous sequence of nucleosides wherein essentially each nucleoside has a chemical modification.

"Hybridization" means the annealing of complementary nucleic acid molecules. In certain embodiments, complementary nucleic acid molecules include, but are not limited to, an oligomeric compound and a nucleic acid target. In certain embodiments, complementary nucleic acid molecules include, but are not limited to, a siRNA and a nucleic acid target.

"Immediately adjacent" means there are no intervening elements between the immediately adjacent elements.

"Individual" means a human or non-human animal selected for treatment or therapy.

"Induce", "inhibit", "potentiate", "elevate", "increase", "decrease" or the like, generally denotes quantitative differences between two states.

"Inhibiting the expression or activity" refers to a reduction, blockade of the expression or activity and does not necessarily indicate a total elimination of expression or activity.

"Internucleoside linkage" refers to the chemical bond between nucleosides. The 3' position of a nucleoside is hereby linked to a 5' position of a subsequent nucleoside via the internucleoside linkage.

"Linked nucleosides" means adjacent nucleosides (e.g., A, G, C, T, or U) linked together by an internucleoside linkage. Examples of linked nucleosides include deoxyribonucleosides (sometimes referred to as DNA nucleosides herein) or ribonucleosides (sometimes referred to as RNA nucleosides herein).

"Mismatch" or "non-complementary nucleobase" refers to the case when a nucleobase of a first nucleic acid is not capable of pairing with the corresponding nucleobase of a second or target nucleic acid through Watson-Crick base-pairing (e.g., A:T, A:U, or C:G).

"Modified internucleoside linkage" refers to a substitution or any change from a naturally occurring internucleoside bond (i.e., a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C) and uracil (U).

"Modified nucleoside" means a nucleoside having, independently, a modified sugar moiety and/or modified nucleobase. As used herein, where the oligomeric compound is RNA based, a substitution of a deoxyribonucleoside (sometimes referred to as DNA nucleoside herein) for a ribonucleoside is considered a modification of the oligomeric compound. Also, where the oligomeric compound is DNA based, a substitution of a ribonucleoside (sometimes referred to as RNA nucleoside herein) for a deoxyribonucleoside is considered a modification of the oligomeric compound.

"Modified nucleotide" means a nucleotide having, independently, a modified sugar moiety, modified internucleoside linkage, a deoxyribonucleoside (sometimes referred to as DNA nucleoside herein) for a ribonucleoside (sometimes referred to as RNA nucleoside herein) substitution, and/or a modified nucleobase.

"Modified oligonucleotide" means an oligonucleotide comprising at least one modified internucleoside linkage, a modified sugar, a deoxyribonucleoside (sometimes referred to as DNA nucleoside herein) for a ribonucleoside (sometimes referred to as RNA nucleoside herein) substitution, and/or a modified nucleobase.

"Modified sugar" means substitution and/or any change from a natural sugar moiety.

"Moiety" means one of the portions into which something is divided i.e., a part or component of something. For example, a sugar moiety of a nucleotide is the sugar component of the nucleotide.

"Monomer" refers to a single unit of an oligomer. Monomers include, but are not limited to, nucleosides and nucleotides, whether naturally occuring or modified.

"Motif" means a pattern of modification in an oligomeric compound. For example, as disclosed herein, ARNATAR designed oligomeric compounds comprising motifs with various modified nucleobases and internucleoside linkages in order to improve delivery, stability, specificity, safety and potency of the compounds. The motif is independent from the nucleobase contained and identifies only the pattern of modifications.

"Natural sugar moiety" means a sugar moiety found in DNA (2'-H) or RNA (2'-OH).

"Naturally occurring internucleoside linkage" means a 3' to 5' phosphodiester linkage.

"Non-complementary nucleobase" refers to a pair of nucleobases that do not form hydrogen bonds with one another or otherwise support hybridization.

"Nucleic acid" refers to molecules composed of monomeric nucleotides. A nucleic acid includes, but is not limited to, ribonucleic acids (RNA), messenger RNA (mRNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase complementarity" refers to a nucleobase that is capable of base pairing (also known as being complementary) with another nucleobase. If a nucleobase at a certain position of an oligomeric compound is capable of hydrogen bonding with a nucleobase at a certain position of a target nucleic acid, then the position of hydrogen bonding between the oligomeric compound and the target nucleic acid is considered to be complementary at that nucleobase pair. For example, in DNA, adenine (A) is complementary to thymine (T); in RNA, adenine (A) is complementary to uracil (U); and, Guanine (G) is complementary to cytosine (C) in both DNA and RNA. Base pairs, or complementary nucleobases, are usually canonical Watson-Crick base pairs (C:G, A:U, A:T), but, non-canonical base pairs such as Hoogsteen base pairs (e.g., A:G, or A:U), Wobble base pairs (e.g., G:U, I:U, I:A, or I:C, wherein I is hypoxanthine) and the like are also included. Nucleobase complementarity facilitates hybridization of the oligomeric compounds described herein to their target nucleic acids.

"Nucleobase sequence" means the order of contiguous nucleobases independent of any sugar, linkage, and/or nucleobase modification.

"Nucleoside" means a nucleobase linked to a sugar.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo or tricyclo sugar mimetics, e.g., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/ or internucleoside linkage. Generally, a mimetic is used in place of the sugar or sugar-internucleoside linkage combination, and the nucleobase is maintained for hybridization to a selected target.

"Nucleotide" means a nucleoside having a linkage group (e.g., a phosphate (p) or phosphorothioate (PS) group) covalently linked to the sugar portion of the nucleoside. Nucleotides include ribonucleotides and deoxyribonucleotides. Ribonucleotides are the linked nucleotide units forming RNA. Deoxyribonucleotides are the linked nucleotide units forming DNA.

"Off-target effect" refers to an unwanted or deleterious biological effect associated with modulation of RNA or protein expression of a gene other than the intended target nucleic acid.

"Oligomeric activity" means any detectable or measurable activity attributable to the hybridization of an oligomeric compound to its target nucleic acid. In certain embodiments, oligomeric activity is a decrease in the amount or expression of a target nucleic acid or protein encoded by such target nucleic acid. Oligomeric activity can be modulated by an oligomeric compound such as a siRNA.

"Oligomeric compound" means a sequence of linked monomeric subunits that is capable of undergoing hybridization to at least a region of a target nucleic acid through hydrogen bonding. The monomeric subunits can be modified or unmodified nucleotides or nucleosides. The oligomeric compound acts as a template for RISC to recognize complementary messenger RNA (mRNA) transcripts to target a specific mRNA transcript for cleavage. Cleavage of the target mRNA blocks translation of the target mRNA and silences the target gene. Examples of oligomeric compounds include single-stranded and double-stranded compounds, such as, antisense oligonucleotides, ssRNAs, siRNAs, shRNAs and miRNAs. According to the invention, the oligomeric compound is double-stranded.

"Oligomeric inhibition" means reduction of target nucleic acid levels in the presence of an oligomeric compound complementary to a target nucleic acid compared to target nucleic acid levels in the absence of the oligomeric compound.

"Oligomeric mechanisms" include RISC or RNase H related mechanisms involving hybridization of an oligomeric compound with target nucleic acid, wherein the outcome or effect of the hybridization is target degradation and inhibition of gene expression.

"Oligonucleotide" as used herein means a polymer of linked nucleosides each of which can be modified or unmodified, independent one from another. Oligonucleotides can have a linking group other than a phosphate group (e.g., a phosphorothioate = thiophosphate group) used as a linking moiety between nucleosides. In certain embodiments, an oligonucleotide comprises one or more ribonucleosides (RNA nucleosides) and/or deoxyribonucleosides (DNA nucleosides).

"Phosphorothioate linkage" or "PS" means a linkage between nucleosides where the phosphodiester bond is modified by replacing one of the non-bridging oxygen atoms with a sulfur atom. A phosphorothioate (= thiophosphate or also known as thiophosphate) linkage is a modified internucleoside linkage.

"Portion" means a defined number of contiguous (i.e., linked) nucleobases of a nucleic acid. In certain embodiments, a portion is a defined number of contiguous nucleobases of a target nucleic acid. In certain embodiments, a portion is a defined number of contiguous nucleobases of an oligomeric compound

"Region" is defined as a portion of the target nucleic acid having at least one identifiable structure, function, or characteristic.

"RNA" or "ribonucleic acid" consists of ribose nucleotides or ribonucleotides (nitrogenous bases attached to a ribose sugar) linked by phosphodiester bonds, forming strands of varying lengths. The nitrogenous bases in RNA are adenine, guanine, cytosine, and uracil. The ribose sugar of RNA is a cyclical structure of five carbons and one oxygen.

"Ribonucleoside" means a nucleoside having a hydroxy at the 2' position of the sugar portion of the nucleoside. Ribonucleosides can be modified with any of a variety of substituents and may be connected by covalent linkages other than naturally occurring phosphodiester such as phosphorothioate. A ribonucleoside is sometimes referred to as RNA nucleoside, "R" or "r" herein.

"Ribonucleotide" means a nucleotide having a hydroxy at the 2' position of the sugar portion of the nucleotide. Ribonucleotides can be modified with any of a variety of substituents and may be connected by covalent linkages other than naturally occurring phosphodiester such as phosphorothioate. A ribonucleotide is sometimes referred to as RNA nucleotide, "R" or "r" herein.

"Segments" are defined as smaller or sub-portions of regions within a target nucleic acid.

"Sites," as used herein, are defined as unique nucleobase positions within a target nucleic acid.

"Specifically hybridizable" refers to an oligomeric compound having a sufficient degree of complementarity between an oligomeric compound (e.g., siRNA) and a target nucleic acid to induce a desired effect, while exhibiting minimal or no effects on non-target nucleic acids under conditions in which specific binding is desired, i.e., under physiological conditions in the case of *in vivo* assays and therapeutic treatments.

"Stringent hybridization conditions" or "stringent conditions" refer to conditions under which an oligomeric compound will hybridize to its target sequence, but to a minimal number of other sequences.

"Subject" means a human or non-human animal selected for treatment or therapy.

"Target" refers to a protein or nucleic acid sequence (e.g., mRNA), the modulation of which is desired.

"Target gene" refers to a gene encoding a target.

"Targeting" means the process of design and selection of an oligomeric compound that will specifically hybridize to a target nucleic acid and induce a desired effect.

"Target nucleic acid," "target RNA," "target RNA transcript" and "nucleic acid target" all mean a nucleic acid capable of being targeted by oligomeric compounds.

"Target region" means a portion of a target nucleic acid to which one or more oligomeric compounds is targeted.

"Target segment" means the sequence of nucleotides of a target nucleic acid to which an oligomeric compound is targeted. "5' target site" refers to the 5'-most nucleotide of a target segment. "3' target site" refers to the 3'-most nucleotide of a target segment. **In** an embodiment, a target segment is at least a 12-nucleobase portion (i.e., at least 12 consecutive nucleobases) of a target region to which an oligomeric compound is targeted.

"Therapeutic efficacy" refers to the effectiveness of a therapeutic compound such as an oligomeric compound. Therapeutic efficacy can be increased by improvements in delivery, stability, specificity, safety and potency of the therapeutic compound.

"Unmodified" RNA nucleobases mean the purine bases adenine (A) and guanine (G), and the pyrimidine bases cytosine (C) and uracil (U). "Unmodified" DNA nucleobases mean the purine bases adenine (A) and guanine (G), and the pyrimidine bases thymine (T) and cytosine (C). In certain embodiments, an unmodified RNA nucleobase is considered modified when a DNA nucleobase is substituted for the RNA nucleobase. In certain embodiments, an unmodified DNA nucleobase is considered modified when a RNA nucleobase is substituted for the DNA nucleobase.

"Unmodified nucleoside" means a nucleoside composed of commonly and naturally occuring nucleobases and sugar moieties. For example, an unmodified nucleoside is an RNA nucleoside if used in an RNA sequence, however, in such RNA sequence, any other nucleoside (e.g., DNA nucleoside, 2'-OMe, or 2'-F) is considered a modified nucleoside.

"Unmodified nucleotide" means a nucleotide composed of commonly and naturally occuring nucleobases, sugar moieties, and internucleoside linkages. For example, an unmodified nucleotide is an RNA nucleotide if used in an RNA sequence, however, in such RNA sequence, any other nucleotide (e.g., DNA nucleotide, 2'-OMe, or 2'-F) is considered a modified nucleotide.

Disclosed herein are improved oligomeric compounds with Advanced RNA Targeting (ARNATAR) designs that enhance their gene silencing activity. Several embodiments provided herein relate to the discovery of certain modification motifs to oligomeric compounds that can enhance their effectiveness in modulating gene expression by improving delivery, stability, specificity, safety and potency of the oligomeric compounds.

The invention is defined by the appended claims.

The oligomeric compound is double-stranded (e.g., a shRNA or a siRNA) and is modified. The double-stranded oligomeric compound comprises a sense strand and an antisense strand. The antisense strands can be fully or partially complementary to a target nucleic acid.

The following embodiments describe oligomeric compounds comprising linked nucleotides that comprise modified and optionally unmodified nucleosides, wherein adjacent nucleosides are either linked by the naturally occurring phosphodiester bond or by a non naturally occurring bond, such as a thiophosphodiester bond (also referred to a "phosphorothioate internucleotide (PS) linkage"). In some embodiments, where the oligomeric compound is RNA based (e.g., shRNA and/or siRNA), the sequence is considered an RNA and unmodified nucleosides refer to ribonucleosides. Modified nucleosides may comprise a modified base and/or a modified sugar (e.g., preferably at 2' position modified sugar). In the RNA based oligomeric compounds, a deoxyribonucleoside (D) is also considered a modified nucleoside.

In certain embodiments, the modifications on the nucleotides comprise modified sugar moieties. In certain embodiments, the modifications on the nucleotides are selected from the group consisting of a deoxyribonucleoside (also referred to herein as a DNA nucleoside) substituted for a ribonucleoside (also referred to herein as a RNA nucleoside), LNA, 2'-OMe, 2'-F, 2'-MOE, UNA, pseudouridine, 2'-thiouridine, N6'-methyladenosine, and 5'-methylcytidine, 5'-fluoro-2'-deoxyuridine, N-ethylpiperidine 5' triazole-modified adenosine, 5'-nitroindole, 2',4'-difluorotolylribonucleoside, N-ethylpiperidine 7'-EAA triazole-modified adenosine, 6'-phenylpyrrolocytosine and combinations thereof. In certain embodiments, the "N" designation in a formula is a modified or unmodified nucleoside wherein the modifications can be selected from the group consisting of a deoxyribonucleoside (also referred to herein as a DNA nucleoside) substituted for a ribonucleoside (also referred to herein as a RNA nucleoside), LNA, 2'-OMe, 2'-F, 2'-MOE, UNA, pseudouridine, 2'-thiouridine, N6'-methyladenosine, and 5'-methylcytidine, 5'-fluoro-2'-deoxyuridine, N-ethylpiperidine 5' triazole-modified adenosine, 5'-nitroindole, 2',4'-difluorotolylribonucleoside, N-ethylpiperidine 7'-EAA triazole-modified adenosine, 6'-phenylpyrrolocytosine. The modifications can also be selected from base substitutions described hereinbelow.

In certain embodiments, a FFNM motif occurs at or near a cleavage site of the sense strand and/or a (FMM)r motif occurs at or near a cleavage site of the antisense strand, where N is a modified or unmodified nucleoside (e.g., M, F, R, D, UNA, or LNA), F is a 2'-Fluoro, M is a 2'-OMe and r is 1-2. In certain embodiments, the FFNM is FFMM or FFRM, where R is a ribonucleoside.

In one embodiment, an oligomeric compound capable of inhibiting the expression of a target nucleic acid comprises a sense strand having 21 linked nucleotides, wherein the sense strand sequence is represented by Formula (III): 5' M*F*MMN*MN*MFFNMN*MN*MMFM*D*D 3'. The D is a deoxyribonucleoside, M is a 2'-OMe modified nucleoside, N is a modified or unmodified nucleoside (e.g., M, F, R, D, UNA, or LNA), F is a 2'-F modified nucleoside, * is a phosphorothioate (PS) linkage, and no single modification type modifies more than two consecutive nucleotides. In an embodiment, the oligomeric compound comprising Formula (III) is a siRNA. In certain embodiments, the FFNM is FFMM or FFRM, where R is a ribonucleoside. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid comprises an antisense strand having 21 linked nucleotides, wherein the antisense strand sequence is represented by Formula (VI): 5' L-M*N*MNMFNMFMMNMFMFMMN*M*M 3'. The M is a 2'-OMe modified nucleoside, N is a modified or unmodified nucleoside (e.g., M, F, R, D, UNA, or LNA), F is a 2'-F modified nucleoside, * is a phosphorothioate (PS) linkage, L is a 5' phosphate, 5' vinyl phosphonate or 5' OH, and no single modification type modifies more than two consecutive nucleotides. In an embodiment, the oligomeric compound comprising Formula (VI) is a siRNA. In certain embodiments, the FNM is FMM. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid comprises: (a) a sense strand having 21 linked nucleotides, wherein the sense strand sequence is represented by Formula (III): 5' M*F*MMN*MN*MFFNMN*MN*MMFM*D*D 3', and (b) antisense strand having 21 linked nucleotides, wherein the antisense strand sequence is represented by Formula (VI): 5' L-M*N*MNMFNMFMMNMFMFMMN*M*M 3'. A duplex is formed by the sense and antisense strands, where the duplex region is 19 nucleotide pairs in length and each strand has a 2 nucleotide overhang at the 3' end. The D is a deoxyribonucleoside, M is a 2'-OMe modified nucleoside, N is a modified or unmodified nucleoside (e.g., M, F, R, D, UNA, or LNA), F is a 2'-F modified nucleoside, * is a phosphorothioate (PS) linkage, L is a 5' phosphate, 5' vinyl phosphonate or 5' OH, and no single modification type modifies more than two consecutive nucleotides. In an embodiment, the oligomeric compound comprising Formulas (III) and (VI) is a siRNA. In certain embodiments, the FFNM in Formula (III) is FFMM or FFRM, where R is a ribonucleoside. In certain embodiments, the FNM in Formula (VI) is FMM. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid comprises an antisense strand having 21 linked nucleotides, wherein the antisense strand sequence is represented by Formula (VII): 5' L-M*D*MFMFNMFMMFMFMFMMN*M*M 3'. The D is a deoxyribonucleoside, M is a 2'-OMe modified nucleoside, N is a modified or unmodified nucleoside (e.g., M, F, R, D, UNA, or LNA), F is a 2'-F modified nucleoside, * is a phosphorothioate (PS) linkage, L is a 5' OH, 5' vinyl phosphonate or a 5' phosphate (p), and no single modification type modifies more than two consecutive nucleotides. In an embodiment, the oligomeric compound comprising Formula (VII) is a siRNA. In certain embodiments, the FNM is FMM. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid comprises: (a) a sense strand having 21 linked nucleotides, wherein the sense strand sequence is represented by Formula (III): 5' M*F*MMN*MN*MFFNMN*MN*MMFM*D*D 3', and (b) antisense strand having 21 linked nucleotides, wherein the antisense strand sequence is represented by Formula (VII): 5' L-M*D*MFMFNMFMMFMFMFMMN*M*M 3'. A duplex is formed by the sense and antisense strands, where the duplex region is 19 nucleotide pairs in length and each strand has a 2 nucleotide overhang at the 3' end. The D is a deoxyribonucleoside, M is a 2'-OMe modified nucleoside, N is a modified or unmodified nucleoside (e.g., M, F, R, D, UNA, or LNA), F is a 2'-F modified nucleoside, * is a phosphorothioate (PS) linkage, L is a 5' phosphate, 5' vinyl phosphonate or 5' OH, and no single modification type modifies more than two consecutive nucleotides. In an embodiment, the oligomeric compound comprising Formulas (III) and (VII) is a siRNA. In certain embodiments, the FFNM in Formula (III) is FFMM or FFRM, where R is a ribonucleoside. In certain embodiments, the FNM in Formula (VII) is FMM. Modified nucleosides may comprise a modified base and/or a modified sugar (preferably at 2' position modified sugar). A deoxyribonucleoside (D) is also considered a modified nucleoside in a RNA based oligomeric compound.

According to the invention, an oligomeric compound capable of inhibiting the expression of a target nucleic acid, comprises a sense strand having 21 linked nucleotides, wherein the sense strand sequence is represented by Formula (X): 5' MFMMNMNMFFNMNMNMMNMDD 3'. The D is a deoxyribonucleoside, M is a 2'-OMe modified nucleoside, N is a modified or unmodified nucleoside (e.g., M, F, R, D, UNA, or LNA), F is a 2'-F modified nucleoside, and no single modification type modifies more than two consecutive nucleotides. In certain embodiments, the sense strand comprises one or more phosphorothioate internucleotide (PS) linkage between 2 nucleosides. In a further embodiment, the sense strand comprises a phosphorothioate internucleotide (PS) linkage adjacent to a deoxyribonucleoside (D) or ribonucleoside (R). The PS linkage can be adjacent to the deoxyribonucleoside (D) or ribonucleoside (R) on the 5' side, the 3' side or both sides. The PS linkage can also be adjacent to 2 nucleosides at the 5' end of the strand and/or 2 nucleosides at the 3' end of the strand. In an embodiment, the oligomeric compound comprising Formula (X) is a siRNA. Modified nucleosides may comprise a modified base and/or a modified sugar (preferably at 2' position modified sugar). A deoxyribonucleoside (D) is also considered a modified nucleoside in a RNA based oligomeric compound.

According to the invention, an oligomeric compound capable of inhibiting the expression of a target nucleic acid, comprises an antisense strand having 21 linked nucleotides, wherein the antisense strand sequence is represented by Formula (VIII): 5' L-MNMNMFNMFMMNMFMFMMNMM 3'. The M is a 2'-OMe modified nucleoside, N is a modified or unmodified nucleoside (e.g., M, F, R, D, UNA, or LNA), F is a 2'-F modified nucleoside, L is a 5' OH, 5' vinyl phosphonate or a 5' phosphate (p), and no single modification type modifies more than two consecutive nucleotides. In certain embodiments, the sense strand comprises one or more phosphorothioate internucleotide (PS) linkage between 2 nucleosides. In a further embodiment, the antisense strand comprises a phosphorothioate internucleotide (PS) linkage adjacent to a deoxyribonucleoside (D) or ribonucleoside (R). The PS linkage can be adjacent to the deoxyribonucleoside (D) or ribonucleoside (R) on the 5' side, the 3' side or both sides. The PS linkage modification can be between positions 1-2, positions 2-3, 19-20 and/or positions 20-21 counting from the 5' end of the antisense strand. The PS linkage can also be adjacent to 2 nucleosides at the 5' end of the strand and/or 2 nucleosides at the 3' end of the strand. In an embodiment, the oligomeric compound comprising Formula (VIII) is a siRNA. Modified nucleosides may comprise a modified base and/or a modified sugar (preferably at 2' position modified sugar). A deoxyribonucleoside (D) is also considered a modified nucleoside in a RNA based oligomeric compound.

In one embodiment, a duplex is formed by the sense and antisense strands, where the duplex region is 19 nucleotide pairs in length and each strand has a 2 nucleotide overhang at the 3' end. In certain embodiments, the sense strand comprises one or more phosphorothioate internucleotide (PS) linkage between 2 nucleosides. In a further embodiment, the strands comprise a phosphorothioate internucleotide (PS) linkage adjacent to deoxyribonucleosides (D) or ribonucleosides (R). The PS linkage can be adjacent to the deoxyribonucleoside (D) or ribonucleoside (R) on the 5' side, the 3' side or both sides. The PS linkage modification on the antisense strand can be between positions 1-2, positions 2-3 and positions 19-20 and/or 20-21 counting from the 5' end of the antisense strand. The PS linkage can also be adjacent to 2 nucleosides at the 5' end of the strands and/or 2 nucleosides at the 3' end of the strands. In an embodiment, the oligomeric compound comprising Formulas (X) and (VIII) is a siRNA. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid, comprises a sense strand having 21 linked nucleotides, wherein the sense strand sequence is represented by Formula (XI): 5' MFMMNMNMFFMMNMNMMFMDD 3'. The D is a deoxyribonucleoside, M is a 2'-OMe modified nucleoside, N is a modified or unmodified nucleoside (e.g., M, F, R, D, UNA, or LNA), F is a 2'-F modified nucleoside, and no single modification type modifies more than two consecutive nucleotides. In certain embodiments, the sense strand comprises one or more phosphorothioate internucleotide (PS) linkage between 2 nucleosides. In a further embodiment, the sense strand comprises a phosphorothioate internucleotide (PS) linkage adjacent to a deoxyribonucleoside (D) or ribonucleoside (R). The PS linkage can be adjacent to the deoxyribonucleoside (D) or ribonucleoside (R) on the 5' side, the 3' side or both sides. The PS linkage can also be adjacent to 2 nucleosides at the 5' end of the strand and/or 2 nucleosides at the 3' end of the strand. In an embodiment, the oligomeric compound comprising Formula (XI) is a siRNA. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid, comprises an antisense strand having 21 linked nucleotides, wherein the antisense strand sequence is represented by Formula (XII): 5' L-MDMFMFNMFMMFMFMFMMNMM 3'. The M is a 2'-OMe modified nucleoside, N is a modified or unmodified nucleoside (e.g., M, F, R, D, UNA, or LNA), F is a 2'-F modified nucleoside, L is a 5' OH, 5' vinyl phosphonate or a 5' phosphate (p), and no single modification type modifies more than two consecutive nucleotides. In certain embodiments, the sense strand comprises one or more phosphorothioate internucleotide (PS) linkage between 2 nucleosides. In a further embodiment, the antisense strand comprises a phosphorothioate internucleotide (PS) linkage adjacent to a deoxyribonucleoside (D) or ribonucleoside (R). The PS linkage can be adjacent to the deoxyribonucleoside (D) or ribonucleoside (R) on the 5' side, the 3' side or both sides. The PS linkage modification can be between positions 1-2, positions 2-3, 19-20 and/or positions 20-21 counting from the 5' end of the antisense strand. The PS linkage can also be adjacent to 2 nucleosides at the 5' end of the strand and/or 2 nucleosides at the 3' end of the strand. In an embodiment, the oligomeric compound comprising Formula (XII) is a siRNA. Modified nucleosides may comprise a modified base and/or a modified sugar (preferably at 2' position modified sugar). A deoxyribonucleoside (D) is also considered a modified nucleoside in a RNA based oligomeric compound.

In one embodiment, an oligomeric compound capable of inhibiting the expression of a target nucleic acid, comprises: (a) a sense strand having 21 linked nucleotides, wherein the sense strand sequence is represented by Formula (XI): 5' MFMMNMNMFFMMNMNMMFMDD 3', and (b) antisense strand having 21 linked nucleotides, wherein the antisense strand sequence is represented by Formula (XII): 5' L- MDMFMFNMFMMFMFMFMMNMM 3'. A duplex is formed by the sense and antisense strands, where the duplex region is 19 nucleotide pairs in length and each strand has a 2 nucleotide overhang at the 3' end. The D is a deoxyribonucleoside, M is a 2'-OMe modified nucleoside, N is a modified or unmodified nucleoside (e.g., M, F, R, D, UNA, or LNA), F is a 2'-F modified nucleoside, L is a 5' OH, 5' vinyl phosphonate or a 5' phosphate (p), and no single modification type modifies more than two consecutive nucleotides. In certain embodiments, the sense strand comprises one or more phosphorothioate internucleotide (PS) linkage between 2 nucleosides. In a further embodiment, the strands comprise a phosphorothioate internucleotide (PS) linkage adjacent to deoxyribonucleosides (D) or ribonucleosides (R). The PS linkage can be adjacent to the deoxyribonucleoside (D) or ribonucleoside (R) on the 5' side, the 3' side or both sides. The PS linkage modification on the antisense strand can be between positions 1-2, positions 2-3 and positions 19-20 and/or 20-21 counting from the 5' end of the antisense strand. The PS linkage can also be adjacent to 2 nucleosides at the 5' end of the strands and/or 2 nucleosides at the 3' end of the strands. In an embodiment, the oligomeric compound comprising Formulas (XI) and (XII) is a siRNA. Modified nucleosides may comprise a modified base and/or a modified sugar (preferably at 2' position modified sugar). A deoxyribonucleoside (D) is also considered a modified nucleoside in a RNA based oligomeric compound. Oligomeric compounds can be trafficked into target cells by a variety of modalities. In certain embodiments, oligomeric compounds enter cells via viral delivery vectors, lipid-based delivery, polymer-based delivery, and/or conjugate-based delivery.

In certain embodiments, the oligomeric compound described herein further comprises a conjugate. The conjugate can be selected from cholesterols, lipids, carbohydrates, phospholipids, biotin, phenazine, folate, phenanthridine, anthraquinone, acridine, fluoresceins, rhodamines, coumarins, and dyes. In a preferred embodiment, the conjugate is N-Acetylgalactosamine (GalNAc). In an embodiment, the conjugate can be attached to the 3' end of a sense strand. In a preferred embodiment, the conjugated oligomeric compound is a siRNA-GalNAc conjugate.

In certain embodiments, the oligomeric compound described herein inhibits the expression of a target nucleic acid by at least about 70%, 75%, 80%, 85%, 90%, 95%, 98% or 99%.

In certain embodiments, a pharmaceutical composition comprises the oligomeric compound described herein, alone or in combination with a pharmaceutically acceptable carrier or excipient.

### Oligomeric compounds

Oligomeric compounds of the invention include double stranded oligomeric compounds such as short hairpin RNAs (shRNAs) and small interfering RNAs (siRNAs). An oligomeric compound may be "antisense" or comprise an "antisense strand" to a target nucleic acid, meaning that is is capable of undergoing hybridization to a target nucleic acid through hydrogen bonding.

In certain embodiments, an oligomeric compound has a nucleobase sequence that, when written in the 5' to 3' direction, comprises the reverse complement of the target segment of a target nucleic acid to which it is targeted. For example, in certain such embodiments, a siRNA comprises an antisense strand which has a nucleobase sequence that, when written in the 5' to 3' direction, comprises the reverse complement of the target segment of a target nucleic acid to which it is targeted.

It is possible to increase or decrease the length of an oligomeric compound and/or introduce base mismatch(s) without eliminating activity (US Patent 7,772,203). For example, it is possible to introduce non-canonical base pairings (e.g., A:G, A:C, G:U, I:U, I:A, or I:C) into an oligomeric compound without eliminating activity. In certain embodiments, designing oligomeric compounds with one or more non-canonical base pairings, i.e., mismatch(s), enhances the activity of the oligomeric compound.

The oligomeric compound can comprise a mismatch(es) with the target, between the oligomeric strands within the duplex, or combinations thereof. The mismatch may occur throughout the siRNA such as in the overhang region or the duplex region.

### Oligomeric Compound Motifs

A motif refers to a pattern of modification of an oligomeric compound. Various motifs have been described in the art (e.g., US Patent 11,203,755; US Patent 10,870,849; EP Patent 1,532,248; US Patent 11,406,716; US Patent 10,668,170; US Patent 9,796,974; US Patent 8,754,201; US Patent 10,837,013; US Patent 7,732,593; US Patent 7,015,315; US Patent 7,750,144; US Patent 8,420,799; US Patent 8,809,516; US Patent 8,796,436; US Patent 8,859,749; US Patent 9,708,615; US Patent 10,233,448; US Patent 10,273,477; US Patent 10,612,024; US Patent 10,612,027; US Patent 10,669,544; US Patent 11,401,517; US Patent 9,260,471; US Patent 9,970,005; US Patent 11,193,126; US Patent 8,604,183; US Patent 9,150,605; US Patent 9,708,610; USSN 2020/0031862; and USSN 2016/0272970). However, it would be clear to skilled artisans that new and improved motifs (also referred to herein as chemical modification patterns) are within the scope of the invention.

In certain embodiments, oligomeric compounds disclosed herein have chemically modified subunits arranged into motifs or patterns (i.e., chemical modification motifs/patterns) to confer on to the oligomeric compounds beneficial properties including, but not limited to: enhanced inhibitory activity to increase potency; increased binding affinity to increase specificity for a target nucleic acid, thereby limiting off-target effects and increasing safety; or enhanced resistance to degradation by *in vivo* nucleases thereby increasing stability and durability. In certain embodiments, the oligomeric compounds are chimeras where the peripheral nucleobases of the oligomeric compounds comprise motifs with various modified or unmodified nucleobases so as to confer increased stability, specificity, safety and potency, while the central region of the compound comprises various modified or unmodified nucleobases to serve as substrates for RISC mediated degradation. Each distinct region can comprise uniform sugar moieties, modified, or alternating sugar moieties. Each region can comprise a varied pattern of phosphate and phosphorothioate linkages.

In certain embodiments, the oligomeric compounds targeted to a nucleic acid comprises a sense strand with motif described by the one of the following formulas:

Formula (III): 5' M*F*MMN*MN*MFFNMN*MN*MMFM*D*D 3',

Formula (X): 5' MFMMNMNMFFNMNMNMMNMDD 3',

or

Formula (XI): 5' MFMMNMNMFFMMNMNMMFMDD 3',

wherein
each D is a deoxyribonucleoside (D is a modification of R),
each R is a ribonucleoside,
each N is a nucleoside, modified or unmodified (e.g., D, R, M, F, UNA modified, or
LNA modified),
each M is a 2'-OMe modified nucleoside,
each F is a 2'-F modified nucleoside,
each * is a phosphorothioate (PS) linkage, and
wherein no single modification type modifies more than two consecutive nucleotides. In certain embodiments, the FFNM is FFRM or FFMM.

In certain embodiments, the oligomeric compounds targeted to a nucleic acid comprises an antisense strand with motif described by one of the following formulas:

Formula (VI): 5' L-M*N*MNMFNMFMMNMFMFMMN*M*M 3',

Formula (VII): 5' L-M*D*MFMFNMFMMFMFMFMMN*M*M 3',

Formula (VIII): 5' L-MNMNMFNMFMMNMFMFMMNMM 3',

Formula (XII): 5' L-MDMFMFNMFMMFMFMFMMNMM 3',

wherein
each D is a deoxyribonucleoside (D is a modification of R),
each R is a ribonucleoside,
each N is a nucleoside, modified or unmodified (e.g., D, R, M, F, UNA modified, or
LNA modified),
each M is a 2'-OMe modified nucleoside,
each F is a 2'-F modified nucleoside,
each L is a 5' phosphate, 5' vinyl phosphonate or 5' OH
each * is a phosphorothioate (PS) linkage, and
wherein no single modification type modifies more than two consecutive nucleotides. In certain embodiments, the FNM is FMM.

In certain embodiments, the oligomeric compounds targeted to an mRNA nucleic acid comprises siRNA duplex with any of the following motifs:
Duplex III with the sense strand of Formula (III) and antisense strand of Formula (VI);
Duplex IV with the sense strand of Formula (III) and antisense strand of Formula (VII);
Duplex VI with the sense strand of Formula (X) and antisense strand of Formula (VIII);
Duplex VII with the sense strand of Formula (XI) and antisense strand of Formula (XII);
Duplex VIII with the sense strand of Formula (X) and antisense strand of Formula (XII); or
Duplex IX with the sense strand of Formula (XI) and antisense strand of Formula (VIII);

wherein the duplex region is 19 to 23 nucleotide pairs in length, and
wherein no single modification type modifies more than two consecutive nucleotides.

### Target mRNAs and Associated Gene Expression

Gene expression may be modulated by oligomeric compound inhibition.

Inhibiting laminin (LMNA) gene expression in a cell may comprise administering to the cell an oligomeric compound targeted to an mRNA transcript of LMNA. In an embodiment, the oligomeric compound is designed to target a 19 nucleotide long sequence of LMNA conserved between human and mouse (SEQ ID NO: 1).

Inhibiting apolipoprotein C3 (ApoC3) gene expression in a cell may comprise administering to the cell an oligomeric compound targeted to an mRNA transcript of ApoC3. In an embodiment, the oligomeric compound is designed to target a 19 nucleotide long sequence of ApoC3 conserved between human and mouse (SEQ ID NO: 177).

Inhibiting Nucleolin (NCL) gene expression in a cell may comprise administering to the cell an oligomeric compound targeted to an mRNA transcript of NCL. In an embodiment, the oligomeric compound is designed to target a 19 nucleotide long sequence of NCL conserved between human and mouse (SEQ ID NO: 98).

### Hybridization

In some embodiments, hybridization occurs between an oligomeric compound disclosed herein and an mRNA. The most common mechanism of hybridization involves hydrogen bonding (e.g., Watson-Crick, Hoogsteen or reversed Hoogsteen hydrogen bonding) between complementary nucleobases of the nucleic acid molecules.

In Watson-Crick canonical base pairings, adenine (A) is complementary to thymine (T) in DNA, adenine (A) is complementary to uracil (U) in RNA, and Guanine (G) is complementary to cytosine (C) in both DNA and RNA. Base pairs, or complementary nucleobases, are usually Watson-Crick base pairs (C:G, A:U, A:T), but, non-canonical base pairs such as Hoogsteen base pairs (e.g., A:G or A:U), Wobble base pairs (e.g., G:U, I:U, I:A, I:C, wherein I is hypoxanthine) and the like are also permitted during hybridization of the oligomeric compound to a target nucleic acid or target region. Wobble base pairs in RNAi agents have previously been described (see e.g., US Patent 7,732,593; US Patent 7,750,144).

Nucleobase complementarity facilitates hybridization of the oligomeric compounds described herein to their target nucleic acids with the stronger the pairing (e.g., the more base pairs and/or the stronger the hydrogen bond), the stronger the hybridization of the oligomeric compound to the target. Hybridization can occur under varying conditions. Stringent conditions are sequence-dependent and are determined by the nature and composition of the oligomeric compound to be hybridized.

Methods of determining whether a sequence is specifically hybridizable to a target nucleic acid are well known in the art. In certain embodiments, the oligomeric compounds provided herein are specifically hybridizable with a target mRNA with little to no off-target binding.

### Complementarity

An oligomeric compound and a target nucleic acid are complementary to each other when a sufficient number of nucleobases of the oligomeric compound can hybridize with the corresponding nucleobases of the target nucleic acid, such that a desired effect will occur (e.g., inhibition of a target nucleic acid, such as an mRNA nucleic acid).

Non-complementary nucleobases between an oligomeric compound and an mRNA nucleic acid may be tolerated provided that the oligomeric compound remains able to specifically hybridize to a target nucleic acid. Moreover, an oligomeric compound may hybridize over one or more segments of an mRNA nucleic acid such that intervening or adjacent segments is not involved in the hybridization event (e.g., a loop structure, mismatch or hairpin structure).

In certain embodiments, the oligomeric compounds provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to an mRNA nucleic acid, a target region, target segment, or specified portion thereof. Percent complementarity of an oligomeric compound with a target nucleic acid can be determined using routine methods.

For example, an oligomeric compound in which 18 of 20 nucleobases of the antisense strand of the oligomeric compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. Percent complementarity of an oligomeric compound with a region of a target nucleic acid can be determined routinely using BLAST programs (basic local alignment search tools) and PowerBLAST programs known in the art (Altschul et al., 1990, J. Mol. Biol., 215:403-410; Zhang and Madden, 1997, Genome Res., 7:649-656) and available through the website for the National Center for Biotechnology Information (NCBI, https://blast.ncbi.nlm.nih.gov/Blast.cgi). Percent homology, sequence identity or complementarity, can be determined by, for example, NCBI Blast (Johnson et al., Nucleic Acids Res. 2008, 36 (Web Server issue): W5-W9).

In certain embodiments, the oligomeric compounds provided herein, or specified portions thereof, are fully complementary (i.e. 100% complementary) to a target nucleic acid, or specified portion thereof. For example, an oligomeric compound may be fully complementary to an mRNA nucleic acid, or a target region, or a target segment or target sequence thereof. As used herein, "fully complementary" means each nucleobase of an oligomeric compound is capable of precise base pairing with the corresponding nucleobases of a target nucleic acid. For example, a 20 nucleobase oligomeric compound is fully complementary to a target sequence that is 400 nucleobases long, so long as there is a corresponding 20 nucleobase portion of the target nucleic acid that is fully complementary to the oligomeric compound.

Fully complementary can also be used in reference to a specified portion of the oligomeric compound or the nucleic acid target. For example, a 20 nucleobase portion of a 30 nucleobase oligomeric compound can be "fully complementary" to a target sequence that is 400 nucleobases long. The 20 nucleobase portion of the 30 nucleobase oligomer is fully complementary to the target sequence if the target sequence has a corresponding 20 nucleobase portion wherein each nucleobase is complementary to the 20 nucleobase portion of the oligomeric compound. At the same time, the entire 30 nucleobase oligomeric compound may or may not be fully complementary to the target sequence, depending on whether the remaining 10 nucleobases of the oligomeric compound are also complementary to the target sequence.

The location of a non-complementary nucleobase may be at the 5' end or 3' end of the oligomeric compound. Alternatively, the non-complementary nucleobase or nucleobases may be at an internal position of the oligomeric compound. When two or more non-complementary nucleobases are present, they may be contiguous (i.e. linked) or non-contiguous.

In certain embodiments, oligomeric compounds that are 21 nucleobases in length comprise no more than 6, no more than 5, no more than 4, no more than 3, no more than 2, or no more than 1 non-complementary nucleobase(s) relative to a target nucleic acid, such as an mRNA nucleic acid, or specified portion thereof.

The oligomeric compounds provided herein also include those which are complementary to a portion of a target nucleic acid. As used herein, "portion" refers to a defined number of contiguous (i.e., linked) nucleobases within a region or segment of a target nucleic acid. A "portion" can also refer to a defined number of contiguous nucleobases of an oligomeric compound. In certain embodiments, the oligomeric compounds, are complementary to at least an 8 nucleobase portion of a target segment. In certain embodiments, the oligomeric compounds are complementary to at least a 9 nucleobase portion of a target segment. In certain embodiments, the oligomeric compounds are complementary to at least a 10 nucleobase portion of a target segment. In certain embodiments, the oligomeric compounds are complementary to at least an 11 nucleobase portion of a target segment. In certain embodiments, the oligomeric compounds are complementary to at least a 12 nucleobase portion of a target segment. In certain embodiments, the oligomeric compounds are complementary to at least a 13 nucleobase portion of a target segment. In certain embodiments, the oligomeric compounds are complementary to at least a 14 nucleobase portion of a target segment. In certain embodiments, the oligomeric compounds are complementary to at least a 15 nucleobase portion of a target segment. Also contemplated are oligomeric compounds that are complementary to at least a 9, 10, 11, 12, 13, 14, 15, 16, 17, 18, 19, 20, or more nucleobase portion of a target segment, or a range defined by any two of these values.

### Identity

The oligomeric compounds provided herein may also have a defined percent identity to a particular nucleotide sequence, SEQ ID NO, or compound represented by a specific ARNATAR number, or portion thereof. As used herein, an oligomeric compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the oligomeric compounds described herein as well as compounds having non-identical bases relative to the oligomeric compounds provided herein also are contemplated. The non-identical bases may be adjacent to each other or dispersed throughout the oligomeric compound. Percent identity of an oligomeric compound is calculated according to the number of bases that have identical base pairing relative to the sequence to which it is being compared.

In certain embodiments, a portion of the oligomeric compound is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion of the oligomeric compound is compared to an equal length portion of the target nucleic acid.

### Chemical Modifications

A nucleoside is a base-sugar combination. The nucleobase (also known as base) portion of the nucleoside is normally a heterocyclic base moiety. Nucleotides are nucleosides that further include a covalent linkage (e.g., phosphate group or a chemically modified linkage as described *infra*) to the sugar portion of the nucleoside. Oligonucleotides are formed through the covalent linkage of adjacent nucleotides to one another, to form a linear polymeric oligonucleotide. Within the oligonucleotide structure, the linkage groups are commonly referred to as forming the internucleoside linkages of the oligonucleotide. Oligomeric compounds according to the invention are made up of multiple oligonucleotides (e.g., siRNAs or shRNAs)

Modifications to oligomeric compounds encompass substitutions or changes to nucleobases, internucleoside linkages or sugar moieties. Modified oligomeric compounds are often preferred over native or unmodified forms because of desirable properties such as, for example, enhanced delivery (e.g., increased cellular uptake), enhanced specificity or affinity for a nucleic acid target, increased stability in the presence of nucleases, enhanced safety (e.g., fewer side effects after administration of the compound to a subject) or increased potency (e.g., inhibitory activity).

### Nucleobase Modifications

A nucleobase is a heterocyclic moiety capable of base pairing with a nucleobase of another nucleic acid. Modifications to nucleobases can be advantageous to an oligomeric compound for various reasons including, but not limited to, increase stability of the oligomeric compound, increase specificity, decreased immunogenicity of the oligomeric compound, increase affinity of the oligomeric compound, increase potency of the oligomeric compound and other desirable features.

Examples of nucleobase modifications and their advantages are well known in the art (Friedrich and Aigner, Therapeutic siRNA: State-of-the-Art and Future Perspectives, 2022, BioDrugs, 36(5):549-571; Hu et al., Therapeutic siRNA: State of the Art, Signal Transduction and Targeted Therapy, 2020, 5:101). Nucleobase modifications can comprise substituting nucleobases with nucleobase analogs or modification of a part of the nucleobase. Examples of nucleobase modifications include, but are not limited to, pseudouridine, 2'-thiouridine, N6'-methyladenosine, and 5'-methylcytidine, 5'-fluoro-2'-deoxyuridine, N-ethylpiperidine 5' triazole-modified adenosine, 5'-nitroindole, 2',4'-difluorotolylribonucleoside, N-ethylpiperidine 7'-EAA triazole-modified adenosine, 6'-phenylpyrrolocytosine and the like.

In certain embodiments, oligomeric compounds targeted to an mRNA nucleic acid comprise one or more modified nucleobases. In certain embodiments, the modified nucleobases are, for example, deoxyribonucleotides substituted for ribonucleotides. In certain embodiments, a modified nucleobase can be a thymine substitution for an uracil. In certain embodiments, multiple nucleobases of an oligomeric compound are modified. In certain embodiments, each nucleobase of an oligomeric compound is modified.

### Internucleoside Linkage Modifications

The naturally occuring internucleoside linkage of RNA and DNA is a 3' to 5' phosphodiester linkage. For nucleosides that include a pentofuranosyl sugar, the phosphate group can be linked to the 2', 3' or 5' hydroxyl moiety of the sugar. Oligomeric compounds having one or more modified, i.e. non-naturally occurring, internucleoside linkages are often selected over oligomeric compounds having naturally occurring internucleoside linkages because of desirable properties such as, for example, enhanced cellular uptake, enhanced affinity for target nucleic acids, decrease toxicity, increased stability and durability, decreased degradation and other desirable features for an oligomeric compound. Modified internucleoside linkages and their advantages are well known in the art (Friedrich and Aigner, Therapeutic siRNA: State-of-the-Art and Future Perspectives, 2022, BioDrugs, 36(5):549-571; Hu et al., Therapeutic siRNA: State of the Art, Signal Transduction and Targeted Therapy, 2020, 5:101).

Oligomeric compounds having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates (e.g., 5'-methylphosphonate (5'-MP)), phosphoramidate, phosphorothioates (e.g., phosphorodithioate Rp isomer (PS,Rp), phosphorodithioate Rp isomer (PS,Sp), or 5'-phosphorothioate (5'-PS)), methoxypropylphosphonate, (*S*)-5' -C-methyl with Phosphate, peptide nucleic acid (PNA), and 5'-(E)-vinylphosphonate.

In certain embodiments, oligomeric compounds targeted to an mRNA nucleic acid comprise one or more modified internucleoside linkages. In certain embodiments, the modified internucleoside linkages are phosphorothioate (PS) linkages. In certain embodiments, one or more internucleoside linkage of an oligomeric compound is a phosphorothioate internucleoside linkage. In certain embodiments, the PS linkage is adjacent to a deoxyribonucleoside (sometimes referred to as a DNA nucleoside, "D" or "d" herein) or a ribonucleoside (sometimes referred to as a RNA nucleoside, "R" or "r" herein). In certain embodiments, each internucleoside linkage of an oligomeric compound is a phosphorothioate internucleoside linkage.

### Sugar Modifications

Oligomeric compounds provided herein can contain one or more nucleosides wherein the sugar group has been modified. Such sugar modified nucleosides may impart desirabe features such as increased stability, increased durability (e.g., increased half-life), increased binding affinity, decreased off-target effects, decreased immunogenicity, decreased toxicity, increased potency, or some other beneficial biological property to the oligomeric compounds. Sugar modifications and their advantages are known in the art (Friedrich and Aigner, 2022, BioDrugs, 36(5):549-571; Hu et al., Therapeutic siRNA: State of the Art, Signal Transduction and Targeted Therapy, 2020, 5:101; Chiu and Rana, 2003, RNA, 9:1034-1048; Choung et al., Biochem Biophys Res Commun, 2006, 342:919-927; Amarzguioui et al., 2003, Nucleic Acids Res, 31(2):589-595; Braasch et al., 2003, Biochemistry, 42(26):7967-7975; Czauderna et al., 2003, Nucleic Acids Res, 31(11):2705-2716; Allerson et al., 2005, J Med Chem, 48:901-904; Layzer et al., 2004, RNA, 10:766-771; Ui-Tei, et al., 2008, Nucleic Acids Res, 36(7):2136-51; Bramsen and Kjems, 2012, Frontiers in Genetics, 3(154):1-22; Bramsen et al., 2010, Nucleic Acids Res, 38(17):5761-5773; Muhonen et al., 2007, Chem & Biodiversity, 4:858-873; Viel et al., 2008, Oligonucleotides, 18:201-212).

In certain embodiments, nucleosides comprise a chemically modified ribofuranose ring moiety. Examples of chemically modified ribofuranose rings can include, without limitation, addition of substitutent groups (e.g., 5' sugar modifications, or 2' sugar modifications); bridging of non-geminal ring atoms to form bicyclic nucleic acids (BNA); replacement of the ribosyl ring oxygen atom with S, N(R), or C(R1)(R)2 (R = H, C₁-C₁₂ alkyl or a protecting group); nucleoside mimetic; and combinations thereof.

A 2'-modified sugar refers to a furanosyl sugar modified at the 2' position. A 2'-modified nucleoside refers to a nucleoside comprising a sugar modified at the 2' position of a furanose ring. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙONH₂, OCH₂C(=O)N(H)CH₃, and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, and a group for improving the pharmacodynamic properties of an oligomeric compound, and other substituents having similar properties.

Further examples of nucleosides having modified sugar moieties include, without limitation, nucleosides comprising 5'-vinyl, 5'-methyl (R or S), 2'-F-5'-methyl, 4'-S, 2'-deoxy-2'-fluoro (2'-F), 2'-OCH₃ (2'-O-methyl, 2'-OMe), 2'-O(CH₂)2OCH₃ (2'-O-methoxyethyl, 2'-O-MOE, 2'-MOE), 2'-O-methyl-4-pyridine, phosphorodiamidate morpholino (PMO), tricyclo-DNA (tcDNA), 2'-arabino-fluoro, 2'-O-benzyl, glycol nucleic acid (GNA), and unlocked nucleic acid (UNA) substituent groups. The substituent at the 2' position can also be selected from allyl, amino, azido, thio, O-allyl, O-C₁-C₁₀ alkyl, OCF₃, O(CH₂)2SCH₃, O(CH₂)2-O-N(Rm)(Rn), and O-CH₂-C(=O)-N(Rm)(Rn), where each Rm and Rn is, independently, H or substituted or unsubstituted C₁-C₁₀ alkyl. 2'-OMe or 2'-OCH₃ or 2'-O-methyl each refers to a nucleoside comprising a sugar comprising an -OCH₃ group at the 2' position of the sugar ring. 2'-F refers to a sugar comprising a fluoro group at the 2' position. 2'-O-methoxyethyl or 2'-O-MOE or 2'-MOE each refers to a nucleoside comprising a sugar comprising an -O(CH₂)2OCH₃ group at the 2' position of the sugar ring.

BNAs refer to modified nucleosides comprising a bicyclic sugar moiety wherein a bridge connecting two carbon atoms of the sugar ring connects the 2' carbon and another carbon of the sugar ring. Examples of bicyclic nucleosides include, without limitation, nucleosides comprising a bridge between the 4' and the 2' ribosyl ring atoms such as in locked nucleic acid (LNA). In certain embodiments, oligomeric compounds provided herein include one or more bicyclic nucleosides wherein the bridge comprises a 4' to 2' bicyclic nucleoside. LNAs and UNAs have been described by Campbell and Wengel (Chem Soc Rev, 2011, 40(12):5680-9).

In certain embodiments, oligomeric compounds comprise one or more nucleotides having modified sugar moieties. In certain embodiments, the modified sugar moiety has a 2'-OMe modification. In certain embodiments, the modified sugar moiety has a 2'-F modification. In certain embodiments, the 2'-OMe and/or 2'-F modified nucleotides are arranged in a motif. In certain embodiments, the motif is selected from any of Formulas (I)-(VII).

### Oligomeric Compound Delivery Systems

Oligomeric compounds require entry into target cells to become active. A variety of modalities have been used to traffic oligomeric compounds into target cells including viral delivery vectors, lipid-based delivery, polymer-based delivery, and conjugate-based delivery (Paunovska et al., Drug Delivery Systems for RNA Therapeutics, 2022, Nature Reviews Genetics, 23(5):265-280; Chen et al., 2022, Molecular Therapy, Nucleic Acids, 29:150-160).

Lipid-based particles can form specific structures such as micelles, liposomes and lipid nanoparticles (LPNs) to carry oligomeric compounds into cells. To form these particles, LPNs can include one or more of a cationic or ionizable lipid (e.g., DLin-MC3-DMA, SM-102, or ALC-0315), cholesterol, a helper lipid, 1,2-distearoyl-sn-glycero-3-phosphocholine (DSPC), poly(ethylene glycol) (PEG) modified lipid (e.g., PEG-2000-C-DMG, PEG-2000-DMG, or ALC-0159), C12-200, cKK-E12 and the like. Different combinations of lipids can be formulated to affect the delivery of the oligomeric compound to different types of cells. In one example, therapeutic siRNA patisiran was formulated in cationic ionizable lipid DLin-MC3-DMA, cholesterol, polar phospholipid DSPC, and PEG-2000-C-DMG for delivery to hepatocytes.

Polymer-based particles are also used in oligomeric compound delivery systems. Such polymers include poly(lactic-co-glycolic acid) (PLGA), polyethylenimine (PEI), poly(l-lysine) (PLL), poly(beta-amino ester) (PBAE), dendrimers (e.g., poly(amidoamine) (PAMAM) or PLL), and other polymers or modified polymers thereof. The polymer composition can be varied depending on the traits desired for delivery of the oligomeric compound.

The oligomeric compounds disclosed herein may be covalently linked to one or more moieties or conjugates which enhance the activity, cellular distribution or cellular uptake of the resulting compound. Conjugate groups can include cholesterols, lipids, carbohydrates, phospholipids, biotin, phenazine, folate, phenanthridine, anthraquinone, acridine, fluoresceins, rhodamines, coumarins, dyes, tocopherol (Nishina et al., 2008, Molecular Therapy, 16(4):734-740), etc. Conjugate-based delivery can actively deliver oligomeric compounds to specific cell types.

In an example, N-Acetylgalactosamine (GalNAc) is conjugated to an oligomeric compound and delivers it into hepatocytes. Various GalNAc conjugates can be found in several publications including the following: Sharma et al., 2018, Bioconjugate Chem, 29:2478-2488; Nair et al., J. Am. Chem. Soc. 2014, 136(49):16958-16961; Keam, 2022, Drugs, 82:1419-1425; US Patent 10,087,208; Prakash et al., 2014, Nucleic Acids Res, 42(13):8796-807; Debacker et al., 2020, Molecular Therapy, 28(8):1759-1771; US Patent 11,110,174; US Patent 9,796,756; US Patent 9,181,549; US Patent 10,344,275; US Patent 10,570,169; US Patent 9,506,030; and, US Patent 7,582,744.

In a further example, the following GalNAc is conjugated at the 3' end of an oligonucleotide, wherein the oligonucleotide comprises the sense strand of an ARNATAR designed siRNA.

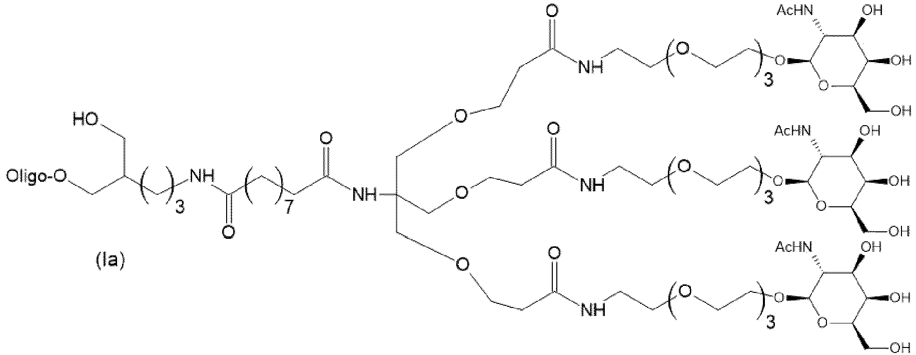

### Oligomeric Compound Synthesis

siRNAs were designed, synthesized, and prepared using methods known in the art.

Solid phase syntheses of oligonucleotides were done on a MerMade^{™} 48x synthesizer (BioAutomation, LGC, Biosearch Technologies, Hoddesdon, UK), which can make up to 48 1µMole or 5µMole scale oligonucleotides per run using standard phosphoramidite chemistry. Solid support is controlled pore glass (500-1400 Å) loaded with 3'-GalNAc conjugates (AM Chemicals, Vista, CA, USA; Primetech ALC, Minsk, Belarus; Gene Link, Elmsford, NY, USA; or any GalNAc conjugate disclosed herein) or universal solid support (AM Chemicals, Vista, CA, USA). Ancillary synthesis reagents and standard 2'-cyanoethyl phosphoramidite monomers (2'-fluoro nucleosides, 2'-O-methyl nucleosides, RNA nucleosides, DNA nucleosides) were obtained from various sources (Hongene Biotech, Shanghai, China; Sigma-Aldrich, St. Louis, MO, USA; Glen Research, Sterling, VA, USA; ThermoFisher Scientific, Waltham, MA, USA; LGC Biosearch Technologies, Hoddesdon, UK). Phosphoramidite mixtures were prepared in anhydrous acetonitrile or 30% DMF:acetonitrile and were coupled using 0.25M 4,5-dicyanoimidazole(DCI) (Sigma-Aldrich, St. Louis, MO, USA) with coupling times ranging from 120-360 seconds. Standard phosphodiester linkages were achieved using 0.02M iodine mixture in Tetrahydrofuran (THF), pyridine and water. Phosphorothiate linkages were generated using 0.05M sulfurizing Reagent II (3-((Dimethylamino-methylidene)amino)-3H-1,2,4-dithiazole-3-thione, DDTT) (40:60,Pyridine/Acetonitrile) (LGC Biosearch Technologies, Hoddesdon, UK) with an oxidation time of 6 minutes. All sequences were synthesized with Dimethoxy Trityl (DMT) protecting group removed.

Upon completion of solid phase synthesis, the oligonucleotides were cleaved from the solid support and deprotection of base labile groups performed by incubation in ammonium hydroxide at 55°C for 6 hours. Ammonium hydroxide was removed using a centrifugal vacuum concentrator to dryness at room temperature. For sequences containing natural ribonucleotides (2'-OH) protected with tert-butyl dimethyl silyl (TBDMS), a second deprotection was performed using triethylamine; trihydrofluoride (TEA:3HF). To each TBDMS protected oligonucleotide 100 µL DMSO and 125µL TEA:3HF was added and incubated at 65°C for 2.5 hours. After incubation 25µL of 3M sodium acetate was added to the solution which was subsequently precipitated in butanol at -20°C for 30 minutes. The cloudy solution was centrifuged to a cake at which time the supernatant was carefully decanted with a pipette. The standard precipitation process was then completed with 75% ethanol:water then 100% ethanol as supernatant solutions. The oligonucleotide cake was dried for 30 minutes in a centrifugal vacuum concentrator.

Desalting without HPLC purification was performed after precipitation with 3M sodium acetate with a follow on G25 Sephadex^{®} column (Sigma-Aldrich, St. Louis, MO, USA) elution. Purification of oligonucleotides was afforded by anion exchange chromatography on a Gilson GX271 prep HPLC system (Middleton, WI, USA) using BioWorks Q40 resin (Uppsala, Sweden). Final desalt was performed by Sephadex^{®} G25 column. All oligonucleotides were analyzed by ion pairing reverse phase HPLC for purity an Agilent 1200 analytical HPLC (Santa Clara, CA, USA), negative ion mass spectrometry for intact mass on an Agilent 6130 single quad mass spectrometer (Santa Clara, CA, USA), and A260 quantification by UV/Vis on a Tecan Infinite^{®} M Plex plate reader (Zurich, Switzerland).

### Double Stranded Oligomeric Compound Duplex Formation

In general, for a double stranded oligomeric compound such as an siRNA compound, a sense and antisense oligonucleotide is annealed together to form a duplex. Duplex formation of 50-300µM can be achieved by heating samples at 94°C for 4 mins in 1x phosphate-buffered saline in a block heater, followed by removal of the heating block containing the samples from the block heater and allowing it to gradually cool down to room temperature over a time course of 1hr.

### Compositions and Methods for Formulating Pharmaceutical Compositions

The oligomeric compounds of the invention, such as siRNA compounds described herein, can be combined with pharmaceutically acceptable active or inert substances, such as a diluent, excipient or carrier, for the preparation of pharmaceutical compositions or formulations.

Compositions and methods for the formulation of pharmaceutical compositions are dependent upon a number of criteria, including, but not limited to, route of administration, extent of disease, or dose to be administered.

In certain embodiments, the pharmaceutical carrier or excipient is a pharmaceutically acceptable solvent, suspending agent or any other pharmacologically inert vehicle for delivering one or more nucleic acid compounds to an animal. The excipient can be liquid or solid and can be selected, with the planned manner of administration in mind, so as to provide for the desired bulk, consistency, etc., when combined with a nucleic acid and the other components of a given pharmaceutical composition. Typical pharmaceutical carriers include, but are not limited to, binding agents (e.g., pregelatinized maize starch, polyvinylpyrrolidone and/or hydroxypropyl methylcellulose, etc.); fillers (e.g., lactose and other sugars, microcrystalline cellulose, pectin, gelatin, calcium sulfate, ethyl cellulose, polyacrylates and/or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, and/or sodium acetate, etc.); disintegrants (e.g., starch, and/or sodium starch glycolate, etc.); and wetting agents (e.g., sodium lauryl sulphate, etc.).

Pharmaceutically acceptable organic or inorganic excipients, which do not deleteriously react with nucleic acid compounds, suitable for parenteral or non-parenteral administration can also be used to formulate the compositions of the present invention. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an oligomeric compound and a pharmaceutically acceptable diluent. In certain embodiments, the pharmaceutically acceptable diluent is PBS. In certain embodiments, the oligomeric compound is a siRNA.

Pharmaceutical compositions comprising oligomeric compounds such as siRNAs can encompass any pharmaceutically acceptable salts, esters, or salts of such esters, or any other dsRNA which, upon administration to an animal, including a human, is capable of providing (directly or indirectly) the biologically active metabolite or residue thereof. Accordingly, for example, the disclosure is also drawn to pharmaceutically acceptable salts of oligomeric compounds, prodrugs, pharmaceutically acceptable salts of such prodrugs, and other bioequivalents. Suitable pharmaceutically acceptable salts include, but are not limited to, sodium and potassium salts.

In certain embodiments, a pharmaceutical composition is prepared for administration by injection (e.g., intravenous, subcutaneous, and/or intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer (e.g., PBS). In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers.

### Dosages

For purposes of the disclosure, the amount or dose of the active agent (oligomeric compound of the invention) administered should be sufficient to e.g., inhibit the expression of a target nucleic acid in an animal. In the animal (e.g., human), dose will be determined by the efficacy of the particular active agent and the condition of the animal, as well as the body weight of the animal to be treated.

Many assays for determining an administered dose are known in the art.

The dose of the active agent of the present disclosure also will be determined by the existence, nature and extent of any adverse side effects that might accompany the administration of a particular active agent of the present disclosure. Typically, the attending physician will decide the dosage of the active agent of the present disclosure with which to treat each individual patient, taking into consideration a variety of factors, such as age, body weight, general health, diet, sex, active agent of the present disclosure to be administered, route of administration, and the severity of the condition being treated.

### Dosing

In certain embodiments, pharmaceutical compositions are administered according to a dosing regimen (e.g., dose, dose frequency, and duration) wherein the dosing regimen can be selected to achieve a desired effect. The desired effect can be, for example, reduction of a target nucleic acid or the prevention, reduction, amelioration or slowing the progression of a disease, disorder and/or condition, or symptom thereof, associated with the target nucleic acid. In certain embodiments, the variables of the dosing regimen are adjusted to result in a desired concentration of pharmaceutical composition in a subject. "Concentration of pharmaceutical composition" as used with regard to dose regimen can refer to the oligomeric compound or active ingredient of the pharmaceutical composition. For example, in certain embodiments, dose and dose frequency are adjusted to provide a tissue concentration or plasma concentration of a pharmaceutical composition at an amount sufficient to achieve a desired effect.

Dosing is dependent on severity and responsiveness of the disease state to be treated, with the course of treatment lasting from several days to several months, or until a cure is effected or a diminution of the disease state is achieved. Dosing is also dependent on drug potency and metabolism. In certain embodiments, dosage is from 0.01 µg to 50 mg per kg of body weight, 0.01 µg to 100 mg per kg of body weight, or within a range of 0.001 mg to 1000 mg dosing, and may be given once or more daily, weekly, monthly, quarterly or yearly, or even once every 2 to 20 years. Following successful treatment, it may be desirable to have the patient undergo maintenance therapy to prevent the recurrence of the disease state, wherein the oligomeric compound is administered in maintenance doses, ranging from 0.01 µg to 100 mg per kg of body weight, once or more daily, once or more weekly, once or more monthly, once or more quarterly, once or more yearly, to once every 20 years or ranging from 0.001 mg to 1000 mg dosing. In certain embodiments, it may be desirable to administer the oligomeric compound from at most once daily, once weekly, once monthly, once quarterly, once yearly, once every two years, once every three years, once every four years, once every five years, once every ten years, to once every 20 years.

In certain embodiments, the range of dosing is between any of 1mg-1500mg, 100mg-1400mg, 100mg-1300mg, 100mg-1200mg, 100mg-1100mg, 100mg-1000mg, 100mg-900mg, 200mg-800mg, 300mg-700mg, 400mg-600mg, 100mg-400mg, 200mg-500mg, 300mg-600mg, and 400mg-700mg. In certain embodiments, a dose is about 100mg, 150mg, 200mg, 250mg, 300mg, 350mg, 400mg, 450mg, 500mg, 550mg, 600mg, 650mg, 700mg, 800mg, 850mg, 900mg, 950mg, 1000mg, 1050mg, 1100mg, 1150mg, 1200mg, 1250mg, 1300mg, 1350mg, 1400mg, 1450mg, or 1500mg.

In certain embodiments, the dsRNA is dosed at any of about 150mg, 200mg, 300mg, 400mg, 500mg, 600mg, 700mg, 800mg, or 900mg twice a year. In certain embodiments, the dsRNA is dosed at about 150mg, 200mg, 300mg, 400mg, 500mg, 600mg, 700mg, 800mg, or 900mg quarterly.

### Administration

The oligomeric compounds, such as siRNAs, or pharmaceutical compositions of the present invention can be administered in a number of ways depending upon whether local or systemic treatment is desired and upon the area to be treated. Administration can be oral, inhaled or parenteral.

In certain embodiments, the compounds and compositions as described herein are for parenteral administration. Parenteral administration includes intravenous, intra-arterial, subcutaneous, intraperitoneal or intramuscular injection or infusion; or intracranial, e.g., intrathecal or intraventricular, administration. In certain embodiments, parenteral administration is by infusion. Infusion can be chronic or continuous or short or intermittent. In certain embodiments, infused pharmaceutical agents are delivered with a pump.

In certain embodiments, parenteral administration is by injection. The injection can be delivered with a syringe or a pump. In certain embodiments, the injection is a bolus injection. In certain embodiments, the injection is administered directly to a tissue or organ.

In certain embodiments, formulations for parenteral, intrathecal or intraventricular administration can include sterile aqueous solutions which can also contain buffers, diluents and other suitable additives such as, but not limited to, penetration enhancers, carrier compounds and other pharmaceutically acceptable carriers or excipients.

In certain embodiments, formulations for oral administration of the compounds or compositions can include, but is not limited to, pharmaceutical carriers, excipients, powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders can be desirable. In certain embodiments, oral formulations are those in which compounds provided herein are administered in conjunction with one or more penetration enhancers, surfactants and chelators.

### In Vitro Testing of siRNAs

Described herein are methods for treatment of cells with siRNAs, which can be modified appropriately for treatment with other oligomeric compounds.

Cells may be treated with siRNAs when the cells reach approximately 60-80% confluency in culture.

One reagent commonly used to introduce siRNAs into cultured cells includes the cationic lipid transfection reagent Lipofectamine^{™} RNAiMAX (Invitrogen, Waltham, MA). siRNAs may be mixed with Lipofectamine^{™} RNAiMAX in OPTI-MEM 1 (Thermo Fisher Scientific, Waltham, MA) to achieve the desired final concentration of siRNA and a Lipofectamine^{™} RNAiMAX concentration that may range from 0.001 to 300 nM siRNAs. Transfection procedures are done according to the manufacturer's recommended protocols.

Another technique used to introduce siRNAs into cultured cells includes electroporation.

siRNAs conjugated with GalNAc can be introduced to cells through incubation of the siRNAs with cells without transfection reagents, referenced herein as "free uptake". The siRNA-GalNAc conjugates are transported into asialoglycoprotein receptor (ASGR) positive cells such as hepatocytes via endocytosis.

Cells are treated with siRNAs by routine methods. Cells may be harvested 4 -144 hours after siRNAs treatment, at which time mRNA (harvested at 4-144 hrs) or protein levels (extracted at 24-96 hrs) of target nucleic acids are measured by methods known in the art and described herein. In general, treatments are performed in multiple replicates, and the data are presented as the average of the replicate treatments plus the standard deviation.

The concentration of siRNAs used varies from cell line to cell line and target to target. Methods to determine the optimal siRNAs concentration for a particular target in a particular cell line are well known in the art. In general, cells are treated with siRNAs in a dose dependent manner to allow for the calculation of the half-maximal inhibitory concentration value (IC50). siRNAs are typically used at concentrations ranging from 0.001 nM to 300 nM when transfected with Lipofectamine^{™} RNAiMAX. siRNAs are used at higher concentrations ranging from 7.5 to 20,000 nM when transfected using electroporation or free uptake.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Thermo Fisher Scientific, Waltham, MA), Qiagen RNeasy kit (Qiagen, Hilden, Germany), or AcroPrep Advance 96-well Filter Plates (Pall Corporation, Port Washington, New York) using Qiagen's RLT, RW1 and RPE buffers. RNA extraction procedures are done according to the manufacturer's recommended protocols.

### In Vivo Testing of Oligomeric Compounds

The oligomeric compounds of the invention, for example, siRNAs, are tested in animals to assess their ability to inhibit expression of a target nucleic acid and produce phenotypic changes such as a change in one or more markers affected by the target nucleic acid. Also, the phenotypic change can be a decrease in a disease, disorder, condition, or symptom related to the target nucleic acid. Testing may be performed in normal animals, or in experimental disease models. For administration to animals, oligomeric compounds are formulated in a pharmaceutically acceptable diluent, such as phosphate-buffered saline (PBS). Administration includes parenteral routes of administration, such as intraperitoneal, intravenous, and subcutaneous. Calculation of dosage and dosing frequency depends upon factors such as route of administration and animal body weight. In one embodiment, following a period of treatment with oligomeric compounds of the invention, RNA encoding the target nucleic acid is isolated from liver tissue and changes in the target nucleic acid expression are measured. Changes in protein levels expressed by the target nucleic acid can also be measured.

### Kits of the Invention

According to another aspect of the invention, kits are provided. Kits according to the invention include package(s) comprising any of the compositions of the invention or oligomeric compound of the invention. In various aspects, the kit comprises any of the compositions of the invention as a unit dose. For purposes herein "unit dose" refers to a discrete amount dispersed in a suitable carrier.

The phrase "package" means any vessel containing compositions presented herein. In preferred embodiments, the package can be a box or wrapping. Packaging materials for use in packaging pharmaceutical products are well known to those of skill in the art. Examples of pharmaceutical packaging materials include, but are not limited to, blister packs, bottles, tubes, inhalers, pumps, bags, vials, containers, syringes (including pre-filled syringes), bottles, and any packaging material suitable for a selected formulation and intended mode of administration and treatment.

The kit can also contain items that are not contained within the package but are attached to the outside of the package, for example, pipettes.

Kits may optionally contain instructions for administering compositions of the present invention to a subject having a condition in need of treatment. Kits may also comprise instructions for approved uses of components of the composition herein by regulatory agencies, such as the United States Food and Drug Administration. Kits may optionally contain labeling or product inserts for the present compositions. The package(s) and/or any product insert(s) may themselves be approved by regulatory agencies. The kits can include compositions in the solid phase or in a liquid phase (such as buffers provided) in a package. The kits also can include buffers for preparing solutions, and pipettes for transferring liquids from one container to another.

The kit may optionally also contain one or more other compositions for use in combination therapies as described herein. In certain embodiments, the package(s) is a container for any of the means for administration such as intravitreal delivery, intraocular delivery, intratumoral delivery, peritumoral delivery, intraperitoneal delivery, intrathecal delivery, intramuscular injection, subcutaneous injection, intravenous delivery, intra-arterial delivery, intraventricular delivery, intrasternal delivery, intracranial delivery, or intradermal injection.

### Methods of Use

Disclosed herein are methods for inhibiting the expression of a target nucleic acid in a subject comprising administering an effective amount of an oligomeric compound of the invention or a pharmaceutical composition of the invention, so as to inhibit the expression of a target nucleic acid in the subject.

The subject may be a mammal, including, but not limited to, mammals of the order Rodentia, such as mice and hamsters, and mammals of the order Logomorpha, such as rabbits, mammals from the order Carnivora, including Felines (cats) and Canines (dogs), mammals from the order Artiodactyla, including Bovines (cows) and Swines (pigs) or of the order Perissodactyla, including Equines (horses). The mammals may be of the order Primates, Ceboids, or Simoids (monkeys) or of the order Anthropoids (humans and apes). The mammal is preferably a human.

### ADVANTAGES OF THE INVENTION

The field of oligomeric therapeutic compounds is still maturing and improvements in delivery, stability, specificity, safety and potency are still being sought to improve the therapeutic efficacy of the oligomeric compound. Three general aspects of oligmer design subject to improvement are, in no particular order of importance, 1) the sequence of the oligomeric compound, 2) the chemical modifications to the oligomeric compound, and 3) the delivery modality of the oligomeric compound. The importance of the three aspects is exemplified in the progression of oligomeric compounds leading to the development of vutrisiran, the latest FDA approved siRNA therapeutic. The developer of vutrisiran, Alnylam, has taken three iterations of TTR targeting siRNAs to clinical trial: patisiran, revusiran and vutrisiran.

The first siRNA developed to target TTR is patisiran (also known as Onpattro^{™}), where some chemically modified nucleosides are scattered along the 21-nucleotide long sense and antisense strands forming the siRNA duplex in order to stabilize the siRNA and increase inhibition potency over non-modified siRNA with the nucleotide same sequence (Friedrich and Aigner, Therapeutic siRNA: State-of-the-Art and Future Perspectives, 2022, BioDrugs, 36(5):549-571). Patisiran was given FDA approval as the first in class, siRNA, therapeutic.

The second siRNA developed to target TTR is revusiran. Although revusiran also targeted TTR, in this siRNA, a new sequence, additional modified nucleosides, modified inter-nucleoside linkages and a N-Acetylgalactosamine (GalNAc) conjugate was introduced. However, during Phase 3 clinical trial, revusiran treatment was stopped early due to an imbalance in mortality of trial patients and the trial discontinued (Judge et al., Phase 3 Multicenter Study of Revusiran in Patients with Hereditary Transthyretin-Mediated (hATTR) Amyloidosis with Cardiomyopathy (ENDEAVOUR), Cardiovascular Drugs and Therapy, 2020, 34:357-370). Ultimately, the revusiran program was discontinued.

The third siRNA developed to target TTR is vutrisiran (also known as ALN-TTRSC02 and Amvuttra^{™}) and combined the sequence of revusiran with additional chemical modifications and GalNAc conjugate delivery modality (Janas et al., Safety evaluation of 2'-deoxy-2'-fluoro nucleotides in GalNAc-siRNA conjugates, Nucleic Acids Research, 2019, 47(7): 3306-3320). The chemical modifications of vitrusiran stabilized it enough to allow a more advantageous dosing schedule and administration method compared to patisiran: 25 mg vitrusiran was administered to a patient once every three months via subcutaneous injection compared to 0.3 mg/kg patisiran administered to patients once every three weeks via intravenous infusion. Vutrisiran received FDA approval after positive data from a Phase 3 clinical trial (HELIOS-A) showing that vitrusiran significantly improved the signs and symptoms of polyneuropathy (Alnylam Press Release in Businesswire, Alnylam Announces FDA Approval of Amvuttra^{™} (vitrusiran), an RNAi Therapeutic for the Treatment of the Polyneuropathy of Hereditary Transthyretin-Mediated Amyloidosis in Adults, June 2022).

Accordingly, the development history of TTR siRNAs shows the importance of the oligomeric compound sequence, types of chemical modifications, patterns of chemical modifications and delivery system of oligomeric compounds.

Disclosed herein are thoughtfully designed chemical modification motifs, Advanced RNA Targeting (ARNATAR), for oligomeric compounds. Bound by no particular theory, the chemical modification motifs, irrespective of sequence, increase stability, specificity, safety and potency of oligomeric compounds. The specific chemical modification motifs of the invention, used in conjunction with sequence selection for targets and delivery modality make potent oligmeric therapeutic compounds.

Without being bound by any particular theory, the ARNATAR designed oligomeric compounds disclosed herein optimize chemical modifications motifs for characteristics such as duplex annealing temperature, RISC loading speed, stability, specificity, safety and potency.

In certain embodiments, ARNATAR designed oligomeric compounds with lower annealing temperature and faster RISC loading speed can produce a faster acting therapeutic compound beneficial for acute diseases which require fast relief after administration.

In certain embodiments, ARNATAR designed oligomeric compounds produce more stable and durable therapeutic compounds allowing longer lasting benefits for acute and chronic diseases and/or less frequent dosing of the therapeutic compound.

In certain embodiments, ARNATAR designed oligomeric compounds have enhanced binding irrespective of the oligomeric compound sequence based on the modification motif. This enhanced binding to a target nucleic acid allows the production of a therapeutic compound with enhanced specificity and safety due to fewer off-target effects. Additionally, the ARNATAR motifs have been designed to be less toxic and safer for a therapeutic compound.

In certain embodiments, ARNATAR designed oligomeric compounds are designed to be very potent inhibitors of a target nucleic acid. This potency allows less of the oligomeric compound to be administered to be therapeutically effective, leading to less general toxicity of any oligomeric compound after administration. Also, less compound required for a therapeutic dose, decreases manufacturing costs.

In certain embodiments, ARNATAR designed oligomeric compounds are very potent inhibitors of a target nucleic acid. The high potency allows target nucleic acid reduction in tissues other than liver.

Accordingly, there is a need for improved oligomeric compounds to treat diseases. ARNATAR oligomeric compounds have been designed to improve speed, stability, specificity, safety and potency in order to produce improved therapeutic compounds.

### EXAMPLES

While certain compounds and compositions described herein have been described with specificity in accordance with certain embodiments, the following examples serve only to illustrate the compounds described herein and are not intended to limit the same.

When the tables below show both an unmodified sequence ("sequence") and a modified sequence ("Sequence + Chemistry") in the same row, the respective SEQ ID NO applies to the modified sequence. For example, SEQ ID NO: 16 in Table 3 stands for the modified sequence mG*mC*mGmUmCmAfCmCfAfAfAmAmAmGmCmGmCmAmA*T*T.

### EXAMPLE 1: DESIGNING LMNA siRNA WITH VARYING SENSE STRAND MODIFICATIONS

Laminin (LMNA) was selected as a target for testing various Advanced RNA Targeting (ARNATAR) designs. A laminin (LMNA) region with a conserved sequence between human and mouse was identified and a 19 nt long sequence (SEQ ID NO: 1) in this homology region was targeted by oligomeric compounds.

**Table 1: LMNA Target Sequences**

| Name | Sense or Antisense | Sequence (5' to 3') | SEQ ID NO: |
|---|---|---|---|
| LMNA DNA Target Sequence | Sense | GCGTCACCAAAAAGCGCAA | 1 |
| LMNA RNA Target Sequence | Sense | GCGUCACCAAAAAGCGCAA | 2 |

siRNAs were designed to target the LMNA mRNA target sequence (SEQ ID NO: 2) and comprise a 2-nucleotide overhang at the 3' end of the strand: either TT (SEQ ID NOs: 3-4) or UU (SEQ ID NO: 5). All antisense strands have a 5'-phosphate. A notation is made before each nucleotide indicating the type of chemical modification, if any, made to the nucleotide. If no modification notation is made before a letter designating a nucleotide, the nucleotide is a deoxyribonucleotide. Notations for the chemical modifications to the strands can be found as follows:
(5p) = 5'-phosphate
r = ribonucleotide (e.g., rA indicates an adenosine)
d (or no notation made before a nucleotide) = deoxyribonucleotide which has been substituted for a ribonucleotide (e.g., dA or A indicates a 2'-deoxy adenosine)
f = 2'-F (i.e., indicating a 2'-fluoro-modified nucleoside, e.g., fA indicates a 2'-fluoro adenosine)
m = 2'-OMe (i.e., indicating a 2'-O-methyl-modified nucleoside, e.g., mA indicates a 2'-O-methyl adenosine)
gna = Glycol nucleic acid (e.g., gnaT indicates a 2,3-dihydroxypropyl thymine)
p = phosphate
* = phosphorothioate (PS) linkage (i.e., indicates that a 5'-phosphorothioate (= 5'-thiophosphate) is present instead of a 5'-phosphate, e.g., *A indicates a 2'-deoxy adenosine 5'-thiophosphate, *rA indicates an adenosine 5'-thiophosphate and *mA indicates a 2'-O-methyl adenosine 5'-thiophosphate)
GA = GalNAc (GA1 = GalNAc1, GA2 = GalNAc2 and GA3 = GalNAc3 are specific GalNAc conjugates described herein)

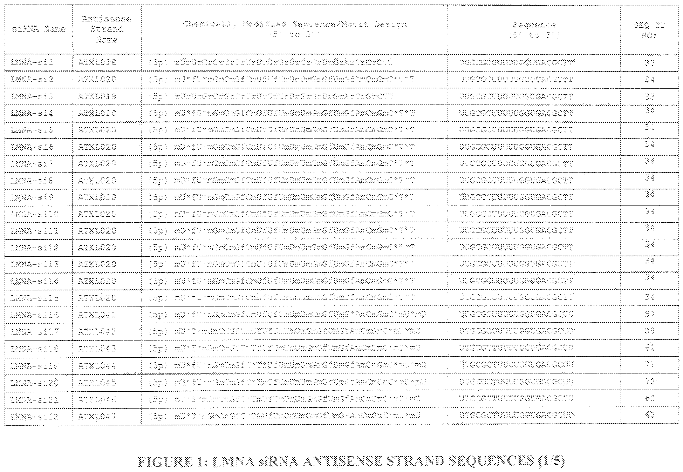

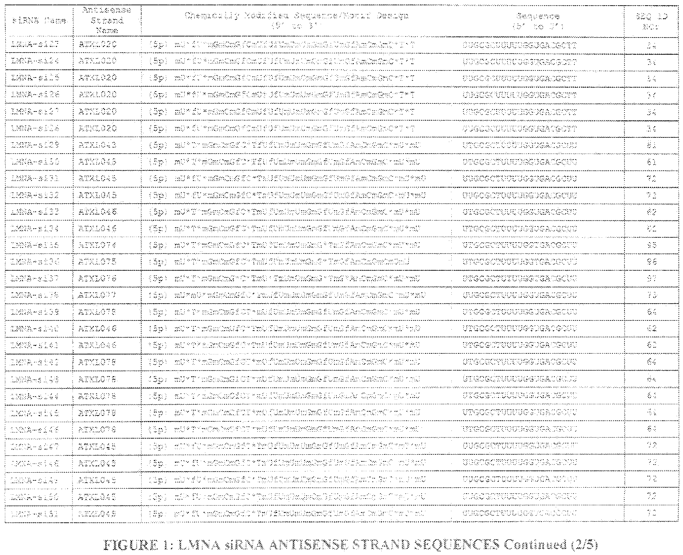

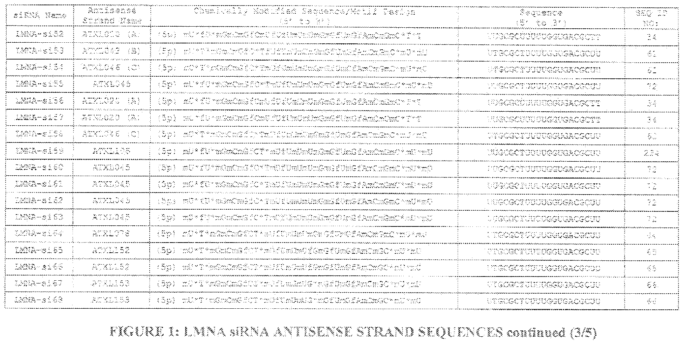

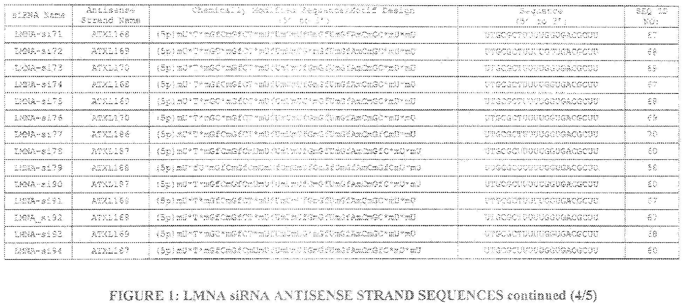

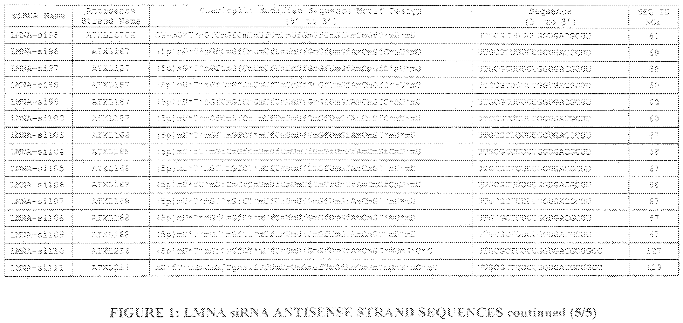

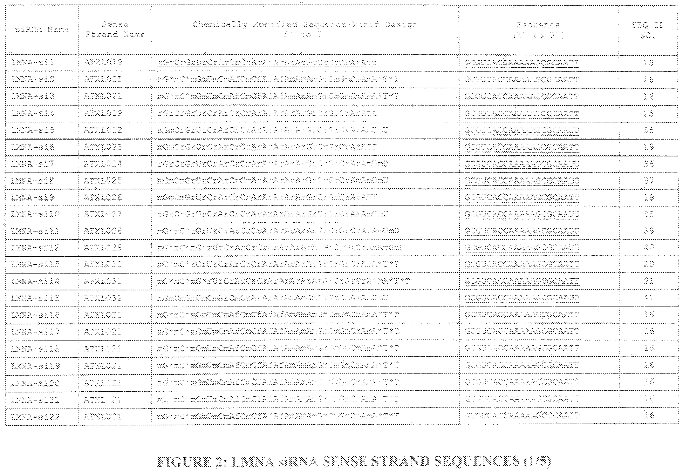

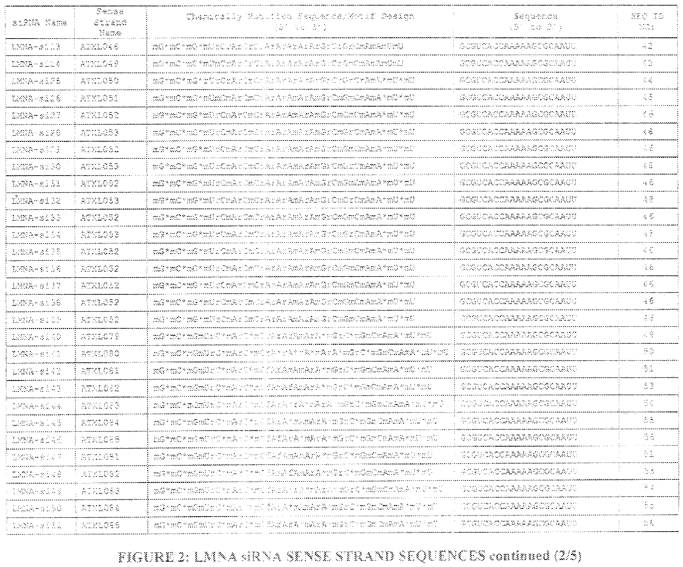

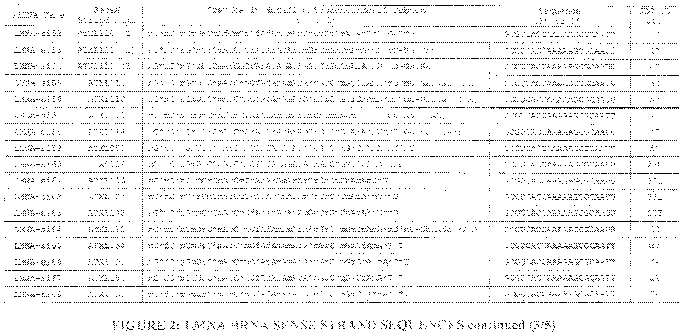

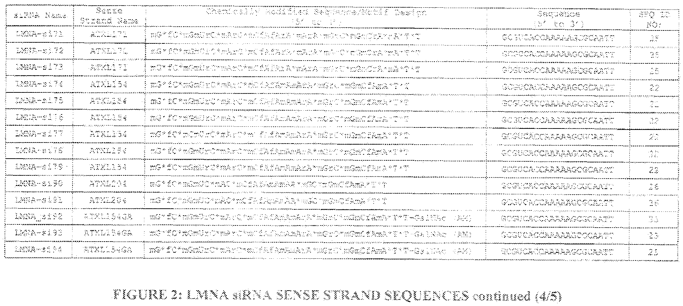

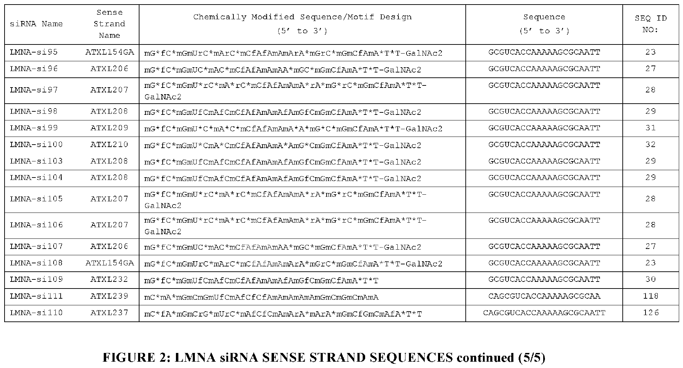

A listing of LMNA siRNAs sequences can be found in the tables, *infra,* or at Figures 1-2.

Cell cultures were grown to approximately 60-80% confluency before varying doses of siRNA were transfected into cells using RNAiMAX (InVitrogen, Waltham, MA) according to the manufacturer's recommended protocol and the cells further cultured for a period of time. siRNA activity was determined by measuring the levels of target mRNA through qRT-PCR using the LMNA primer probe set listed in Table 2. qRT-PCR was performed using AgPath-ID^{™} One-Step RT-PCR Reagents in QS3 real-time PCR system (ThermoFisher Scientific, Waltham, MA, USA). The target RNA levels detected in qRT-PCR assay were normalized to either total RNA levels measured with Ribogreen^{™} (ThermoFisher Scientific, Waltham, MA, USA) or GAPDH mRNA levels detected in the aliquotes of RNA samples using qRT-PCR.

**Table 2: human and mouse LMNA Primer-Probe Sets**

| Primer Name | Primer Sequence (5' to 3') | SEQ ID NO: |
|---|---|---|
| hsLMNA-S | CGGGTGGATGCTGAGAAC | 3 |
| hsLMNA-A | TGCTTCCCATTGTCAATCTCC | 4 |
| hsLMNA-P | AGTGAGGAGCTGCGTGAGACCAA | 5 |
| msLMNA-S | GGACCAGGTGGAACAGTATAAG | 6 |
| msLMNA-A | TCAATGCGGATTCGAGACTG | 7 |
| msLMNA-P | CAGCTTGGCGGAGTATGTCTTTTCTAGC | 8 |

### A. Minimal Sense Strand Modifications Improves siRNA Activity

As shown in Table 3, LMNA-si1 is a siRNA with TT overhangs attached to an unmodified ribonucleotide sequence targeting LMNA. LMNA-si2 is a modified siRNA where the chemical modification pattern reflects that of lumasiran, a FDA approved siRNA, with the exception that the sense strand of LMNA-si2 has an additional TT overhang connected by PS linkages and does not have a GalNAc conjugate, while the antisense strand is 21 nts with TT overhang rather than 23 nt long. LMNA-si3 has a modified sense strand and an antisense strand with a TT overhand attached to an unmodified ribonucleotide sequence. LMNA-si4 has a sense strand with a TT overhang attached to an unmodified ribonucleotide sequence and a modified antisense strand. In this example, all antisense strands have a 5'-phosphate.

**Table 3: LMNA Oligomeric Compounds**

| siRNA Name | Strand Name | Sense or Antisense | Sequence + Chemistry* (5' to 3') | Sequence | SEQ ID NO: |
|---|---|---|---|---|---|
| LMNA -si1 | ATXL 019 | Sense | rGrCrGrUrCrArCrCrArArA rArArGrCrGrCrArATT | GCGUCACCAAAA AGCGCAATT | 15 |
| | ATXL 018 | Antisense | rUrUrGrCrGrCrUrUrUrU rGrGrUrGrArCrGrCTT | UUGCGCUUUUG GUGACGCTT | 33 |
| LMNA -si2 | ATXL 021 | Sense | mG*mC*mGmUmCmAfC mCfAfAfAmAmAmGmCm GmCmAmA*T*T | GCGUCACCAAAA AGCGCAATT | 16 |
| | ATXL 020 | Antisense | mU*fU*mGmCmGfCmUfU fUmUmUmGmGfUmGfAm CmGmC*T*T | UUGCGCUUUUG GUGACGCTT | 34 |
| LMNA -si3 | ATXL 021 | Sense | mG*mC*mGmUmCmAfC mCfAfAfAmAmAmGmCm GmCmAmA*T*T | GCGUCACCAAAA AGCGCAATT | 16 |
| | ATXL 018 | Antisense | rUrUrGrCrGrCrUrUrUrU rGrGrUrGrArCrGrCTT | UUGCGCUUUUG GUGACGCTT | 33 |
| LMNA -si4 | ATXL 019 | Sense | rGrCrGrUrCrArCrCrArArA rArArGrCrGrCrArATT | GCGUCACCAAAA AGCGCAATT | 15 |
| | ATXL 020 | Antisense | mU*fU*mGmCmGfCmUfU fUmUmUmGmGfUmGfAm CmGmC*T*T | UUGCGCUUUUG GUGACGCTT | 34 |

| | | | | | |
|---|---|---|---|---|---|
| * the oligomeric compounds in this Table do not comprise the motifs claimed | | | | | |

siRNA was transfected at 0-10 nM final concentration into HeLa cells (ATCC, Manassas, VA, USA) with RNAiMAX (Invitrogen, Waltham, MA) and the cells further cultured for 24 hr. siRNA activity was determined by qRT-PCR using the LMNA primer probe set in Table 2 and the half-maximal inhibitory concentration value (IC50) of the siRNAs targeting LMNA was calculated (Table 4).

**Table 4: LMNA siRNA Inhibition in HeLa Cells after 24hrs**

| | LMNA-si1 | LMNA-si2 | LMNA-si3 | LMNA-si4 |
|---|---|---|---|---|
| IC50 (nM) | 0.094 | 5.664 | 12.22 | 0.095 |
| Fold | 60.0 | 1.00 | 0.5 | 59.7 |

LMNA-si2 was used as the benchmark for activity. The results show that LMNA-si1 and LMNA-si4, with minimal modification on the sense strand, exhibited greater potency than LMNA-si2 and LMNA-si3, suggesting that reducing the modifications to the sense strand improves siRNA activity.

### B. Addition of Peripheral Sense Strand Modifications to Protect the siRNA

The previous study, *supra,* indicated that reducing modifications to the siRNA sense strand improved siRNA activity 24 hr after transfection. However, siRNAs need to be stable metabolically to maintain a more durable activity *in vivo.* Some chemical modifications have been able to improve such stability without compromising activity (Choung et al., Biochem Biophys Res Commun, 2006, 342:919-927), therefore, strategic modifications 2'-OMe were made to the 3'and/or 5' end of the sense strand of LMNA-si4 to determine whether the modifications would affect siRNA activity over time. Also, the effect of mUmU or TT overhangs at the 3' ends of the sense strands was assessed. In this example, all antisense strands have a 5'-phosphate.

**Table 5: LMNA Oligomeric Compounds with Sense Strand Peripheral Modifications**

| siRNA Name | Strand Name | Sense or Antisense | Sequence + Chemistry* (5' to 3') | Sequence | SEQ ID NO: |
|---|---|---|---|---|---|
| LMNA -si5 | ATXL 022 | Sense | mGmCrGrUrCrArCrCrArArA rArArGrCrGrCrArAmUmU | GCGUCACCAAA AAGCGCAAUU | 35 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si6 | ATXL 023 | Sense | mGmCrGrUrCrArCrCrArArA rArArGrCrGrCrArATT | GCGUCACCAAA AAGCGCAATT | 19 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si7 | ATXL 024 | Sense | rGrCrGrUrCrArCrCrArArAr ArArGrCrGrCrArAmUmU | GCGUCACCAAA AAGCGCAAUU | 36 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si8 | ATXL 025 | Sense | mGmCmGrUrCrArCrCrArAr ArArArGrCrGrCrAmAmUm U | GCGUCACCAAA AAGCGCAAUU | 37 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| **LMNA** -si9 | ATXL 026 | Sense | mGmCmGrUrCrArCrCrArAr ArArArGrCrGrCrArATT | GCGUCACCAAA AAGCGCAATT | 18 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si10 | ATXL 027 | Sense | rGrCrGrUrCrArCrCrArArAr ArArGrCrGrCrAmAmUmU | GCGUCACCAAA AAGCGCAAUU | 38 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si11 | ATXL 028 | Sense | mG*mC*rGrUrCrArCrCrArA rArArArGrCrGrCrArAmUm U | GCGUCACCAAA AAGCGCAAUU | 39 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si12 | ATXL 029 | Sense | mG*mC*mG*rUrCrArCrCrA rArArArArGrCrGrCrAmAm UmU | GCGUCACCAAA AAGCGCAAUU | 40 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si13 | ATXL 030 | Sense | mG*mC*rGrUrCrArCrCrArA rArArArGrCrGrCrArA*T*T | GCGUCACCAAA AAGCGCAATT | 20 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si14 | ATXL 031 | Sense | mG*mC*mG*rUrCrArCrCrA rArArArArGrCrGrCrA*mA* T*T | GCGUCACCAAA AAGCGCAATT | 21 |
| | ATXL 020 | Antisense | mU*fU*mGmCmGfCmUfUf UmUmUmGmGfUmGfAmC mGmC*T*T | UUGCGCUUUU UGGUGACGCTT | 34 |
| LMNA -si15 | ATXL 032 | Sense | mGmCmGmUmCmArCmCr ArArArAmAmGmCmGmCm AmAmUmU | GCGUCACCAAA AAGCGCAAUU | 41 |
| | ATXL 020 | Antisense | mU*fU*mGmCmGfCmUfUf UmUmUmGmGfUmGfAmC mGmC*T*T | UUGCGCUUUU UGGUGACGCTT | 34 |

| | | | | | |
|---|---|---|---|---|---|
| * the oligomeric compounds in this Table do not comprise the motifs claimed | | | | | |

siRNA was transfected at 0-10 nM into HeLa cells with RNAiMAX (Invitrogen, Waltham, MA) and the cells further cultured for 24 hr. siRNA activity was determined by qRT-PCR using the primer probe set in Table 2 and the IC50 of the siRNAs targeting LMNA was calculated (Table 6).

**Table 6: LMNA siRNA Inhibition in HeLa Cells after 24hrs**

| | IC50 (nM) | Fold |
|---|---|---|
| LMNA-si2 | 5.781 | 1.0 |
| LMNA-si5 | 0.088 | 65.7 |
| LMNA-si6 | 0.039 | 149.1 |
| LMNA-si7 | 0.084 | 68.7 |
| LMNA-si8 | 0.051 | 113.2 |
| LMNA-si9 | 0.075 | 77.4 |
| LMNA-si10 | 0.236 | 24.5 |
| LMNA-si11 | 0.155 | 37.3 |
| LMNA-si12 | 0.188 | 30.8 |
| LMNA-si13 | 0.123 | 47 |
| LMNA-si14 | 0.097 | 59.8 |
| LMNA-si15 | 8.336 | 0.7 |

All the siRNAs with 3' and 5' sense strand modifications were more active than LMNA-si2. However, the addition of more 2'-OMe beyond the 3' and 5' periphery of the sense strand did not contribute to siRNA activity as LMNA-si15, which had most of its sense strand modified, was the least active siRNA. 2'-OMe modifications at the 5' end of the sense strand made the siRNA more active than modification at the 3' end (compare LMNA-si6 and LMNA-si7, LMNA-si9 and LMNA-si10). No PS linkages at the 5' end of the sense strand made the siRNA more active than addition of three PS linkages at the 5' end of the sense strand (compare LMNA-si12 and LMNA-si8). There was no obvious difference between having a mUmU versus a TT overhang to activity.

Selected siRNAs were further tested for a longer time in cell culture and in different cell types: LMNA-si2, LMNA-si8, LMNA-si12 and LMNA-si14.

siRNA was transfected at 0-10 nM into HeLa cells with RNAiMAX (Invitrogen, Waltham, MA) and the cells further cultured for 24 hr and 72 hr. siRNA was also transfected into HEK293 cells (ATCC, Manassas, VA, USA) with RNAiMAX (Invitrogen, Waltham, MA) and the cells further cultured for 24 hr and 120 hr. siRNA activity was determined by qRT-PCR using the primer probe set in Table 2 and the IC50 of the siRNAs targeting LMNA was calculated (Table 7).

**Table 7: LMNA siRNA Inhibition in HeLa or HEK293 Cells**

| | HeLa Cells (24hrs) | | HeLa Cells (72 hrs) | | HEK293 Cells (24 hrs) | | HEK293 Cells (120 hrs) | |
|---|---|---|---|---|---|---|---|---|
| siRNA | IC50 (nM) | Fold | IC50 (nM) | Fold | IC50 (nM) | Fold | IC50 (nM) | Fold |
| LMNA-si2 | 0.411 | 1.00 | 0.182 | 1 | 78.93 | 1.00 | 0.631 | 1 |
| LMNA-si8 | 0.027 | 15.4 | 0.029 | 6.3 | 0.595 | 132.7 | 0.191 | 3.3 |
| LMNA-si12 | 0.022 | 18.8 | 0.025 | 7.4 | 1.132 | 69.7 | 0.03 | 21.1 |
| LMNA-si14 | 0.026 | 16 | 0.029 | 6.3 | 1.799 | 43.9 | 0.036 | 17.6 |

Tables 6-7 show that siRNAs with modifications in the sense strand can increase siRNA activity in different cell types for various time periods.

### C. Additional Sense Strand Modifications to Protect the siRNA

The previous study, *supra,* indicated that peripheral modifications to the siRNA sense strand improved siRNA activity. Additional strategic 2'-OMe modifications were made to the sense strand of LMNA-si4 to determine whether the additional modifications would affect siRNA activity over time (Table 8) with the objective to increase 2'-OMe modifications in the sense strand in order to increase stability, but not compromise on potency. In this example, all antisense strands have a 5'-phosphate.

**Table 8: LMNA Oligomeric Compounds with Additional 2'-OMe Sense Strand Modifications**

| siRNA Name | Strand Name | Sense or Antisense | Sequence + Chemistry* (5' to 3') | Sequence | SEQ ID NO: |
|---|---|---|---|---|---|
| LMNA -si23 | ATXL048 | Sense | mG*mC*mG*mUrCrArCr CrArArArArArGrCrGrCm AmAmUmU | GCGUCACCAAA AAGCGCAAUU | 42 |
| | ATXL020 | Antisense | mU*fU*mGmCmGfCmUf UfUmUmUmGmGfUmGf AmCmGmC*T*T | UUGCGCUUUUU GGUGACGCTT | 34 |
| LMNA -si24 | ATXL049 | Sense | mG*mC*mG*mUmCrAr CrCrArArArArArGrCrGm CmAmAmUmU | GCGUCACCAAA AAGCGCAAUU | 43 |
| | ATXL020 | Antisense | mU*fU*mGmCmGfCmUf UfUmUmUmGmGfUmGf AmCmGmC*T*T | UUGCGCUUUUU GGUGACGCTT | 34 |
| LMNA -si25 | ATXL050 | Sense | mG*mC*mG*rUrCrArCr CrArArArArArGrCrGrCr AmA*mU*mU | GCGUCACCAAA AAGCGCAAUU | 44 |
| | ATXL020 | Antisense | mU*fU*mGmCmGfCmUf UfUmUmUmGmGfUmGf AmCmGmC*T*T | UUGCGCUUUUU GGUGACGCTT | 34 |
| LMNA -si26 | ATXL051 | Sense | mG*mC*mG*mUmCmAr CmCrArArAmArAmGrC mGmCmAmA*mU*mU | GCGUCACCAAA AAGCGCAAUU | 45 |
| | ATXL020 | Antisense | mU*fU*mGmCmGfCmUf UfUmUmUmGmGfUmGf AmCmGmC*T*T | UUGCGCUUUUU GGUGACGCTT | 34 |
| LMNA -si27 | ATXL052 | Sense | mG*mC*mG*mUrCmAr CmCrArArAmArAmGrC mGmCmAmA*mU*mU | GCGUCACCAAA AAGCGCAAUU | 46 |
| | ATXL020 | Antisense | mU*fU*mGmCmGfCmUf UfUmUmUmGmGfUmGf AmCmGmC*T*T | UUGCGCUUUUU GGUGACGCTT | 34 |
| LMNA -si28 | ATXL053 | Sense | mG*mC*mG*mUrCmAr CmCrArArAmArAmGrC mGrCmAmA*mU*mU | GCGUCACCAAA AAGCGCAAUU | 48 |
| | ATXL020 | Antisense | mU*fU*mGmCmGfCmUf UfUmUmUmGmGfUmGf AmCmGmC*T*T | UUGCGCUUUUU GGUGACGCTT | 34 |

| | | | | | |
|---|---|---|---|---|---|
| * the oligomeric compounds in this Table do not comprise the motifs claimed | | | | | |

siRNA was transfected at 0-10 nM into HeLa cells with RNAiMAX (Invitrogen, Waltham, MA) and the cells further cultured for 24 hr. siRNA was transfected at 0-10 nM into HEK293 cells with RNAiMAX (Invitrogen, Waltham, MA) and the cells further cultured for 48 hr. siRNA activity was determined by qRT-PCR using the primer probe set in Table 2 and the IC50 of the siRNAs targeting LMNA was calculated (Table 9).

**Table 9: LMNA siRNA Inhibition in HeLa or HEK293 Cells**

| | HeLa Cells (24 hrs) | | HEK293 Cells (48 hrs) | |
|---|---|---|---|---|
| siRNA | IC50 (nM) | Fold | IC50 (nM) | Fold |
| LMNA-si2 | 9.287 | 1 | 1.088 | 1 |
| LMNA-si14 | 0.194 | 47.8 | 0.087 | 12.6 |
| LMNA-si23 | 0.366 | 25.4 | 0.08 | 13.7 |
| LMNA-si24 | 0.287 | 32.4 | 0.1 | 10.9 |
| LMNA-si25 | 0.320 | 29.0 | 0.096 | 11.3 |
| LMNA-si26 | 1.668 | 5.6 | 0.407 | 2.7 |
| LMNA-si27 | 0.846 | 11.0 | 0.053 | 20.4 |
| LMNA-si28 | 0.386 | 24.1 | 0.110 | 9.9 |

A subset of siRNAs with robust activity and more 2'-OMe modified nucleotides in the sense strand was further tested for activity: LMNA-si27 (sense strand ATXL052), LMNA-si28 (sense strand ATXL053).

siRNA was transfected at 0-10 nM into HeLa cells with RNAiMAX (Invitrogen, Waltham, MA) and the cells further cultured for 24 hr or 72 hr. siRNA was transfected at 0-10 nM into HEK293 cells with RNAiMAX (Invitrogen, Waltham, MA) and the cells further cultured for 72 hr. siRNA activity was determined by qRT-PCR using the primer probe set in Table 2 and the IC50 of the siRNAs targeting LMNA was calculated (Table 10).

**Table 10: LMNA siRNA Inhibition in HeLa or HEK293 Cells**

| | HeLa Cells (24 hrs) | | HeLa Cells (72 hrs) | | HEK293 Cells (72 hrs) | |
|---|---|---|---|---|---|---|
| siRNA | IC50 (nM) | Fold | IC50 (nM) | Fold | IC50 (nM) | Fold |
| LMNA-si2 | 3.228 | 1.0 | 0.431 | 1.0 | 1.415 | 1.0 |
| LMNA-si12 | 0.228 | 14.1 | 0.073 | 5.9 | 0.364 | 3.9 |
| LMNA-si27 | 0.371 | 8.7 | 0.075 | 5.7 | 0.1 | 14.2 |
| LMNA-si28 | 0.42 | 7.7 | 0.158 | 2.7 | 0.255 | 5.5 |

LMNA-si27 is more potent than LMNA-si28 due to the difference in sense strand modifications.

### EXAMPLE 2: DESIGNING LMNA siRNA WITH VARYING ANTISENSE STRAND MODIFICATIONS

In Example 1, *supra,* siRNA antisense strands were held constant while the sense strands underwent various chemical modifications to find motifs that would convey stability and potency to the siRNAs. In this example, siRNA sense strands are held constant while the antisense strand is variously modified. The modified antisense strand from LMNA-si2 was used as a template from which individual nucleotides were modified. Nucleotide positions are counted starting from the 5' end of strand; the 5' most nucleotide is position 1.

Without being bound by any particular theories, it has been previously demonstrated that 2'-F modification increase the melting temperature (Tm) of RNA duplex more than 2'-OMe does (Bramsen and Kjems, 2012, Frontiers in Genetics, 3(154):1-22), and deoxyribonucleotide that further reduce Tm as compared with 2'-OMe at certain positions can be tolerated by RISC (Ui-Tei, et al., Nucl Acids Res, 2008, 36(7): 2136-51). We thus altered the number of 2'-F modifications and incorporated deoxyribonucleotide at certain positions, and evaluated the effect of such modification patterns on siRNA activity.

As shown in Table 11, in some antisense strands: nucleotide 2 is modified from a ribonucleotide with a 2'-F modification to a deoxyribonucleotide; nucleotide 7 is modified from a ribonucleotide with a 2'-OMe modification to a deoxyribonucleotide; the 2'-F modification at nucleotide 8 is changed to a 2'-OMe modification; nucleotide 16 is modified from a ribonucleotide with a 2'-F modification to a deoxyribonucleotide; PS linkages added to link the deoxyribonucleotides to some adjacent ribonucleotides to determine whether the PS linkage is protective of deoxyribonucleotide; and the TT overhang is replaced with a mUmU overhang at the 3' end of the antisense strand.

**Table 11: LMNA Oligomeric Compounds with Antisense Strand Modifications**

| siRNA Name | Strand Name | Sense or Antisense | Sequence + Chemistry* (5' to 3') | Sequence | SEQ ID NO: |
|---|---|---|---|---|---|
| LMN A-si2 | ATXL021 | Sense | mG*mC*mGmUmCmAfCmCfAfAf AmAmAmGmCmGmCmAmA*T*T | GCGUCACCAAA AAGCGCAATT | 16 |
| | ATXL020 | Antisense | mU*fU*mGmCmGfCmUfUfUmU mUmGmGfUmGfAmCmGmC*T*T | UUGCGCUUUUU GGUGACGCTT | 34 |
| LMN A-si16 | ATXL021 | Sense | mG*mC*mGmUmCmAfCmCfAfAf AmAmAmGmCmGmCmAmA*T*T | GCGUCACCAAA AAGCGCAATT | 16 |
| | ATXL041 | Antisense | mU*fU*mGmCmGfCmUfUfUmU mUmGmGfUmG*AmCmGmC*mU *mU | UUGCGCUUUUU GGUGACGCUU | 57 |
| LMN A-si17 | ATXL021 | Sense | mG*mC*mGmUmCmAfCmCfAfAf AmAmAmGmCmGmCmAmA*T*T | GCGUCACCAAA AAGCGCAATT | 16 |
| | ATXL042 | Antisense | mU*T*mGmCmGfCmUfUfUmUm UmGmGfUmGfAmCmGmC*mU* mU | UTGCGCUUUUU GGUGACGCUU | 59 |
| LMN A-si18 | ATXL021 | Sense | mG*mC*mGmUmCmAfCmCfAfAf AmAmAmGmCmGmCmAmA*T*T | GCGUCACCAAA AAGCGCAATT | 16 |
| | ATXL043 | Antisense | mU*T*mGmCmGfC*TfUfUmUmU mGmGfUmGfAmCmGmC*mU*m U | UTGCGCTUUUU GGUGACGCUU | 61 |
| LMN A-si19 | ATXL021 | Sense | mG*mC*mGmUmCmAfCmCfAfAf AmAmAmGmCmGmCmAmA*T*T | GCGUCACCAAA AAGCGCAATT | 16 |
| | ATXL044 | Antisense | mU*fU*mGmCmGfC*TfUfUmUm UmGmGfUmGfAmCmGmC*mU* mU | UUGCGCTUUUU GGUGACGCUU | 71 |
| LMN A-si20 | ATXL021 | Sense | mG*mC*mGmUmCmAfCmCfAfAf AmAmAmGmCmGmCmAmA*T*T | GCGUCACCAAA AAGCGCAATT | 16 |
| | ATXL045 | Antisense | mU*fU*mGmCmGfC*TmUfUmU mUmGmGfUmGfAmCmGmC*mU *mU | UUGCGCTUUUU GGUGACGCUU | 72 |
| LMN A-si21 | ATXL021 | Sense | mG*mC*mGmUmCmAfCmCfAfAf AmAmAmGmCmGmCmAmA*T*T | GCGUCACCAAA AAGCGCAATT | 16 |
| | ATXL046 | Antisense | mU*T*mGmCmGfC*TmUfUmUm UmGmGfUmGfAmCmGmC*mU* mU | UTGCGCTUUUU GGUGACGCUU | 62 |
| LMN A-si22 | ATXL021 | Sense | mG*mC*mGmUmCmAfCmCfAfAf AmAmAmGmCmGmCmAmA*T*T | GCGUCACCAAA AAGCGCAATT | 16 |
| | ATXL047 | Antisense | mU*T*mGmCmGfC*TmUfUmUm UmGmGfUmG*AmCmGmC*mU* mU | UTGCGCTUUUU GGUGACGCUU | 63 |

| | | | | | |
|---|---|---|---|---|---|
| * the oligomeric compounds in this Table do not comprise the motifs claimed | | | | | |

In two sets of experiments, siRNA was transfected at 0-10 nM into HeLa cells with RNAiMAX (Invitrogen, Waltham, MA) and the cells further cultured for 24 hr. siRNA activity was determined by qRT-PCR using the primer probe set in Table 2 and the IC50 of the siRNAs targeting LMNA was calculated (Table 12).

**Table 12: LMNA siRNA Inhibition in HeLa Cells**

| | HeLa Cells (24 hrs) | |
|---|---|---|
| siRNA | IC50 (nM) | Fold |
| LMNA-si2 | 12.32 | 1.0 |
| LMNA-si17 | 186.9 | 0.07 |
| LMNA-si18 | 0.529 | 23.3 |
| LMNA-si20 | 0.208 | 59.1 |
| LMNA-si21 | 0.546 | 22.5 |
| LMNA-si22 | 1.129 | 10.9 |
| LMNA-si2 | 1.961 | 1.0 |
| LMNA-si16 | 1.875 | 1.0 |
| LNMA-si19 | 0.086 | 22.9 |

The results at 24 hrs show changing the nucleotide in position 7 to a deoxyribonucleotide improved activity of LNMA-si18, LMNA-si19, LMNA-si20, LMNA-si21 and LMNA-si22. The change to a deoxyribonucleotide at position 2 or position 16 was tolerated as shown by the activity of LMNA-si16, LMNA-si17, LMNA-si18, LMNA-si21 and LMNA-si22.

In two sets of experiments, siRNA was transfected at 0-10 nM into HeLa cells with RNAiMAX (Invitrogen, Waltham, MA) and the cells further cultured for 72 hr. siRNA activity was determined by qRT-PCR using the primer probe set in Table 2 and the IC50 of the siRNAs targeting LMNA was calculated (Table 13).

**Table 13: LMNA siRNA Inhibition in Hela Cells**

| | HeLa Cells (72 hrs) | |
|---|---|---|
| siRNA | IC50 (nM) | Fold |
| LMNA-si2 | 0.154 | 1.0 |
| LMNA-si17 | 0.59 | 0.3 |
| LMNA-si18 | 0.068 | 2.3 |
| LMNA-si20 | 0.033 | 4.7 |
| LMNA-si21 | 0.059 | 2.6 |
| LMNA-si22 | 0.115 | 1.3 |
| LMNA-si2 | 0.067 | 1.0 |
| LMNA-si16 | 0.296 | 0.2 |
| LMNA-si19 | 0.028 | 2.4 |

At the 72 hr timepoint, LMNA-si20 and LMNA-si21 showed the best activity.

### EXAMPLE 3: DESIGNING LMNA siRNA WITH VARYING SENSE AND ANTISENSE STRAND MODIFICATIONS

Modified sense and antisense strands disclosed in the previous examples were mixed and matched to form new siRNA compounds (Table 14) and assessed for stability and activity (Table 15).

**Table 14: LMNA Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| LMNA-si29 | ATXL052 | Sense | mG*mC*mG*mUrCmAr CmCrArArAmArAmGrC mGmCmAmA*mU*mU | GCGUCACCAAA AAGCGCAAUU | 46 |
| | ATXL043 | Antisense | mU*T*mGmCmGfC*TfU fUmUmUmGmGfUmGfA mCmGmC*mU*mU | UTGCGCTUUUU GGUGACGCUU | 61 |
| LMNA-si30 | ATXL053 | Sense | mG*mC*mG*mUrCmAr CmCrArArAmArAmGrC mGrCmAmA*mU*mU | GCGUCACCAAA AAGCGCAAUU | 48 |
| | ATXL043 | Antisense | mU*T*mGmCmGfC*TfU fUmUmUmGmGfUmGfA mCmGmC*mU*mU | UTGCGCTUUUU GGUGACGCUU | 61 |
| LMNA-si31 | ATXL052 | Sense | mG*mC*mG*mUrCmAr CmCrArArAmArAmGrC mGmCmAmA*mU*mU | GCGUCACCAAA AAGCGCAAUU | 46 |
| | ATXL045 | Antisense | mU*fU*mGmCmGfC*T mUfUmUmUmGmGfUm GfAmCmGmC*mU*mU | UUGCGCTUUUU GGUGACGCUU | 72 |
| LMNA-si32 | ATXL053 | Sense | mG*mC*mG*mUrCmAr CmCrArArAmArAmGrC mGrCmAmA*mU*mU | GCGUCACCAAA AAGCGCAAUU | 48 |
| | ATXL045 | Antisense | mU*fU*mGmCmGfC*T mUfUmUmUmGmGfUm GfAmCmGmC*mU*mU | UUGCGCTUUUU GGUGACGCUU | 72 |
| LMNA-si33 | ATXL052 | Sense | mG*mC*mG*mUrCmAr CmCrArArAmArAmGrC mGmCmAmA*mU*mU | GCGUCACCAAA AAGCGCAAUU | 46 |
| | ATXL046 | Antisense | mU*T*mGmCmGfC*Tm UfUmUmUmGmGfUmGf AmCmGmC*mU*mU | UTGCGCTUUUU GGUGACGCUU | 62 |
| LMNA-si34 | ATXL053 | Sense | mG*mC*mG*mUrCmAr CmCrArArAmArAmGrC mGrCmAmA*mU*mU | GCGUCACCAAA AAGCGCAAUU | 48 |
| | ATXL046 | Antisense | mU*T*mGmCmGfC*Tm UfUmUmUmGmGfUmGf AmCmGmC*mU*mU | UTGCGCTUUUU GGUGACGCUU | 62 |

| | | | | | |
|---|---|---|---|---|---|
| * the oligomeric compounds in this Table do not comprise the motifs claimed | | | | | |

siRNA was transfected at 0-10 nM into HeLa cells with RNAiMAX (Invitrogen, Waltham, MA) and the cells further cultured for 24 hr, 96 hr, or 144 hr. siRNA activity was determined by qRT-PCR using the primer probe set in Table 2 and the IC50 of the siRNAs targeting LMNA was calculated (Table 15).

**Table 15: LMNA siRNA Inhibition in HeLa Cells**

| | HeLa Cells (24 hrs) | | HeLa Cells (96 hrs) | | HeLa Cells (144 hrs) | |
|---|---|---|---|---|---|---|
| siRNA | IC50 (nM) | Fold | IC50 (nM) | Fold | IC50 (nM) | Fold |
| LMNA-si2 | 1.205 | 1.0 | 0.39 | 1.0 | 0.7 | 1.0 |
| LMNA-si1 | 0.131 | 9.2 | 0.125 | 3.1 | 0.625 | 1.1 |
| LMNA-si29 | 0.018 | 68.1 | 0.053 | 7.4 | 0.135 | 5.2 |
| LMNA-si30 | 0.029 | 41.0 | 0.098 | 4.0 | 0.244 | 2.9 |
| LMNA-si31 | 0.021 | 57.5 | 0.036 | 10.8 | 0.075 | 9.4 |
| LMNA-si32 | 0.031 | 39.1 | 0.036 | 11.0 | 0.081 | 8.6 |
| LMNA-si33 | 0.023 | 51.7 | 0.042 | 9.3 | 0.045 | 15.5 |
| LMNA-si34 | 0.043 | 27.9 | 0.065 | 6.0 | 0.137 | 5.1 |

Sense strand ATXL052 was more potent than ATXL053 when used in a siRNA compound. Antisense strands ATXL045 and ATXL046 were more potent than ATXL043. LMNA-si31 and LMNA-si33 had the best activity over time.

siRNA was transfected at 0-10 nM into HEK293 cells with RNAiMAX (Invitrogen, Waltham, MA) and the cells further cultured for 96 hr. siRNA activity was determined by qRT-PCR using the primer probe set in Table 2 and the IC50 of the siRNAs targeting LMNA was calculated (Table 16).

**Table 16: LMNA siRNA Inhibition in HEK293 Cells**

| | HEK293 Cells (96 hrs) | |
|---|---|---|
| siRNA | IC50 (nM) | Fold |
| LMNA-si2 | 0.528 | 1 |
| LMNA-si1 | 0.169 | 3.1 |
| LMNA-si29 | 0.077 | 6.8 |
| LMNA-si30 | 0.134 | 3.9 |
| LMNA-si31 | 0.027 | 19.2 |
| LMNA-si32 | 0.08 | 6.6 |
| LMNA-si33 | 0.054 | 9.7 |
| LMNA-si34 | 0.174 | 3.0 |

Similar to the results in HeLa cells, the sense strand ATXL052 is more active than ATXL053. LMNA-si31 and LMNA-si33 are more active than others.

siRNA was transfected at 0-10 nM into murine HePa1-6 cells (ATCC, Manassas, VA, USA) with RNAiMAX (Invitrogen, Waltham, MA) and the cells further cultured for 24 hr, 72 hr, or 120 hr. siRNA activity was determined by qRT-PCR using the primer probe set in Table 2 and the IC50 of the siRNAs targeting LMNA was calculated (Table 17).

**Table 17: LMNA siRNA Inhibition in Mouse HePa1-6 Cells**

| | HePa1-6 Cells (24 hrs) | | HePa1-6 Cells (72 hrs) | | HePa1-6 Cells (120 hrs) | |
|---|---|---|---|---|---|---|
| siRNA | IC50 (nM) | Fold | IC50 (nM) | Fold | IC50 (nM) | Fold |
| LMNA-si2 | 8.278 | 1.0 | 0.593 | 1.0 | 0.562 | 1.0 |
| LMNA-si29 | 0.496 | 16.7 | 0.219 | 2.7 | 0.622 | 0.9 |
| LMNA-si31 | 0.103 | 80.1 | 0.078 | 7.6 | 0.072 | 7.8 |
| LMNA-si33 | 0.557 | 14.9 | 0.123 | 4.8 | 0.756 | 0.7 |

LMNA-si31 was found to be more potent than the other compounds in mouse cells. LMNA-si29 and LMNA-si33 were more potent than the benchmark LMNA-si2.

### Serum Stability Assay

LMNA-si2, LMNA-si29 and LMNA-si33 were exposed to human serum to test for stability. siRNAs were incubated with human serum (Sigma-Aldrich, St. Louis, MO, USA) at 0.4uM concentration, at 37°C for a desired time (0, 8, 16, and 24 h). Then BlueJuice^{™} gel loading buffer (ThermoFisher Scientific, Waltham, MA, USA) was added and the siRNA mix was loaded onto 4-20% native TBE gel (ThermoFisher Scientific, Waltham, MA, USA). After running the gel, it was stained with 1:10000 SYBR^{™} Gold (ThermoFisher Scientific, Waltham, MA, USA) in water, at room temperature for 10 min and imaged under UV light.

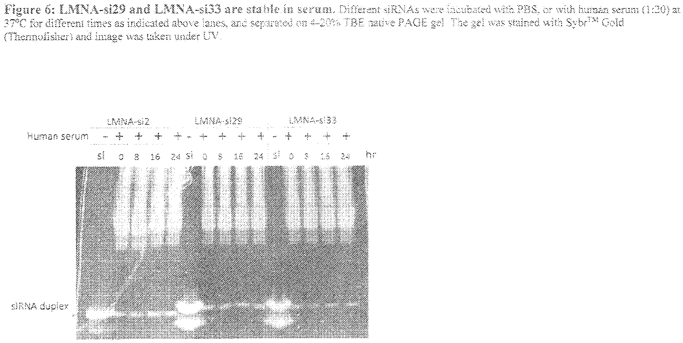

The siRNA compounds were found to be stable after 24 hr serum treatment (Figure 6).

### EXAMPLE 4: DESIGNING ApoC3 siRNA WITH VARYING SENSE AND ANTISENSE STRAND MODIFICATIONS

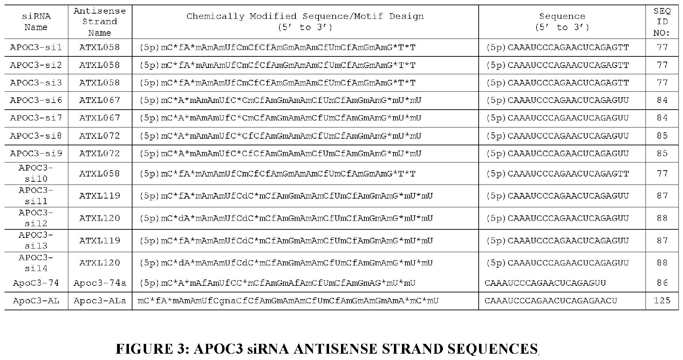

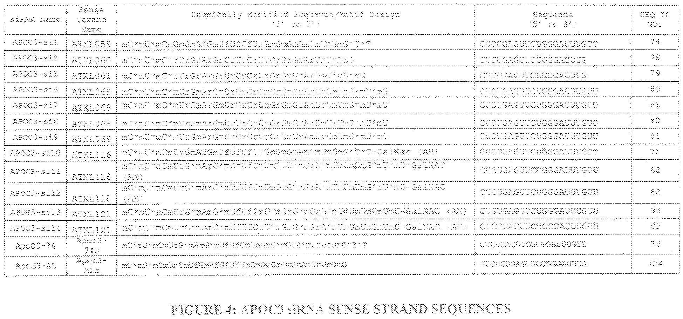

Based on the previous studies, *supra,* with LMNA, some of the chemical modification conferring high activity was assessed in a different target to determine whether the chemical modification motifs would also be beneficial. ApoC3 was chosen as the second test target and siRNAs designed, as shown in Table 18 and Figure 3-4, to target a 19 nt long sequence of human ApoC3: CUCUGAGUUCUGGGAUUUG (SEQ ID NO: 177). The primer probe sets of human ApoC3 used for RT-PCR is shown in Table 19. ApoC3-si1, is a modified siRNA where the chemical modification pattern reflects that of lumasiran with the exception that the sense strand of ApoC3-si1 has an addition TT overhang connected by PS linkages and does not have a GalNAc conjugate, and the antisense strand is 21 nt rather than 23 nt. All antisense strands have a 5'-phosphate. The modified siRNAs were tested for activity in a couple of cell lines.

**Table 18: ApoC3 Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| ApoC3-si1 | ATXL059 | Sense | mC*mU*mCmUmGmAf GmUfUfCfUmGmGmGm AmUmUmUmG*T*T | CUCUGAGUUCU GGGAUUUGTT | 74 |
| | ATXL058 | Antisense | mC*fA*mAmAmUfCmC fCfAmGmAmAmCfUmC fAmGmAmG*T*T | CAAAUCCCAGAA CUCAGAGTT | 77 |
| ApoC3-si2 | ATXL060 | Sense | mC*mU*mC*rUrGrArGr UrUrCrUrGrGrGrArUmU mUmG | CUCUGAGUUCU GGGAUUUG | 78 |
| | ATXL058 | Antisense | mC*fA*mAmAmUfCmC fCfAmGmAmAmCfUmC fAmGmAmG*T*T | CAAAUCCCAGAA CUCAGAGTT | 77 |
| ApoC3-si3 | ATXL061 | Sense | mC*mU*mC*rUrGrArGr UrUrCrUrGrGrGrArUmU *mU*mG | CUCUGAGUUCU GGGAUUUG | 79 |
| | ATXL058 | Antisense | mC*fA*mAmAmUfCmC fCfAmGmAmAmCfUmC fAmGmAmG*T*T | CAAAUCCCAGAA CUCAGAGTT | 77 |
| ApoC3-si6 | ATXL068 | Sense | mC*mU*mC*mUrGmAr GmUrUrCrUmGrGmGrA mUmUmUmG*mU*mU | CUCUGAGUUCU GGGAUUUGUU | 80 |
| | ATXL067 | Antisense | mC*A*mAmAmUfC*Cm CfAmGmAmAmCfUmCf AmGmAmG*mU*mU | CAAAUCCCAGAA CUCAGAGUU | 84 |
| ApoC3-si7 | ATXL069 | Sense | mC*mU*mC*mUrGmAr GmUrUrCrUmGrGmGrA mUrUmUmG*mU*mU | CUCUGAGUUCU GGGAUUUGUU | 81 |
| | ATXL067 | Antisense | mC*A*mAmAmUfC*Cm CfAmGmAmAmCfUmCf AmGmAmG*mU*mU | CAAAUCCCAGAA CUCAGAGUU | 84 |
| ApoC3-si8 | ATXL068 | Sense | mC*mU*mC*mUrGmAr GmUrUrCrUmGrGmGrA mUmUmUmG*mU*mU | CUCUGAGUUCU GGGAUUUGUU | 80 |
| | ATXL072 | Antisense | mC*A*mAmAmUfC*Cf CfAmGmAmAmCfUmCf AmGmAmG*mU*mU | CAAAUCCCAGAA CUCAGAGUU | 85 |
| ApoC3-si9 | ATXL069 | Sense | mC*mU*mC*mUrGmAr GmUrUrCrUmGrGmGrA mUrUmUmG*mU*mU | CUCUGAGUUCU GGGAUUUGUU | 81 |
| | ATXL072 | Antisense | mC*A*mAmAmUfC*Cf CfAmGmAmAmCfUmCf AmGmAmG*mU*mU | CAAAUCCCAGAA CUCAGAGUU | 85 |

| | | | | | |
|---|---|---|---|---|---|
| * the oligomeric compounds in this Table do not comprise the motifs claimed | | | | | |

**Table 19: ApoC3 primer probe set sequences**

| Name | Sequence | SEQ ID NO: |
|---|---|---|
| ApoC3-F | AGGGTTACATGAAGCACGC | 9 |
| ApoC3-R | AGAGAACTTGTCCTTAACGGTG | 10 |
| ApoC3-P | AGGGAACTGAAGCCATCGGTCAC | 11 |

siRNA was transfected at 0-10 nM into Hep3B cells (ATCC, Manassas, VA, USA) with RNAiMAX (Invitrogen, Waltham, MA) and the cells further cultured for 20 hr, 60 hr, or 168 hr. siRNA activity was determined by measuring the levels of target mRNA through qRT-PCR using the primer probe set in Table 19 and the IC50 of the siRNAs targeting ApoC3 was calculated (Table 20). qRT-PCR was performed using AgPath-ID^{™} One-Step RT-PCR Reagents in QS3 real-time PCR system (ThermoFisher Scientific, Waltham, MA, USA). The target RNA levels detected in qRT-PCR assay were normalized to either total RNA levels measured with Ribogreen^{™} (ThermoFisher Scientific, Waltham, MA, USA) or GAPDH mRNA levels detected in the aliquots of RNA samples using qRT-PCR.

**Table 20: ApoC3 siRNA Inhibition in Hep3B Cells**

| | Hep3B Cells (20 hrs) | | Hep3B Cells (60 hrs) | | Hep3B Cells (168 hrs) | |
|---|---|---|---|---|---|---|
| siRNA | IC50 (nM) | Fold | IC50 (nM) | Fold | IC50 (nM) | Fold |
| ApoC3-si1 | 20.66 | 1 | 0.085 | 1 | 0.305 | 1 |
| ApoC3-si3 | 0.104 | 199.4 | 0.013 | 6.7 | 0.375 | 0.8 |
| ApoC3-si6 | 0.151 | 137.3 | 0.014 | 6 | 0.132 | 2.3 |
| ApoC3-si7 | 0.134 | 153.9 | 0.016 | 5.2 | 0.054 | 5.6 |
| ApoC3-si8 | 0.068 | 305.4 | 0.008 | 10.1 | 0.072 | 4.2 |
| ApoC3-si9 | 0.143 | 144.5 | 0.019 | 4.6 | 0.133 | 2.3 |

siRNA was transfected at 0-10 nM into HepG2 cells with RNAiMAX (Invitrogen, Waltham, MA) and the cells further cultured for 24 hr or 72 hr. siRNA activity was determined by qRT-PCR using the primer probe set in Table 2 and the IC50 of the siRNAs targeting ApoC3 was calculated (Table 21).

**Table 21: ApoC3 siRNA Inhibition in HepG2 Cells**

| | HepG2 Cells (24 hrs) | | HepG2 Cells (72 hrs) | |
|---|---|---|---|---|
| siRNA | IC50 (nM) | Fold | IC50 (nM) | Fold |
| ApoC3-si1 | 13.61 | 1 | 0.907 | 1 |
| ApoC3-si3 | 1.329 | 10.2 | 1.074 | 0.8 |
| ApoC3-si6 | 0.209 | 65.2 | 0.098 | 9.3 |
| ApoC3-si8 | 0.413 | 33 | 0.736 | 1.2 |

The new chemical modification motif initially designed with LMNA targeting siRNAs, when used to modify ApoC3 targeting siRNAs, generally conveyed increased activity irrespective of target or the siRNA sequence.

### EXAMPLE 5: DESIGNING ADDITIONAL LMNA siRNAs WITH VARYING PS LINKAGES AND 2'-F IN SENSE AND ANTISENSE STRANDS

Based on the previous studies, *supra,* with LMNA and ApoC3, showing some patterns of chemical modification confer high activity, additional chemical modification patterns were designed for LMNA and assessed to determine whether the new motifs would also be beneficial. The motif for ATXL046 was the base used to add or change chemical modifications to the antisense strand. Some of the additional modifications that may have been included in the new LMNA siRNAs include substituting more deoxyribonucleotides for ribonucleotides, reducing PS linkages, reducing 2'F, adding more 2'-OMe. The motif for ATXL052 was the base used to add or change chemical modifications to the sense strand. Some of the additional modifications include adding a PS linkage to a non-cleavage site or adding PS linkage to all ribonucleotide. See Table *22, infra.* All antisense strands have a 5'-phosphate.

**Table 22: LMNA Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| LMNA -si33 | ATXL052 | Sense | mG*mC*mG*mUrCmArCm CrArArAmArAmGrCmGmC mAmA*mU*mU | GCGUCACCAAA AAGCGCAAUU | 46 |
| | ATXL046 | Antisense | mU*T*mGmCmGfC*TmUfU mUmUmGmGfUmGfAmCm GmC*mU*mU | UTGCGCTUUUU GGUGACGCUU | 62 |
| LMNA -si35 | ATXL052 | Sense | mG*mC*mG*mUrCmArCm CrArArAmArAmGrCmGmC mAmA*mU*mU | GCGUCACCAAA AAGCGCAAUU | 46 |
| | ATXL074 | Antisense | mU*T*mGmCmGfC*TmUfU mUmUmGmG*TmGfAmCm GmC*mU*mU | UTGCGCTUUUU GGTGACGCUU | 95 |
| LMNA -si36 | ATXL052 | Sense | mG*mC*mG*mUrCmArCm CrArArAmArAmGrCmGrCm AmA*mU*mU | GCGUCACCAAA AAGCGCAAUU | 48 |
| | ATXL075 | Antisense | mU*T*mGmCmGfC*TmUfU mUmUmGmG*TmGfAmCm GmCmUmU | UTGCGCTUUUU GGTGACGCUU | 96 |
| LMNA -si37 | ATXL052 | Sense | mG*mC*mG*mUrCmArCm CrArArAmArAmGrCmGmC mAmA*mU*mU | GCGUCACCAAA AAGCGCAAUU | 46 |
| | ATXL076 | Antisense | mU*T*mGmCmG*C*TmU* TmUmUmGmG*TmG*AmC mGmC*mU*mU | UTGCGCTUTUU GGTGACGCUU | 97 |
| LMNA -si38 | ATXL052 | Sense | mG*mC*mG*mUrCmArCm CrArArAmArAmGrCmGrCm AmA*mU*mU | GCGUCACCAAA AAGCGCAAUU | 48 |
| | ATXL077 | Antisense | mU*mU*mGmCmGfC*TmU fUmUmUmGmGfUmGfAmC mGmC*mU*mU | UUGCGCTUUUU GGUGACGCUU | 73 |
| LMNA -si39 | ATXL052 | Sense | mG*mC*mG*mUrCmArCm CrArArAmArAmGrCmGmC mAmA*mU*mU | GCGUCACCAAA AAGCGCAAUU | 46 |
| | ATXL078 | Antisense | mU*T*mGmCmGfCT*mUfU mUmUmGmGfUmGfAmCm GmC*mU*mU | UTGCGCTUUUU GGUGACGCUU | 64 |
| LMNA -si40 | ATXL079 | Sense | mG*mC*mGmUrC*mArC*m CrArArAmArA*mGrC*mGm CmAmA*mU*mU | GCGUCACCAAA AAGCGCAAUU | 49 |
| | ATXL046 | Antisense | mU*T*mGmCmGfC*TmUfU mUmUmGmGfUmGfAmCm GmC*mU*mU | UTGCGCTUUUU GGUGACGCUU | 62 |
| LMNA -si41 | ATXL080 | Sense | mG*mC*mGmUrC*mArC*m CrA*rA*rA*mArA*mGrC*m GmCmAmA*mU*mU | GCGUCACCAAA AAGCGCAAUU | 50 |
| | ATXL046 | Antisense | mU*T*mGmCmGfC*TmUfU mUmUmGmGfUmGfAmCm GmC*mU*mU | UTGCGCTUUUU GGUGACGCUU | 62 |

| | | | | | |
|---|---|---|---|---|---|
| * the oligomeric compounds in this Table do not comprise the motifs claimed | | | | | |

siRNA was transfected at 0-10 nM into HeLa cells with RNAiMAX (Invitrogen, Waltham, MA) and the cells further cultured for 20 hr. siRNA was transfected at 0-10 nM into HEK293 cells with RNAiMAX (Invitrogen, Waltham, MA) and the cells further cultured for 48 hr. siRNA activity was determined by qRT-PCR using the primer probe set in Table 2 and the IC50 of the siRNAs targeting LMNA was calculated (Table 23).

**Table 23: LMNA siRNA Inhibition in HeLa or HEK293 Cells**

| | HeLa Cells (20 hrs) | | HEK293 Cells (48 hrs) | |
|---|---|---|---|---|
| siRNA | IC50 (nM) | Fold | IC50 (nM) | Fold |
| LMNA-si2 | 4.682 | 1 | 3.243 | 1 |
| LMNA-si31 | 0.098 | 47.5 | 0.181 | 17.9 |
| LMNA-si33 | 0.095 | 49.2 | 0.185 | 17.5 |
| LMNAsi35 | 0.752 | 6.2 | 0.48 | 6.8 |
| LMNAsi36 | 0.216 | 21.7 | 0.316 | 10.3 |
| LMNAsi37 | 100.6 | 0 | 6.294 | 0.5 |
| LMNAsi40 | 0.052 | 90 | 0.082 | 39.6 |
| LMNAsi41 | 9.32 | 0.5 | 9.905 | 0.3 |

The sense strand cleavage site is among the central nucleotides of the sense strand. Adding PS linkages to the central nucleotides of the sense strand can inhibit activity (LMNA-si41), while adding PS linkages outside the central cleavage site can increase activity (LMNA-si40). Introducing two PS deoxyribonucleotides at positions 6 and 7 of antisense strand was not beneficial for activity (see LMNA-si37). Selected siRNAs were further tested in another cell line for a longer time period.

siRNA was transfected at 0-10 nM into Hepa1-6 cells with RNAiMAX (Invitrogen, Waltham, MA) and the cells further cultured for 24 hr or 72 hr. siRNA activity was determined by qRT-PCR using the primer probe set in Table 2 and the IC50 of the siRNAs targeting LMNA was calculated (Table 24).

**Table 24: LMNA siRNA Inhibition in Hepa1-6 Cells**

| | Hepa1-6 Cells (24 hrs) | | Hepa1-6 Cells 72 hrs) | |
|---|---|---|---|---|
| siRNA | IC50 (nM) | Fold | IC50 (nM) | Fold |
| LMNA-si31 | 0.862 | 1 | 0.148 | 1 |
| LMNA-si38 | 96.12 | 0 | 6.403 | 0 |
| LMNA-si39 | 1.381 | 0.6 | 0.224 | 0.7 |
| LMNA-si40 | 2.618 | 0.3 | 0.740 | 0.2 |

In Hepa1-6 cells, LMNA-si39 and LMNA-si31 had similar activity over time, but LMNA-si38 had worse activity and LMNA-si40 had slightly worse activity compared with LMNA-si31.

### EXAMPLE 6: ADDITIONAL MODIFICATIONS TO INCREASE STABILITY OF THE SENSE STRAND

Additional chemical modification patterns were designed for LMNA and assessed to determine whether the new motifs would be beneficial. The motif for ATXL046 was the base used to add or change chemical modifications to the antisense strand. The new modifications varied the amount of PS linkages and added 2'-F to the central nucleotides of the sense strand (Table 25). All antisense strands have a 5'-phosphate.

**Table 25: LMNA Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| LMNA-si42 | ATXL081 | Sense | mG*mC*mGmUrC*mArC* mCfAfAmAmArA*mGrC* mGmCmAmA*mU*mU | GCGUCACCA AAAAGCGCA AUU | 51 |
| | ATXL078 | Antisense | mU*T*mGmCmGfCT*mU fUmUmUmGmGfUmGfAm CmGmC*mU*mU | UTGCGCTUU UUGGUGACG CUU | 64 |
| LMNA-si43 | ATXL082 | Sense | mG*mC*mGmUrC*mArC* mCfAmAfAmArA*mGrC* mGmCmAmA*mU*mU | GCGUCACCA AAAAGCGCA AUU | 53 |
| | ATXL078 | Antisense | mU*T*mGmCmGfCT*mU fUmUmUmGmGfUmGfAm CmGmC*mU*mU | UTGCGCTUU UUGGUGACG CUU | 64 |
| LMNA-si44 | ATXL083 | Sense | mG*mC*mGmUrC*mArC* mCfArA*rA*mArA*mGrC *mGmCmAmA*mU*mU | GCGUCACCA AAAAGCGCA AUU | 54 |
| | ATXL078 | Antisense | mU*T*mGmCmGfCT*mU fUmUmUmGmGfUmGfAm CmGmC*mU*mU | UTGCGCTUU UUGGUGACG CUU | 64 |
| LMNA-si45 | ATXL084 | Sense | mG*mC*mGmUrC*mArC* mCfArA*mAmArA*mGrC *mGmCmAmA*mU*mU | GCGUCACCA AAAAGCGCA AUU | 55 |
| | ATXL078 | Antisense | mU*T*mGmCmGfCT*mU fUmUmUmGmGfUmGfAm CmGmC*mU*mU | UTGCGCTUU UUGGUGACG CUU | 64 |
| LMNA-si46 | ATXL085 | Sense | mG*mC*mGmUrC*mArC* mCfAfArA*mArA*mGrC* mGmCmAmA*mU*mU | GCGUCACCA AAAAGCGCA AUU | 56 |
| | ATXL078 | Antisense | mU*T*mGmCmGfCT*mU fUmUmUmGmGfUmGfAm CmGmC*mU*mU | UTGCGCTUU UUGGUGACG CUU | 64 |
| LMNA-si47 | ATXL081 | Sense | mG*mC*mGmUrC*mArC* mCfAfAmAmArA*mGrC* mGmCmAmA*mU*mU | GCGUCACCA AAAAGCGCA AUU | 51 |
| | ATXL045 | Antisense | mU*fU*mGmCmGfC*Tm UfUmUmUmGmGfUmGfA mCmGmC*mU*mU | UUGCGCTUU UUGGUGACG CUU | 72 |
| LMNA-si48 | ATXL082 | Sense | mG*mC*mGmUrC*mArC* mCfAmAfAmArA*mGrC* mGmCmAmA*mU*mU | GCGUCACCA AAAAGCGCA AUU | 53 |
| | ATXL045 | Antisense | mU*fU*mGmCmGfC*Tm UfUmUmUmGmGfUmGfA mCmGmC*mU*mU | UUGCGCTUU UUGGUGACG CUU | 72 |
| LMNA-si49 | ATXL083 | Sense | mG*mC*mGmUrC*mArC* mCfArA*rA*mArA*mGrC *mGmCmAmA*mU*mU | GCGUCACCA AAAAGCGCA AUU | 54 |
| | ATXL045 | Antisense | mU*fU*mGmCmGfC*Tm UfUmUmUmGmGfUmGfA mCmGmC*mU*mU | UUGCGCTUU UUGGUGACG CUU | 72 |
| LMNA-si50 | ATXL084 | Sense | mG*mC*mGmUrC*mArC* mCfArA*mAmArA*mGrC *mGmCmAmA*mU*mU | GCGUCACCA AAAAGCGCA AUU | 55 |
| | ATXL045 | Antisense | mU*fU*mGmCmGfC*Tm UfUmUmUmGmGfUmGfA mCmGmC*mU*mU | UUGCGCTUU UUGGUGACG CUU | 72 |
| LMNA-si51 | ATXL085 | Sense | mG*mC*mGmUrC*mArC* mCfAfArA*mArA*mGrC* mGmCmAmA*mU*mU | GCGUCACCA AAAAGCGCA AUU | 56 |
| | ATXL045 | Antisense | mU*fU*mGmCmGfC*Tm UfUmUmUmGmGfUmGfA mCmGmC*mU*mU | UUGCGCTUU UUGGUGACG CUU | 72 |

| | | | | | |
|---|---|---|---|---|---|
| * the oligomeric compounds in this Table do not comprise the motifs claimed | | | | | |

siRNA was transfected at 0-10 nM into HeLa cells with RNAiMAX (Invitrogen, Waltham, MA) and the cells further cultured for 24 hr. siRNA activity was determined by qRT-PCR using the primer probe set in Table 2 and the IC50 of the siRNAs targeting LMNA was calculated (Table 26).

**Table 26: LMNA siRNA Inhibition in HeLa Cells**

| | HeLa Cells (24 hrs) | | HeLa Cells (120 hrs) | |
|---|---|---|---|---|
| siRNA | IC50 (nM) | Fold | IC50 (nM) | Fold |
| LMNA-si2 | 9.478 | 1 | 0.95 | 1 |
| LMNA-si31 | 0.176 | 54 | 0.148 | 6.4 |
| LMNA-si42 | 0.381 | 25 | 0.446 | 2.1 |
| LMNA-si43 | 0.179 | 53 | 0.26 | 3.7 |
| LMNA-si44 | 0.44 | 21.6 | 0.338 | 2.8 |
| LMNA-si45 | 0.267 | 35.5 | 0.335 | 2.8 |
| LMNA-si46 | 0.235 | 40.3 | 0.456 | 2.1 |
| LMNA-si2 | 9.478 | 1 | 0.95 | 1 |
| LMNA-si31 | 0.176 | 54 | 0.148 | 6.4 |
| LMNA-si47 | 0.104 | 91.2 | 0.104 | 9.2 |
| LMNA-si48 | 0.44 | 21.6 | 0.169 | 5.6 |
| LMNA-si49 | 0.337 | 28.2 | 0.115 | 8.3 |
| LMNA-si50 | 0.391 | 24.2 | 0.15 | 6.4 |
| LMNA-si51 | 0.256 | 37 | 0.079 | 12.1 |

siRNA was transfected at 0-10 nM into HEK293 cells with RNAiMAX (Invitrogen, Waltham, MA) and the cells further cultured for 48 hr and 120 hr. siRNA activity was determined by qRT-PCR using the primer probe set in Table 2 and the IC50 of the siRNAs targeting LMNA was calculated (Table 27).

**Table 27: LMNA siRNA Inhibition in HEK293 Cells**

| | HEK293 Cells (48 hrs) | | HEK293 Cells (120 hrs) | |
|---|---|---|---|---|
| siRNA | IC50 (nM) | Fold | IC50 (nM) | Fold |
| LMNA-si2 | 5.433 | 1 | 2.021 | 1 |
| LMNA-si31 | 0.055 | 99.5 | 0.078 | 25.9 |
| LMNA-si42 | 0.031 | 177 | 0.578 | 3.5 |
| LMNA-si43 | 0.054 | 100.8 | 0.087 | 23.1 |
| LMNA-si44 | 0.084 | 64.9 | 0.193 | 10.5 |
| LMNA-si45 | 0.044 | 123.8 | 0.129 | 15.6 |
| LMNA-si46 | 0.161 | 33.7 | 0.108 | 18.7 |
| LMNA-si2 | 5.433 | 1 | 2.021 | 1 |
| LMNA-si31 | 0.055 | 99.5 | 0.078 | 25.9 |
| LMNA-si47 | 0.031 | 177 | 0.077 | 26.3 |
| LMNA-si48 | 0.051 | 105.5 | 0.118 | 17.2 |
| LMNA-si49 | 0.060 | 89.9 | 0.087 | 23.2 |
| LMNA-si50 | 0.036 | 148.9 | 0.135 | 15 |
| LMNA-si51 | 0.021 | 255.8 | 0.113 | 17.9 |

All the newly designed siRNAs in this example showed better activity for a longer duration compared to benchmark siRNA LMNA-si2. LMNA-si39, LMNA-si47, LMNA-si49 and LMNA-si51 have consistently good activity and duration of activity in the different cell types. Selected siRNA designs were re-assessed for activity for over an even longer time period.

siRNA was transfected at 0-10 nM into HeLa cells with RNAiMAX (Invitrogen, Waltham, MA) and the cells further cultured for different times. siRNA activity was determined by qRT-PCR using the primer probe set in Table 2 and the IC50 of the siRNAs targeting LMNA was calculated (Table 28).

**Table 28: LMNA siRNA Inhibition in HeLa Cells**

| | HeLa Cells (24 hrs) | | HeLa Cells (72 hrs) | | HeLa Cells (120 hrs) | | HeLa Cells (168 hrs) | |
|---|---|---|---|---|---|---|---|---|
| siRNA | IC50 (nM) | Fold | IC50 (nM) | Fold | IC50 (nM) | Fold | IC50 (nM) | Fold |
| LMNA-si31 | 0.0354 | 100 | 0.025 | 11.1 | 0.026 | 11.8 | 0.144 | 8.1 |
| LMNA-si42 | 0.007 | 472.7 | 0.021 | 13.3 | 0.111 | 2.7 | 0.631 | 1.8 |
| LMNA-si44 | 0.056 | 63.6 | 0.025 | 10.9 | 0.095 | 3.2 | 0.482 | 2.4 |
| LMNA-si46 | 0.068 | 51.9 | 0.016 | 17.2 | 0.164 | 1.8 | 0.581 | 2 |
| LMNA-si47 | 0.038 | 94.1 | 0.014 | 19.9 | 0.055 | 5.5 | 0.141 | 8.2 |
| LMNA-si49 | 0.188 | 18.9 | 0.02 | 14 | 0.071 | 4.3 | 0.251 | 4.6 |
| LMNA-si51 | 0.044 | 80.1 | 0.018 | 15.4 | 0.095 | 3.2 | 0.142 | 8.2 |
| LMNA-si2 | 3.538 | 1 | 0.277 | 1 | 0.303 | 1 | 1.164 | 1 |

When assessed at the longest time point, 168 hrs, the results show that LMNA-si47 and LMNA-si51 were the most active, while LMNA-si31 was also very active. ARNATAR designed compounds showed faster onset of activity than the benchmark LMNA-si2 and still stayed more active at later time points.

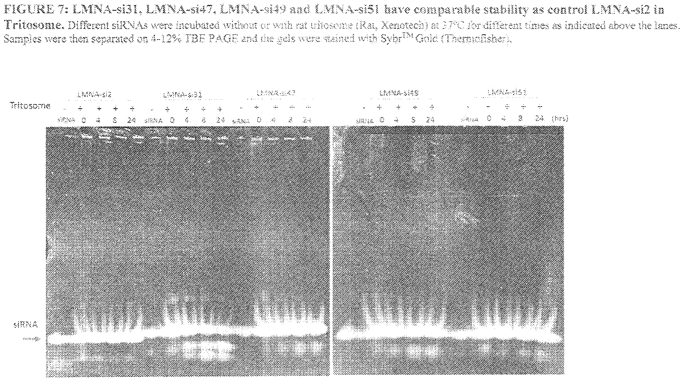

Stability assays were performed on LMNA-si31, LMNA-si47, LMNA-si49 and LMNA-si51. The first assay exposed the compounds to human serum to test for stability, by mixing siRNA with human serum. The second assay exposed the compounds to rat tritosome to test for stability. The test compounds were found to have comparable stability compared to benchmark LMNA-si2 (Figures 7-8).

### Serum Stability Assay

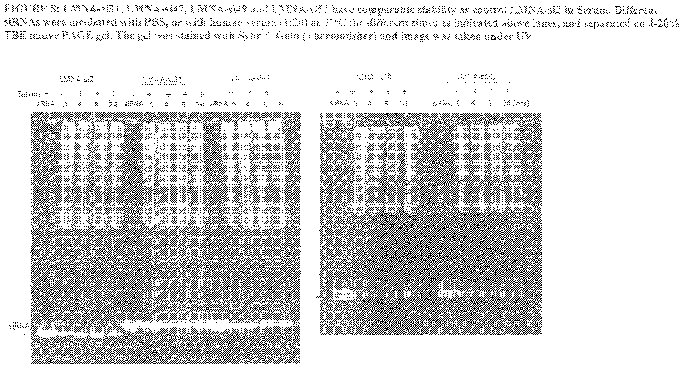

siRNAs were incubated with human serum (Sigma-Aldrich, St. Louis, MO, USA) at 0.4µM concentration, at 37°C for a desired time (0, 4, 8, and 24 h). Then BlueJuice^{™} gel loading buffer (ThermoFisher Scientific, Waltham, MA, USA) was added and the siRNA mix was loaded onto 4-20% native TBE gel (ThermoFisher Scientific, Waltham, MA, USA). After running the gel, it was stained with 1:10000 SYBR^{™} Gold (ThermoFisher Scientific, Waltham, MA, USA) in water, at room temperature for 10 min and imaged under UV light (Figure 8).

### Tritosome Stability Assay

The tritosome stability assay described here is based on an assay by Weingartner et al. (Molecular Therapy, Nucleic Acids, 2020, 21:242-250). siRNA was incubated for 0, 4, 24, or 72 h in Rat Liver Tritosomes (R0610.LT; XenoTech, Kansas City, KS, USA). To mimic the acidified environment, tritosome lysates were mixed 10:1 with low pH buffer (1.5M acetic acid, 1.5M sodium acetate, pH 4.75). 4 µL of these acidified tritosomes was mixed with 1 µL siRNA (10 µM) and incubated for the indicated times at 37°C. Then BlueJuice^{™} gel loading buffer (ThermoFisher Scientific, Waltham, MA, USA) was added and the siRNA mix was loaded onto 4-20% native TBE gel (ThermoFisher Scientific, Waltham, MA, USA). After running the gel, it was stained with 1:10000 SYBR^{™} Gold (ThermoFisher Scientific, Waltham, MA, USA) in water, at room temperature for 10 min and imaged under UV light (Figure 7).

### EXAMPLE 7: DESIGNING NCL siRNA WITH VARYING SENSE AND ANTISENSE STRAND MODIFICATIONS

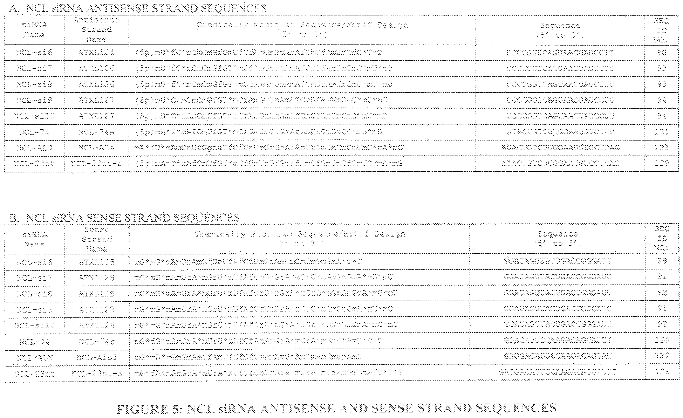

Based on the previous studies, *supra,* with LMNA and ApoC3, some of the chemical modification conferring high activity was assessed in a different target to determine whether the chemical modification motifs would also be beneficial. NCL was chosen as the third test target and siRNAs designed, as shown in Table 29 and Figure 5, to target a 19 nucleotide long sequence of human NCL: GGAUAGUUACUGACCGGGA (SEQ ID NO: 98). The primer probe set sequences are shown in Table 30. NCL-si6, a modified siRNA where the chemical modification pattern reflects that of lumasiran with the exception that the sense strand of NCL-si6 has an addition TT overhang connected by PS linkages and does not have a GalNAc conjugate, and the antisense strand is 21 nt rather than 23 nt. The chemical modification pattern of NCL-si7 is based on that of LMNA-si47 described *supra.* The chemical modification pattern of NCL-si8 is based on that of LMNA-si51 described *supra.* The chemical modification pattern of NCL-si9 is based on that of LMNA-si42 described *supra.* The chemical modification pattern of NCL-si10 is based on that of LMNA-si46 described *supra.* All antisense strands have a 5'-phosphate. The modified siRNAs were tested for activity in a HeLa cells.

**Table 29: NCL Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| NCL-si6 | ATXL125 | Sense | mG*mG*mAmUmAmGfUm UfAfCfUmGmAmCmCmG mGmGmA*T*T | GGAUAGUUA CUGACCGGG ATT | 89 |
| | ATXL124 | Antisense | (5p)mU*fC*mCmCmGfGm UfCfAmGmUmAmAfCmUf AmUmCmC*T*T | UCCCGGUCA GUAACUAUC CTT | 90 |
| NCL-si7 | ATXL128 | Sense | mG*mG*mAmUrA*mGrU* mUfAfCmUmGrA*mCrC*m GmGmGmA*mU*mU | GGAUAGUUA CUGACCGGG AUU | 91 |
| | ATXL126 | Antisense | (5p)mU*fC*mCmCmGfGT* mCfAmGmUmAmAfCmUf AmUmCmC*mU*mU | UCCCGGTCA GUAACUAUC CUU | 93 |
| NCL-si8 | ATXL129 | Sense | mG*mG*mAmUrA*mGrU* mUfAfCrU*mGrA*mCrC* mGmGmGmA*mU*mU | GGAUAGUUA CUGACCGGG AUU | 92 |
| | ATXL126 | Antisense | (5p)mU*fC*mCmCmGfGT* mCfAmGmUmAmAfCmUf AmUmCmC*mU*mU | UCCCGGTCA GUAACUAUC CUU | 93 |
| NCL-si9 | ATXL128 | Sense | mG*mG*mAmUrA*mGrU* mUfAfCmUmGrA*mCrC*m GmGmGmA*mU*mU | GGAUAGUUA CUGACCGGG AUU | 91 |
| | ATXL127 | Antisense | (5p)mU*C*mCmCmGfGT* mCfAmGmUmAmAfCmUf AmUmCmC*mU*mU | UCCCGGTCA GUAACUAUC CUU | 94 |
| NCL-si10 | ATXL129 | Sense | mG*mG*mAmUrA*mGrU* mUfAfCrU*mGrA*mCrC* mGmGmGmA*mU*mU | GGAUAGUUA CUGACCGGG AUU | 92 |
| | ATXL127 | Antisense | (5p)mU*C*mCmCmGfGT* mCfAmGmUmAmAfCmUf AmUmCmC*mU*mU | UCCCGGTCA GUAACUAUC CUU | 94 |

| | | | | | |
|---|---|---|---|---|---|
| * the oligomeric compounds in this Table do not comprise the motifs claimed | | | | | |

**Table 30. The primer probe set sequences for human NCL mRNA**

| Name | Sequence | SEQ ID NO: |
|---|---|---|
| hsNCL-F | GCTTGGCTTCTTCTGGACTCA | 12 |
| hsNCL-R | TCGCGAGCTTCACCATGA | 13 |
| hsNCL-P | CGCCACTTGTCCGCTTCACACTCC | 14 |

siRNA was transfected at 0-10 nM into HeLa cells with RNAiMAX (Invitrogen, Waltham, MA) and the cells further cultured for 24 hr. siRNA activity was determined by measuring the levels of target mRNA through qRT-PCR using the primer probe set in Table 30 and the IC50 of the siRNAs targeting NCL was calculated (Table 31). qRT-PCR was performed using AgPath-ID^{™} One-Step RT-PCR Reagents in QS3 real-time PCR system (ThermoFisher Scientific, Waltham, MA, USA). The target RNA levels detected in qRT-PCR assay were normalized to either total RNA levels measured with Ribogreen^{™} (ThermoFisher Scientific, Waltham, MA, USA) or GAPDH mRNA levels detected in the aliquots of RNA samples using qRT-PCR.

**Table 31: NCL siRNA Inhibition in HeLa Cells**

| | HeLa Cells (24 hrs) | |
|---|---|---|
| siRNA | IC50 (nM) | Fold |
| NCL-si6 | 0.528 | 1 |
| NCL-si7 | 0.004 | 136.7 |
| NCL-si8 | 0.003 | 207.1 |
| NCL-si9 | 0.0002 | 2945.8 |
| NCL-si10 | 0.002 | 326.3 |

The new chemical modification motif initially designed with LMNA targeting siRNAs, when used to modify NCL targeting siRNAs, generally conveyed increased activity irrespective of target or the siRNA sequence.

### EXAMPLE 8: FURTHER CHARACTERIZATION OF THE ARNATAR DESIGNED MODIFIED OLIGOMERIC COMPOUNDS IN VITRO

Previous studies, *supra,* assess the inhibition activity over time of oligomeric compounds after transfection into cell lines. Oligomeric compounds may have different activity over time depending on the mode of transportation into cells e.g., transfection versus free uptake.

When transfected into cells, the oligomeric compound is quickly released into the cytoplasm of the cells and quickly starts actively inhibiting the target. In other words, the oligomeric compounds are released into the cytoplasm in a quick burst, have early onset and robust activity, but the activity period is short.

When oligomeric compounds are transported into cell by free uptake (e.g., endocytosis via a receptor such as asialoglycoprotein receptor (ASGR)), the compounds are initially stored and protected from nucleases in endosomes/lysosomes and slowly released into the cytoplasm. This slow release of the compounds leads to a delayed onset and slower inhibition activity initially, but, allow a prolonged and sustained time period of activity.

When siRNA duplex is released to the cytoplasm, the duplex needs to interact with RISC and undergoes strand separation, in order to allow antisense strand-guided target nucleic acid target degradation. The efficiency of RISC loading and strand separation also affects siRNA activity and kinetics.

Select potent oligomeric compounds from the previous studies were assessed for additional characteristics such as melting temperature (Tm) and loading of the compound into the RISC system. LMNA-si2 was used as a benchmark for comparison.

### A. ARNATAR Motifs Show Faster Onset of siRNA Activity After Transfection

The ARNATAR design siRNAs assessed in previous examples showed a faster onset of siRNA activity. Without being bound by any particular theory, this faster onset may be due to faster RISC loading and strand separation than the benchmark compound. Studies were performed with selected oligomeric compounds to assess the time course, melting temperature (Tm) and RISC loading (Eamens et al., 2009, RNA, 15:2219-2235).

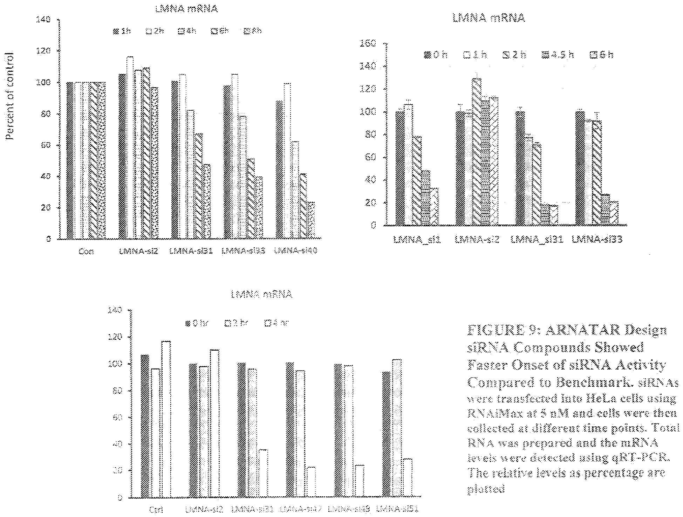

Selected siRNAs were transfected at 0-10 nM into HeLa cells for 2 or 4 hrs, and LMNA mRNA levels were detected through qRT-PCR using primer probe sets listed in Table 2. The percentage of mRNA levels relative to the level in the time point 0 of the LMNA-si2 samples is shown in Table 32 and Figure 9.

**Table 32: LMNA mRNA Levels in Cells Treated with Different siRNAs for Different Time**

| Time | Ctrl | LMNA-si2 | LMNA-si31 | LMNA-si47 | LMNA-si49 | LMNA-si51 |
|---|---|---|---|---|---|---|
| 0 | 106.90 | 100.00 | 100.42 | 100.48 | 99.25 | 92.98 |
| 2 hr | 96.21 | 97.82 | 95.43 | 94.22 | 97.81 | 102.12 |
| 4 hr | 116.49 | 109.93 | 35.12 | 21.43 | 22.89 | 27.72 |

The ARNATAR designed siRNAs showed faster onset of triggering target reduction since substantial reduction of the target LMNA mRNA was observed at 4 hr after transfection ARNATAR designed siRNAs but not the reference siRNA (LMNA-si2).

### Melting Temperature (Tm) Determination by Melting Curve

The Tm of a double stranded oligomeric compound is the temperature at which one half of the duplex dissociates and becomes single stranded. Tm is a measure of duplex stability. A lower Tm may facilitate faster dissociation between sense and antisense strands and allow faster availability of the antisense strand to interacting with target RNA.

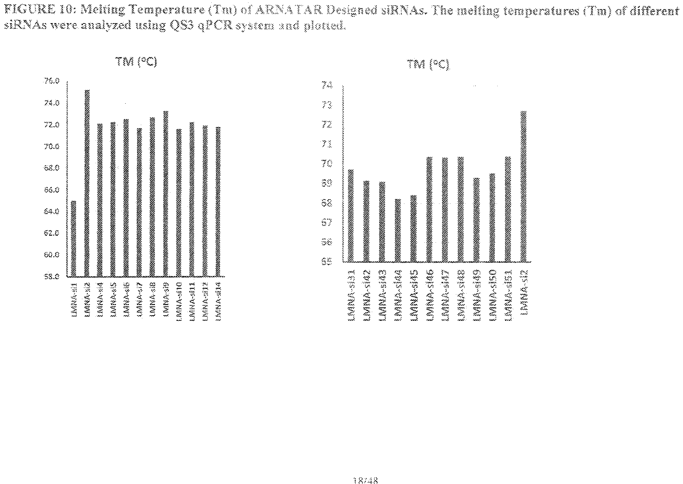

0.5µl of 10µM siRNAs were incubated in 20µl 1X TE buffer containing 1:1000 Ribogreen (Invitrogen, ThermoFisher Scientific, Waltham, MA, USA). Triplicates for each sample were plated in a 96-well qPCR plate and placed in a QuantStudio^{®} 3 Real-Time PCR System (ThermoFisher Scientific, Waltham, MA, USA) and under the following conditions to obtain the melting temperature (Tm): 95°C, 15 sec; 40°C, 1 min; melt curve to 95°C, 15sec/°C). Tm was analyzed with the QuantStudio^{™} Design & Analysis Software v1.5.2 and the results shown in Table 33 and Figure 10.

**Table 33: Tm value of selected siRNAs**

| | LMN A-si31 | LMN A-si42 | LMN A-si43 | LMN A-si44 | LMN A-si45 | LMN A-si46 | LMN A-si47 | LMN A-si48 | LMN A-si49 | LMN A-si50 | LMN A-si51 | LMN A-si2 |
|---|---|---|---|---|---|---|---|---|---|---|---|---|
| Tm (°C) | 69.69 | 69.12 | 69.09 | 68.20 | 68.39 | 70.36 | 70.32 | 70.37 | 69.28 | 69.51 | 70.38 | 72.71 |

Most of the siRNAs that show increased activity have lower Tm than the reference siRNA si2.

### Measurement of siRNA loaded into RISC

siRNAs must be loaded to RISC and associate with Ago2 to degrade target RNAs. Measurement of RISC-loaded siRNA (antisense strand) in cells was based on the study of Castellanos-Rizaldos et al. (Nucleic Acid Therapeutics, 2020, 30(3):133-142) using a combination of immunoprecipitation followed by the stem-loop RT-qPCR of the final lysates of the RISC-loaded siRNAs.

### i. Sample Preparation

For each siRNA to be tested, 1.5 X10⁶ HELA cells were transfected with 2 nM siRNA and harvested after 16 hrs. The cells were harvested and the cell pellet washed with 1XPBS then resuspended with 500 µL pre-chilled RIPA Lysis and Extraction Buffer (ThermoFisher Scientific, Waltham, MA, USA) supplemented with 5 µL RNAse Out^{™} (ThermoFisher Scientific, Waltham, MA, USA) and Protease Inhibitor Cocktail (Roche, Sigma-Aldrich, St. Louis, MO, USA). The cellular mixture was gently mixed by pipetting and incubated on ice for 30 min. The cellular mixture was centrifuged to separate the pellet and 400 µL of the supernatant decanted into a clean tube. 10 µL (2.5%) supernatant was set aside as input and the rest stored at -80°C until needed.

### ii. Ago2 Bead Coating Preparation

40 µL magnetic beads were washed with 200 µL RIPA Lysis and Extraction Buffer. The washed beads were resuspended in 100 µL RIPA Lysis and Extraction Buffer and 10 µL (5 µg) Ago2 antibody (ab57113, Abcam, Cambridge, UK) and incubated at 4°C for 2 hrs.

### iii. Ago2 Immunoprecipitation

200 µL of the supernatant from the sample preparation was incubated with Ago2 antibody precoated beads for 3 hrs with gentle rotation at 4°C. The mixture was centrifuged, the supernatant removed and the beads washed 7X with 500 µL wash buffer (50 mM Tris·Cl, pH7.5; 150 mM NaCl; 5 mM EDTA; 0.1% NP-40). 250 µL PBS/TritonX100 buffer was added to the washed beads and the mixture incubate at 95°C for 10 min, vortexed, then iced for 10 min. The mixture was centrifuged at 16,000g for 10 mins at 4°C, then the supernatant was transferred to a clean tube and set aside for the next step.

### iv. Prepare Samples for Reverse Transcription (RT)

The 10 µL (2.5%) input supernatant was mixed with 250 µL PBS/TritonX100 buffer, heated to 95°C for 10 min, vortexed, then placed on ice for 10 min before being centrifuged for 10 min at 4°C. The supernatant was transferred to clean tube. 20 µL of the supernatant for each sample was added to a 96-well plate, and heated in a PCR machine at 95°C for 10 min to allow the duplexes to denature, then held at 4°C for 5 min. To prepare a control, 250 µL PBST buffer (0.25% TritonX100 in 1XPBS buffer) was added to untreated beads.

### v. Reverse Transcription (RT)

For each RT reaction, 5 µL of prepared sample was heated to 90°C for 3 min to unpair the sense and antisense strands then the samples were iced immediately afterward to keep the strand separated. hsLMNA-A primer was used for antisense strand reaction, hsLMNA-S primer was used for the sense strand reaction. RT was performed using reverse transcription kit (ThermoFisher Scientific, Waltham, MA, USA) according to the manufacturer's suggested protocol. For a control, in an RT reaction, 5 µL of 0.2 µM siRNA was used as a RNA template to set up a standard curve.

### vi. qPCR Quantitation

cDNA from the RT reaction was diluted 1:3 with DEPC water. The control RT reaction was also diluted 1:3 then serially diluted 1:10 an additional 5X. The concentration of cDNA in the serial dilution of the control reaction was: 16.66 nM, 1.666 nM, 0.1666 nM, 0.01666 nM, 0.001666 nM, 0 nM.

4 µL diluted RT cDNA template was mixed with 5 µL of 2X TaqMan^{™} Universal Master Mix II without UNG (ThermoFisher Scientific, Waltham, MA, USA) and 0.5 µL DDW. Primer hsLMNA-S or hsLMNA-A was used as appropriate. PCR was run under the following conditions: 95°C, 3 min; 40 cycles of 95°C, 15 second; 60°C, 20 second.

In summary, siRNAs associated with Ago2 were isolated using Ago2 antibody through immunoprecipitation from HELA cells transfected with 2 nM of each siRNA at two different time points. The isolated siRNAs and total cellular siRNAs were quantified using a kit from ThermoFisher (Waltham, MA, USA) with their proprietary stem-loop primer probe sets. The recovery rate of antisense siRNA associated with Ago2 relative to the levels of total cellular antisense siRNA was calculated, and normalized to the recovery rate of miRNA-16 as detected through qRT-PCR using stem-loop primer probe sets specific to human miRNA-16 (ThermoFisher Scientific, Waltham, MA, USA). The calculated recovery rates are shown in Table 34.

**Table 34: The Relative Level of Antisense siRNA Associated with Ago2 in Cells Transfected with the siRNAs for Different Times**

| | LMNA-si2 | LMNA-si31 | LMNA-si47 | LMNA-si49 | LMNA-si51 |
|---|---|---|---|---|---|
| 48 hr | 1.5 | 2.66 | 4.12 | 2.3 | 3.31 |
| 52 hr | 1.19 | 3.60 | 4.47 | 2.62 | 2.64 |

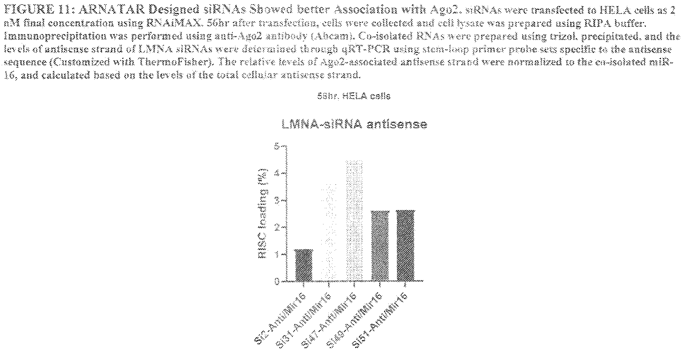

This study shows more efficient loading of ARNATAR motif siRNAs into RISC than the LMNA-si2 reference siRNA (Table 34 and Figure 11).

### B. ARNATAR Motifs Show Better Activity Upon Free Uptake into Cells In Vitro

### LMNA Study

Select LMNA siRNAs were conjugated with various types of N-Acetylgalactosamine (GalNAc) (Table 35) in order to allow free uptake of the compound into cells. GalNAc was attached to the sense strand of the siRNAs.

**Table 35: LMNA Oligomeric Compounds with Modifications to Both Strands**

| siRNA Name | Sequence and Chemistry* | GalNAc |
|---|---|---|
| LMNA-si52 | LMNA-si2 | GalNAc 1 |
| LMNA-si55 | LMNA-si47 | GalNAc 2 |
| LMNA-si64 | LMNA-si42 | GalNAc 2 |

| | | |
|---|---|---|
| * the oligomeric compounds in this Table do not comprise the motifs claimed | | |

LMNA-si52 was designed to be another benchmark to compare ARNATAR design compounds. It has the sequence and chemistry as LMNA-si2 as described in Example 1 and is conjugated to a GalNAc used by Alnylam in their siRNA compounds (Nair et al., J. Am. Chem. Soc. 2014, 136(49):16958-16961): also known as GalNAc 1 or GA1.

LMNA-si55 and LMNA-si64 use the sequence and chemistry of LMNA-si47 and LMNA-si42, respectively, and which were shown in previous examples to have a potent and stable chemical modification motif, conjugated to a GalNAc from AM Chemicals (US Patent 10,087,208): also known as GalNAc 2, GalNAc (AM) or GA2.

GalNAc-conjugated siRNA was directly added to primary hepatocytes *in vitro* at 6.4, 32, and 160 nM final concentrations and the cells further cultured for 48 hr. siRNA activity was determined by qRT-PCR using the primer probe set in Table 2 and the IC50 of the siRNAs targeting LMNA was calculated (Table 36).

**Table 36: LMNA siRNA Inhibition in Primary Hepatocytes**

| | Primary Hepatocytes (48 hrs) | |
|---|---|---|
| siRNA | IC50 (nM) | Fold |
| LMNA-si52 | 29.45 | 1 |
| LMNA-si55 | 2.343 | 12.57 |
| LMNA-si64 | 1.526 | 19.3 |

After free uptake into cells, LMNA-si55 and LMNA-si64 showed over 10-fold increased activity over benchmark LMNA-si52.

### ApoC3 Study

Select ApoC3 siRNAs were conjugated with N-Acetylgalactosamine (GalNAc) (Table 37) in order to allow free uptake of the compound into cells. GalNAc was attached to the sense strand of the siRNAs.

**Table 37: ApoC3 Oligomeric Compounds with Modifications to Both Strands**

| siRNA Name* | Chemical Modifications | GalNAc |
|---|---|---|
| ApoC3-si10 | LMNA-si2 motif | GalNAc 2 |
| ApoC3-si11 | LMNA-si47 motif | GalNAc 2 |
| ApoC3-si12 | LMNA-si42 motif | GalNAc 2 |
| ApoC3-si13 | LMNA-si52 motif with 2 less PS linkages | GalNAc 2 |
| ApoC3-si14 | LMNA-si46 motif without 3' PS linkages | GalNAc 2 |

| | | |
|---|---|---|
| * the oligomeric compounds in this Table do not comprise the motifs claimed | | |

Chemical Modifications in Table 37 describe the pattern of modifications regardless of the nucleoside sequence. For example, ApoC3-si10 - having an LMNA-si2 motif - has the same pattern of chemical modifications as LMNA-si2, while the nucleoside sequence is different from that of LMNA-si2. For the full sequence and chemistry of ApoC3 siRNAs see Figures 3-4.

0-4 µM GalNAc-conjugated siRNA was directly added to primary hepatocytes *in vitro* and the cells further cultured for 48 hr and 72 hr. siRNA activity was determined by qRT-PCR using the primer probe set in Table 19 and the IC50 of the siRNAs targeting ApoC3 was calculated (Table 38).

**Table 38: IC50 of Apoc3 siRNA in primary hepatocytes upon free uptake**

| | ApoC3-10 (reference) | ApoC3-11 | ApoC3-12 | ApoC3-13 | ApoC3-14 |
|---|---|---|---|---|---|
| IC50 (nM) | NA | 4.243 | 2.616 | 3.007 | 0.179 |

### EXAMPLE 9: CHARACTERIZATION OF THE ARNATAR DESIGNED MODIFIED OLIGOMERIC COMPOUNDS 72 HOURS IN VIVO

LMNA-si55, LMNA-si64 and benchmark LMNA-si52 were previously assessed for their inhibition potency and other characteristics *in vitro.* In this study, the oligomeric compounds were tested in mice to determine whether *in vitro* results could be correlated *in vivo.*

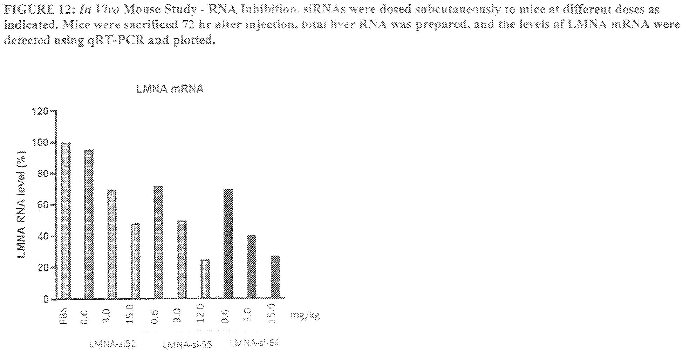

Three animals per group of 7- to 8-week-old Balb/C male mice were injected subcutaneously with 0.6mg/kg, 3mg/kg or 15mg/kg of LMNA-si52, LMNA-si55 or LMNA-si64. For LMNA-si55, the high dose was 12 mg/kg rather than 15 mg/kg due to material limitation. Phosphate buffered saline (PBS) was injected as a control. Post dosing 72 hr, the mice were sacrificed and organs harvested for analysis (Tables 39-40 and Figure 12).

### A. Inhibition Potency

**Table 39: LMNA siRNA Inhibition in Mouse Liver at 72 hrs**

| siRNA | LMNA-si52 | | | LMNA-si55 | | | LMNA-si64 | | |
|---|---|---|---|---|---|---|---|---|---|
| Dose | 0.6 mg/kg | 3 mg/kg | 15 mg/kg | 0.6 mg/kg | 3 mg/kg | 12 mg/kg | 0.6 mg/kg | 3 mg/kg | 15 mg/kg |
| LMNA mRNA | 95.74 | 69.86 | 48.27 | 72.27 | 49.97 | 24.99 | 69.77 | 40.54 | 26.95 |

**Table 40: LMNA siRNA Inhibition in Mouse Liver**

| | Mouse Liver at 72 hrs | |
|---|---|---|
| siRNA | IC50 (mg/kg) | Fold |
| LMNA-si52 | 12.46 | 1 |
| LMNA-si55 | 2.664 | 4.7 |
| LMNA-si64 | 2.09 | 6 |

LMNA-si55 and LMNA-si64 were more potent that benchmark LMNA-si52 and inhibited LMNA mRNA in a dose dependent manner.

### B. Safety

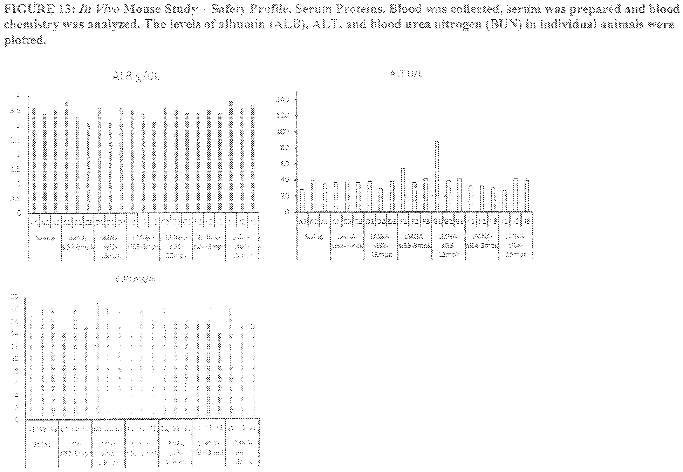

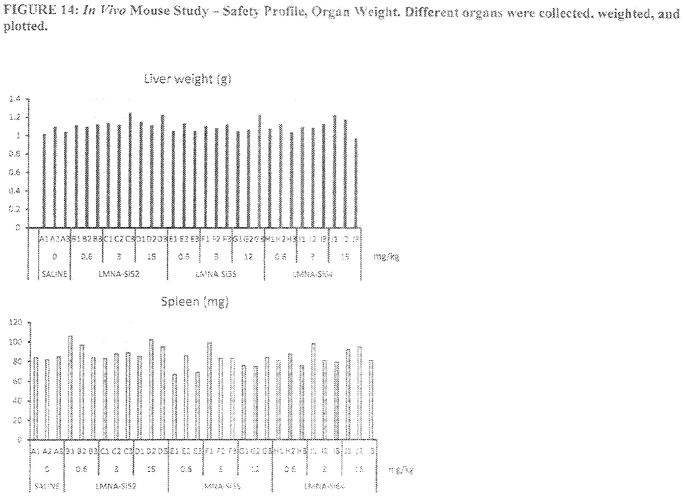

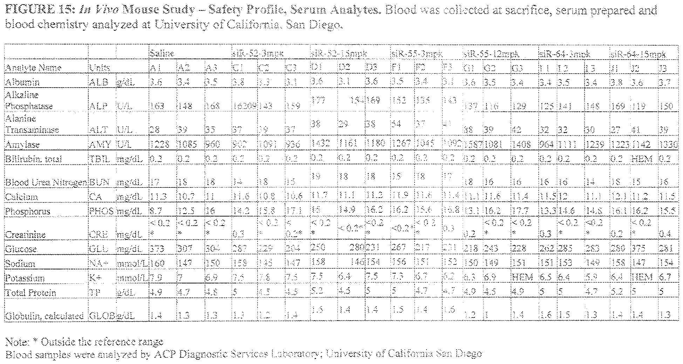

As shown in the tables below, stress or damage markers were assessed for various organs. The blood chemistry was measured by ACP Diagnostic Services Laboratory; University of California San Diego. The markers assessed include: serum albumin (ALB), alanine transaminase (also alanine amino transferase or ALT), blood urea nitrogen (BUN), total bilirubin (TBIL), liver weight, spleen weight and other analytes (Figures 13-15). ALB, if low, can be a marker for liver disease, kidney disease or other medical issue. ALT is an enzyme mainly found in the liver. High levels of ALT may indicate the presence of liver disease such as acute hepatitis. BUN is a waste product from protein breakdown by the liver and is excreted by the kidneys. A high BUN level is a marker for kidney or liver dysfunction. TBIL measures the amount of bilirubin in the blood. High levels of TBIL may indicate liver disease, blockage of the bile ducts or other medical issue. Body and organ (e.g., liver and spleen) weights were taken to make sure the mice were not feeling a general malaise and off their feed.

Generally, all the assessed markers were found to be within tolerable parameters compared to PBS control after administering the oligomeric compounds (Figures 13-15).

### C. Immunogenicity

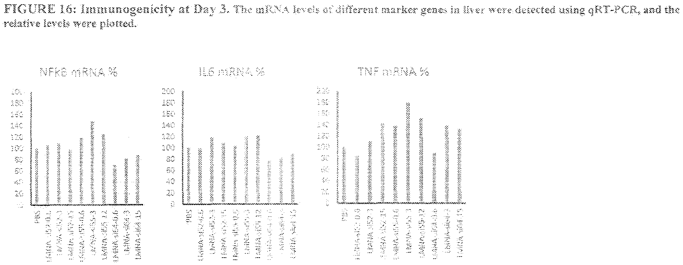

Liver samples from the same animals as used above were analyzed for immunogenetic signals. mRNA levels of some immune response markers were examined using qRT-PCR with primer probe sets specific for mouse NfkB, IL6 and TNF (ThermoFisher Scientific, Waltham, MA, USA) as shown in Table 41 with the results presented in Table 42 and Figure 16. Primer probe sets were from ThermoFisher Scientific (Waltham, MA, USA).

**Table 41: Primer-Probe Sets**

| Primer/Probe Name | Sequence | SEQ ID NO |
|---|---|---|
| hsNFkB-F | AAACACTGTGAGGATGGGATC | 99 |
| hsNFkB-R | TCTGTCATTCGTGCTTCCAG | 100 |
| hsNFkB-P | TGTCACATGAAGTATACCCAGGTTTGCG | 101 |
| msNfkB-F | GTGTCAGAGCCCTTGTAACTG | 102 |
| msNfkB-R | ACATTTGCCCAGTTCCGTAG | 103 |
| msNfkB-P | TCGTCTGCCATGGTGAAGATGCG | 104 |
| msIL6-F | AAACCGCTATGAAGTTCCTCTC | 105 |
| msIL6-R | GTGGTATCCTCTGTGAAGTCTC | 106 |
| msIL6-P | TTGTCACCAGCATCAGTCCCAAGAA | 107 |
| msTNF-F | AGACCCTCACACTCAGATCA | 108 |
| msTNF-R | TGTCTTTGAGATCCATGCCG | 109 |
| msTNF-P | CCTGTAGCCCACGTCGTAGCAAA | 110 |

**Table 42. mRNA Levels in Liver Samples of Animals Treated with siRNAs for 72 Hr**

| | PBS | LMNA -si52-0.6 | LMNA -si52-3 | LMNA -si52-15 | LMNA -si55-0.6 | LMNA -si55-3 | LMNA -si55-12 | LMNA -si64-0.6 | LMNA -si64-3 | LMNA -si64-15 |
|---|---|---|---|---|---|---|---|---|---|---|
| NFkB mRNA % | 100.0 | 105.95 | 109.24 | 98.69 | 118.47 | 147.70 | 125.26 | 70.42 | 81.19 | 88.11 |
| IL6 mRNA % | 100.0 | 99.75 | 119.20 | 107.96 | 103.17 | 120.68 | 122.05 | 76.71 | 82.41 | 89.31 |
| TNF mRNA % | 100.0 | 85.34 | 111.30 | 142.46 | 137.86 | 179.44 | 151.28 | 89.55 | 138.90 | 133.25 |

In general, all the assessed markers were found to be within tolerable levels compared to PBS control 72 hrs after administering the oligomeric compounds (Figure 16).

### EXAMPLE 10: CHARACTERIZATION OF THE ARNATAR DESIGNED MODIFIED OLIGOMERIC COMPOUNDS 7 DAYS IN VIVO

LMNA-si52, LMNA-si55 or LMNA-si64 were assessed for a longer time in mice.

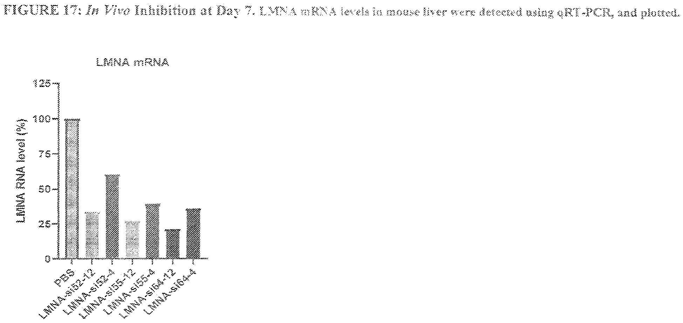

3 animals per group of 7- to 8-week-old male Balb/C mice were injected subcutaneously with 4mg/kg or 12mg/kg of LMNA-si52, LMNA-si55 or LMNA-si64. Phosphate buffered saline (PBS) was injected as a control. Post dosing 7 days, the mice were sacrificed and organs harvested for analysis (Tables 43-44 and Figures 17-18).

**Table 43: LMNA siRNA % Inhibition in Mouse Liver at Day 7**

| Treatment | Dose | % LMNA mRNA |
|---|---|---|
| PBS | -- | 100.0 |
| LMNA-si52 | 12 mg/kg | 33.5 |
| LMNA-si52 | 4 mg/kg | 60.13 |
| LMNA-si55 | 12 mg/kg | 26.93 |
| LMNA-si55 | 4 mg/kg | 39.1 |
| LMNA-si64 | 12 mg/kg | 20.98 |
| LMNA-si64 | 4 mg/kg | 35.83 |

LMNA-si55 and LMNA-si64 knocked down LMNA mRNA levels in a dose dependent manner at day 7 after dosing mice. LMNA knockdown by LMNA-si55 and LMNA-si64 was greater than LMNA-si52 at equivalent dose levels Table 43 and Figure 17).

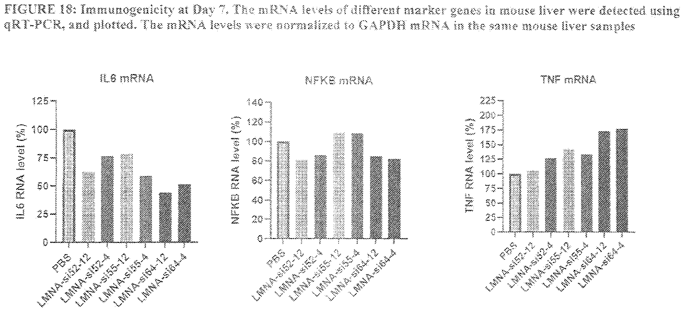

Immunogenicity was assessed after dosing mice with LMNA-si52, LMNA-si55 and LMNA-si64. In general, the immune markers did not change substantially in the siRNA treated mice at Day 7 (Table 44 and Figure 18).

**Table 44: mRNA levels of selected immune response markers in liver samples**

| | PBS | LMNA-si52-12 | LMNA-si52-4 | LMNA-si55-12 | LMNA-si55-4 | LMNA-si64-12 | LMNA-si64-4 |
|---|---|---|---|---|---|---|---|
| Il-6 mRNA % | 100.0 | 62.75 | 76.94 | 78.46 | 59.27 | 44.18 | 51.6 |
| NfKB mRNA % | 100.0 | 80.92 | 86.44 | 109.84 | 108.77 | 85.20 | 82.39 |
| TNF | 100.0 | 106.63 | 126.69 | 142.40 | 133.94 | 173.45 | 177.37 |

### EXAMPLE 11: ADDITIONAL MODIFICATIONS TO IMPROVE THE CHEMICAL MODIFICATION MOTIF

Additional chemical modification patterns were designed for LMNA and assessed to determine whether the new motifs would be beneficial. The motif for LMNA-si47 was the base used to add or change chemical modifications to the antisense strand (Table 45). All antisense strands have a 5'-phosphate.

**Table 45: LMNA Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| LMNA-si2 | ATXL021 | Sense | mG*mC*mGmUmCmAfCm CfAfAfAmAmAmGmCmG mCmAmA*T*T | GCGUCACCA AAAAGCGCA ATT | 16 |
| | ATXL020 | Antisense | mU*fU*mGmCmGfCmUfU fUmUmUmGmGfUmGfAm CmGmC*T*T | UUGCGCUUU UUGGUGACG CTT | 34 |
| LMNA-si42 | ATXL081 | Sense | mG*mC*mGmUrC*mArC* mCfAfAmAmArA*mGrC* mGmCmAmA*mU*mU | GCGUCACCA AAAAGCGCA AUU | 51 |
| | ATXL078 | Antisense | mU*T*mGmCmGfCT*mUf UmUmUmGmGfUmGfAmC mGmC*mU*mU | UTGCGCTUU UUGGUGACG CUU | 64 |
| LMNA-si47 | ATXL081 | Sense | mG*mC*mGmUrC*mArC* mCfAfAmAmArA*mGrC* mGmCmAmA*mU*mU | GCGUCACCA AAAAGCGCA AUU | 51 |
| | ATXL045 | Antisense | mU*fU*mGmCmGfC*TmU fUmUmUmGmGfUmGfAm CmGmC*mU*mU | UUGCGCTUU UUGGUGACG CUU | 72 |
| LMNA-si65 | ATXL154 | Sense | mG*fC*mGmUrC*mArC*m CfAfAmAmArA*mGrC*mG mCfAmA*T*T | GCGUCACCA AAAAGCGCA ATT | 22 |
| | ATXL152 | Antisense | mU*T*mGmCmGfCT*mUf UmUmUfGmGfUmGfAmC mGC*mU*mU | UTGCGCTUU UUGGUGACG CUU | 65 |
| LMNA-si66 | ATXL155 | Sense | mG*fC*mGmUrC*mArC*m CfAfAmAmArA*mGrC*mG mCrA*mA*T*T | GCGUCACCA AAAAGCGCA ATT | 24 |
| | ATXL152 | Antisense | mU*T*mGmCmGfCT*mUf UmUmUfGmGfUmGfAmC mGC*mU*mU | UTGCGCTUU UUGGUGACG CUU | 65 |
| LMNA-si67 | ATXL154 | Sense | mG*fC*mGmUrC*mArC*m CfAfAmAmArA*mGrC*mG mCfAmA*T*T | GCGUCACCA AAAAGCGCA ATT | 22 |
| | ATXL153 | Antisense | mU*T*mGmCmGfCT*mUf UmUmUG*mGfUmGfAmC mGC*mU*mU | UTGCGCTUU UUGGUGACG CUU | 66 |
| LMNA-si68 | ATXL155 | Sense | mG*fC*mGmUrC*mArC*m CfAfAmAmArA*mGrC*mG mCrA*mA*T*T | GCGUCACCA AAAAGCGCA ATT | 24 |
| | ATXL153 | Antisense | mU*T*mGmCmGfCT*mUf UmUmUG*mGfUmGfAmC mGC*mU*mU | UTGCGCTUU UUGGUGACG CUU | 66 |

| | | | | | |
|---|---|---|---|---|---|
| * the oligomeric compounds in this Table of SEQ ID NOs: 16, 34, 51, 64, 72, 65 and 66 do not comprise the motifs claimed | | | | | |

siRNA was transfected at 0-10 nM into HeLa or Hepa1-6 cells with RNAiMAX (Invitrogen, Waltham, MA) and the cells further cultured for 24 hr. siRNA activity was determined by qRT-PCR using the primer probe set in Table 2 and the IC50 of the siRNAs targeting LMNA was calculated (Table 46).

**Table 46: LMNA siRNA Inhibition in HeLa or Hepa1-6 Cells**

| | HeLa Cells (24 hrs) | | Hepa 1-6 Cells (24 hrs) | |
|---|---|---|---|---|
| siRNA | IC50 (nM) | Fold | IC50 (nM) | Fold |
| LMNAsi2 | 7.785 | 1 | 4.321 | 1 |
| LMNAsi42 | 0.126 | 61.7 | 0.497 | 8.7 |
| LMNAsi47 | 0.025 | 305.8 | 0.197 | 21.9 |
| LMNAsi65 | 0.086 | 90.3 | 0.432 | 10 |
| LMNAsi66 | 0.1 | 78 | 0.353 | 12.2 |
| LMNAsi67 | 0.025 | 314.5 | 0.103 | 41.9 |
| LMNAsi68 | 0.022 | 352.4 | 0.314 | 13.8 |

The new design motif for LMNA-si67 demonstrated slightly better activity than LMNA-si47.

### EXAMPLE 12: ADDITIONAL MODIFICATIONS TO VARY THE CHEMICAL MODIFICATIONS IN OLIGOMERIC COMPOUNDS

Additional chemical modification patterns were designed for LMNA and assessed to determine whether the new motifs would be beneficial. The motif for LMNA-si68 was the base used to vary chemical modifications in the oligomeric compound to determine if different 2'-F/2'-OMe modification combinations are beneficial for siRNA activity (Table 47). All antisense strands have a 5'-phosphate.

**Table 47: LMNA Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| LMNA -si71 | ATXL171 | Sense | mG*fC*mGmUrC*mArC*mC fAfArA*mArA*mGrC*mGm CrA*mA*T*T | GCGUCACCAAAAG CGCAATT | 25 |
| | ATXL168 | Antisense | mU*T*mGfCmGfCT*mUfUm UmUfGmGfUmGfAmCmGC* mU*mU | UTGCGCTUUUUGGU GACGCUU | 67 |
| LMNA -si72 | ATXL171 | Sense | mG*fC*mGmUrC*mArC*mC fAfArA*mArA*mGrC*mGm CrA*mA*T*T | GCGUCACCAAAAG CGCAATT | 25 |
| | ATXL169 | Antisense | mU*T*mGC*mGfCT*mUfU mUmUG*mGfUmGfAmCmG C*mU*mU | UTGCGCTUUUUGGU GACGCUU | 68 |
| LMNA -si73 | ATXL171 | Sense | mG*fC*mGmUrC*mArC*mC fAfArA*mArA*mGrC*mGm CrA*mA*T*T | GCGUCACCAAAAG CGCAATT | 25 |
| | ATXL170 | Antisense | mU*T*mGC*mGfCT*mUfU mUmUfGmGfUmGfAmCmG C*mU*mU | UTGCGCTUUUUGGU GACGCUU | 69 |
| LMNA -si74 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL168 | Antisense | mU*T*mGfCmGfCT*mUfUm UmUfGmGfUmGfAmCmGC* mU*mU | UTGCGCTUUUUGGU GACGCUU | 67 |
| LMNA -si75 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL169 | Antisense | mU*T*mGC*mGfCT*mUfU mUmUG*mGfUmGfAmCmG C*mU*mU | UTGCGCTUUUUGGU GACGCUU | 68 |
| LMNA -si76 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL170 | Antisense | mU*T*mGC*mGfCT*mUfU mUmUfGmGfUmGfAmCmG C*mU*mU | UTGCGCTUUUUGGU GACGCUU | 69 |
| LMNA -si77 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL186 | Antisense | mU*T*mGfCmGfCT*mUfUm UmUfGmGfUmGfAmCmGfC mU*mU | UTGCGCTUUUUGGU GACGCUU | 70 |
| LMNA -si78 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL187 | Antisense | mU*T*mGfCmGfCmUmUfU mUmUfGmGfUmGfAmCmGf C*mU*mU | UTGCGCUUUUGGU GACGCUU | 60 |
| LMNA -si79 | ATXL154 | Sense | mG*fC*mGmUrC*mArC*mC fAfAmAmArA*mGrC*mGm CfAmA*T*T | GCGUCACCAAAAAG CGCAATT | 22 |
| | ATXL188 | Antisense | mU*fU*mGfCmGfCmUmUf UmUmUfGmGfUmGfAmCm GfCmU*mU | UUGCGCUUUUGGU GACGCUU | 58 |

siRNA was transfected at 0-10 nM into HeLa cells with RNAiMAX (Invitrogen, Waltham, MA) and the cells further cultured for 24 hr, 72 and 96 hrs. siRNA activity was determined by qRT-PCR using the primer probe set in Table 2 and the IC50 of the siRNAs targeting LMNA was calculated (Table 48).

**Table 48: LMNA siRNA Inhibition in HeLa Cells**

| | HeLa Cells (30 hrs) | | HeLa Cells (72 hrs) | | HeLa Cells (96 hrs) | |
|---|---|---|---|---|---|---|
| siRNA | IC50 (nM) | Fold | IC50 (nM) | Fold | IC50 (nM) | Fold |
| LMNA-si2 | 4.112 | 1 | 0.322 | 1 | 0.519 | 1 |
| LMNA-si47 | 0.087 | 47.4 | 0.031 | 10.2 | 0.115 | 4.5 |
| LMNA-si71 | 0.063 | 65.3 | 0.032 | 10.2 | 0.093 | 5.6 |
| LMNA-si74 | 0.062 | 66.8 | 0.035 | 9.1 | 0.154 | 3.4 |
| LMNA-si75 | 0.072 | 57 | 0.032 | 10.1 | 0.136 | 3.8 |
| LMNA-si77 | 0.092 | 44.7 | 0.044 | 7.4 | 0.117 | 4.4 |
| LMNA-si78 | 0.052 | 79.6 | 0.039 | 8.3 | 0.117 | 4.4 |
| LMNA-si79 | 0.064 | 64.7 | 0.031 | 10.4 | 0.125 | 4.1 |

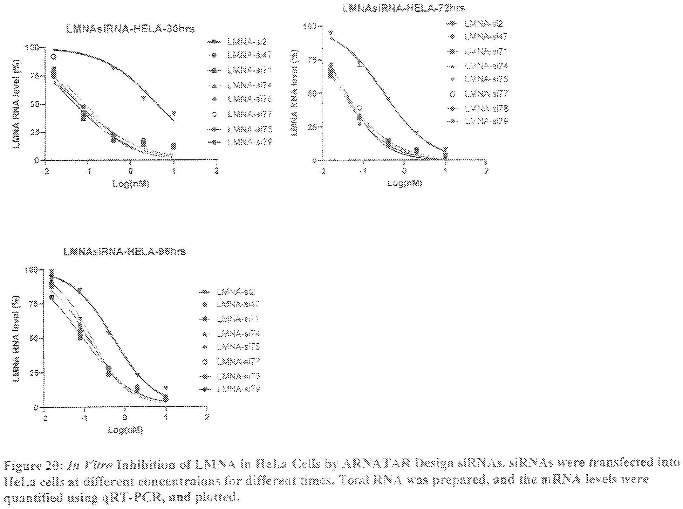

The results show that LMNA-si78 had the highest activity at the earliest time point, 30 hrs, while LMNA-si71, LMNA-si74 and LMNA-si79 also had good activity early on. At the longest time point, 96 hrs, LMNA-si47, LMNA-si71, LMNA-si77 and LMNA-si78 were the most active. ARNATAR designed compounds showed faster onset of activity and higher activity levels at later time points than the benchmark LMNA-si2 (Figure 20). The results indicate, altering 2'-OMe/2'-F modification sites as determined here is tolerated for siRNA activity.

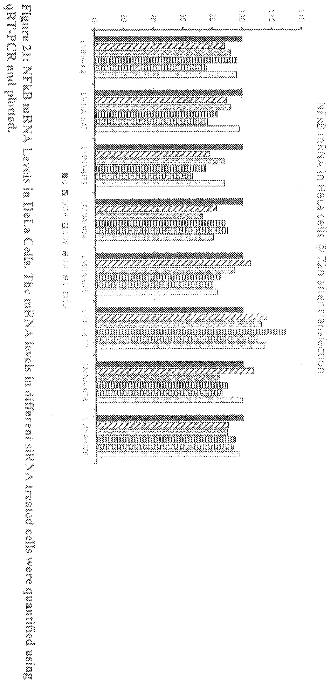

Human NFkB mRNA levels were assessed through qRT-PCT using TaqMan primer probe sets (Table 41) in HeLa cells 72 hrs after transfection. The results show that new ARNATAR siRNA designs are at comparable immunogenicity with the benchmark LMNA-si2 when measured by NFkB (Figure 21).

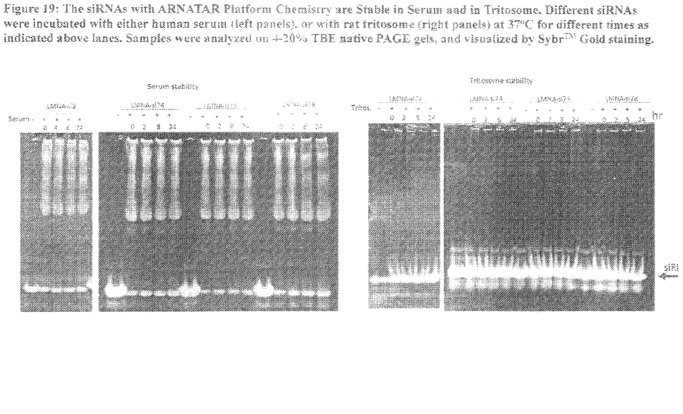

Stability assays were performed on LMNA-si2, LMNA-si74, LMNA-si75 and LMNA-si78. The first assay exposed the compounds human serum to test for stability. The second assay exposed the compounds tritosome to test for stability. The three compounds have comparable stability in serum and tritosome compared to benchmark LMNA-si2 (Figure 19).

### EXAMPLE 13: ADDITIONAL MODIFICATIONS TO SUBSTITUTE DNA BASES FOR RNA BASES IN THE SENSE STRAND

Additional chemical modification patterns were designed for LMNA siRNA and assessed to determine whether the new motifs would be beneficial. The motif for LMNA- si78 was the base used to modify the sense strand by replacing RNA with DNA nucleotides (Table 49). All antisense strands have a 5'-phosphate.

**Table 49: LMNA Oligomeric Compounds with Modifications to Both Strands**

| siRNA Name | Strand Name | Sense or Antisense | Sequence + Chemistry (5' to 3') | Sequence | SEQ ID NO: |
|---|---|---|---|---|---|
| LMNA -si90 | ATXL204 | sense | mG*fC*mGmUC*mAC*mCfAfAm AmAA*mGC*mGmCfAmA*T*T | GCGUCACCAAA AAGCGCAATT | 26 |
| | ATXL187 | antisense | mU*T*mGfCmGfCmUmUfUmUmU fGmGfUmGfAmCmGfC*mU*mU | UTGCGCUUUUU GGUGACGCUU | 60 |
| LMNA -si91 | ATXL204 | sense | mG*fC*mGmUC*mAC*mCfAfAm AmAA*mGC*mGmCfAmA*T*T | GCGUCACCAAA AAGCGCAATT | 26 |
| | ATXL168 | antisense | mU*T*mGfCmGfCT*mUfUmUmUf GmGfUmGfAmCmGC*mU*mU | UTGCGCTUUUU GGUGACGCUU | 67 |

siRNA was transfected at 0-10 nM into HEK293 cells with RNAiMAX (Invitrogen, Waltham, MA) and the cells further cultured for 30 hr. siRNA activity was determined by qRT-PCR using the primer probe set in Table 2 and the IC50 of the siRNAs targeting LMNA was calculated (Table 50).

**Table 50: LMNA siRNA Inhibition in HEK293 Cells**

| | HEK293 Cells (30 hrs) | |
|---|---|---|
| siRNA | IC50 (nM) | Fold |
| LMNA-si2 | 4.712 | 1 |
| LMNA-si74 | 0.346 | 13.6 |
| LMNA-si78 | 0.19 | 24.9 |
| LMNA-si90 | 0.439 | 10.7 |
| LMNA-si91 | 0.335 | 14.1 |

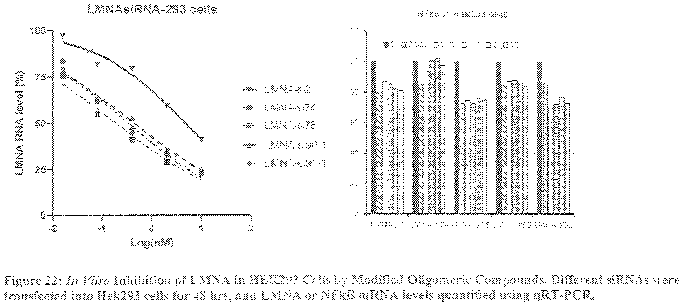

The results show that ARNATAR designed compounds were more potent than benchmark LMNA-si2 (Table 50 and Figure 22).

NFkB mRNA level was assessed through RT-qPCR in HEK293 cells 48 hrs after transfection. The results show that several new ARNATAR siRNA designs are at least, or less, immunogenic when measured by NFkB than the benchmark LMNA-si2 (Figure 22).

### EXAMPLE 14: IN VIVO STUDY CHARACTERIZING ARNATAR DESIGN OLIGOMERIC COMPOUNDS

Select LMNA siRNAs were conjugated with various types of N-acetylgalactosamine (GalNAc) (Table 51) in order to allow free uptake of the compound into cells.

**Table 51: LMNA Oligomeric Compounds with Modifications to Both Strands**

| siRNA Name | Sequence and Chemistry | GalNAc |
|---|---|---|
| LMNA-si52* | LMNA-si2 | GalNAc 1 |
| LMNA-si57* | LMNA-si2 | GalNAc 2 |
| LMNA-si92 | LMNA-si74 | GalNAc 2 |
| LMNA-si93 | LMNA-si75 | GalNAc 2 |
| LMNA-si94 | LMNA-si78 | GalNAc 2 |

| | | |
|---|---|---|
| * both strands of the oligomeric compound do not comprise the motifs claimed | | |

LMNA-si52 was designed to be another benchmark to compare ARNATAR design compounds. It has the sequence and chemistry of LMNA-si2 as described in Example 1 and is conjugated to a GalNAc used by Alnylam in their siRNA compounds such as vutrisiran (Keam, 2022, Drugs, 82:1419-1425; Nair et al., J. Am. Chem. Soc. 2014, 136(49):16958-16961): GalNAc1 (also known as GA1).

LMNA-si57, LMNA-si92, LMNA-si93 and LMNA-si94 use the sequence and chemistry as described in Table 51 and are conjugated to a GalNAc from AM Chemicals (US Patent 10,087,208): GalNAc2 (also known as GalNAc (AM) or GA2).

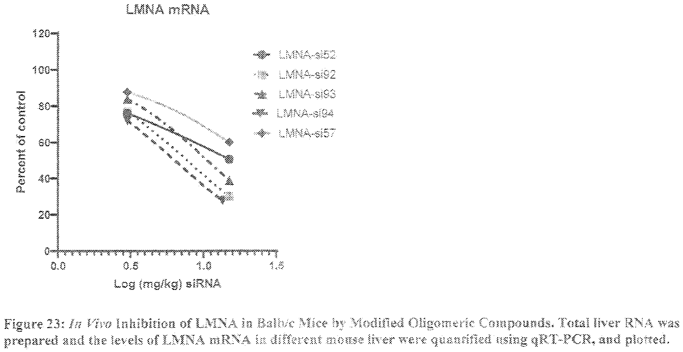

7- to 8-week old Balb/c mice were injected subcutaneously with 3mg/kg or 15mg/kg of GalNAc conjugated siRNA, with 3 mice per group. Phosphate buffered saline (PBS) was injected as a control. Post dosing 3 days, the mice were sacrificed and organs harvested for analysis (Table 52 and Figure 23).

**Table 52: Percent LMNA mRNA in Mouse Liver at Day 3**

| | LMNA mRNA % | |
|---|---|---|
| siRNA | 3 mg/kg | 15 mg/kg |
| LMNA-si52 | 75.96 | 50.57 |
| LMNA-si92 | 76.64 | 30.16 |
| LMNA-si93 | 83.73 | 38.86 |
| LMNA-si94 | 71.98 | 27.77* |
| LMNA-si57 | 87.64 | 60 |

| | | |
|---|---|---|
| * dosed at 13.5 mg/kg due to material limitation | | |

At the 3 mg or 15 mg dose, LMNA-si92, -si93, and -si94 are more potent than the reference siRNA LMNA-si57. These four siRNAs share the same GalNAc2. In addition, LMNA-si52, which is essentially the same as LMNA-si57 but differs only in GalNAc, appears to be more potent than LMNA-si57, suggesting that different GalNAc can affect delivery efficiency. However, this data further suggests that the LMNA-si55 and LMNA-si64 design evaluated in the above described experiment in EXAMPLE 9 (Figure 12) should have higher increase in potency compared with reference siRNA chemistry, if the same GalNAc is used.

### EXAMPLE 15: COMPARISON OF BENCHMARK siRNA TO ARNATAR siRNAs

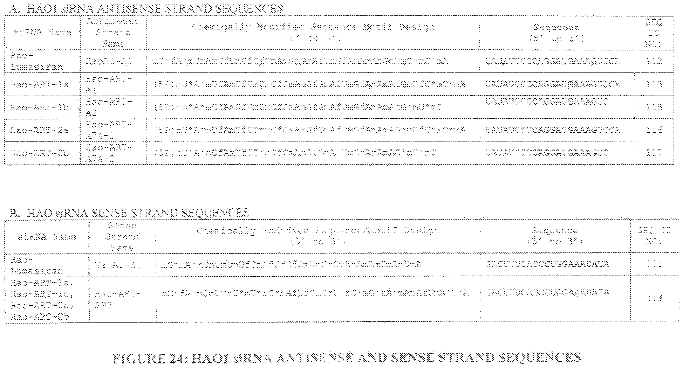

Homo Sapiens Hydroxyacid Oxidase (HAO1) was chosen as a fourth test target and siRNAs designed as shown in Table 53 and Figure 24.

The previous studies used as benchmarks modified siRNAs where the chemical modification pattern reflected that of lumasiran with the exception that the sense strand had an addition TT overhang connected by PS linkages and did not have a GalNAc conjugate, and the antisense strand was 21 nt rather than 23 nt.

In this example, the exact chemical modification motif for lumasiran (without a GalNAc conjugate) was used to modify a siRNA targeting HAO1 (see Friedrich and Aigner for a description of the chemical modification of lumasiran (BioDrugs, 2022, 36(5):549-571)). Additional siRNAs targeting the same HAO1 target sequence were designed based on ARNATAR motifs.

The HAO-lumasiran benchmark and ARNATAR motif siRNAs were tested head-to-head to compare HAO1 inhibition efficacy. All ARNATAR designed antisense strands have a 5'-phosphate.

**Table 53: HAO1 Oligomeric Compounds with Modifications to Both Strands**

| siRNA Name | Strand Name | Sense or Antisense | Sequence + Chemistry* (5' to 3') | Sequence | SEQ ID NO: |
|---|---|---|---|---|---|
| Hao-Lumasiran | HaoAl-S1 | sense | mG*mA*mCmUmUmUfCmAfUfCf CmUmGmGmAmAmAmUmAmUm A | GACUUUCAUCC UGGAAAUAUA | 111 |
| | HaoAl-A1 | Antisense | mU*fA*mUmAmUfUmUfCfCmAm GmGmAfUmGfAmAmAmGmUmC *mC*mA | UAUAUUUCCAG GAUGAAAGUCC A | 112 |
| Hao-ART-1a | Hao-ART-S97 | sense | mG*fA*mCmU*rU*mU*rC*mAfUf CmCmU*rG*mG*rA*mAmAfUmA *T*A | GACUUUCAUCC UGGAAAUATA | 114 |
| | Hao-ART-A1 | Antisense | mU*A*mUfAmUfUmUmCfCmAm GfGmAfUmGfAmAmAfGmUfC*m C*mA | UAUAUUUCCAG GAUGAAAGUCC A | 113 |
| Hao-ART1b | Hao-ART-S97 | sense | mG*fA*mCmU*rU*mU*rC*mAfUf CmCmU*rG*mG*rA*mAmAfUmA *T*A | GACUUUCAUCC UGGAAAUATA | 114 |
| | Hao-ART-A2 | Antisense | mU*A*mUfAmUfUmUmCfCmAm GfGmAfUmGfAmAmAfG*mU*mC | UAUAUUUCCAG GAUGAAAGUC | 115 |
| Hao-ART2a | Hao-ART-S97 | sense | mG*fA*mCmU*rU*mU*rC*mAfUf CmCmU*rG*mG*rA*mAmAfUmA *T*A | GACUUUCAUCC UGGAAAUATA | 114 |
| | Hao-ART-A74-1 | Antisense | mU*A*mUfAmUfUT*mCfCmAmGf GmAfUmGfAmAmAG*mUfC*mC* mA | UAUAUUTCCAG GAUGAAAGUCC A | 116 |
| Hao-ART2b | Hao-ART-S97 | sense | mG*fA*mCmU*rU*mU*rC*mAfUf CmCmU*rG*mG*rA*mAmAfUmA *T*A | GACUUUCAUCC UGGAAAUATA | 114 |
| | Hao-ART-A74-2 | Antisense | mU*A*mUfAmUfUT*mCfCmAmGf GmAfUmGfAmAmAG*mU*mC | UAUAUUTCCAG GAUGAAAGUC | 117 |

| | | | | | |
|---|---|---|---|---|---|
| * the oligomeric compounds of this Table of SEQ ID NOs: 111, 112, 113 and 116 do not have the motifs claimed | | | | | |

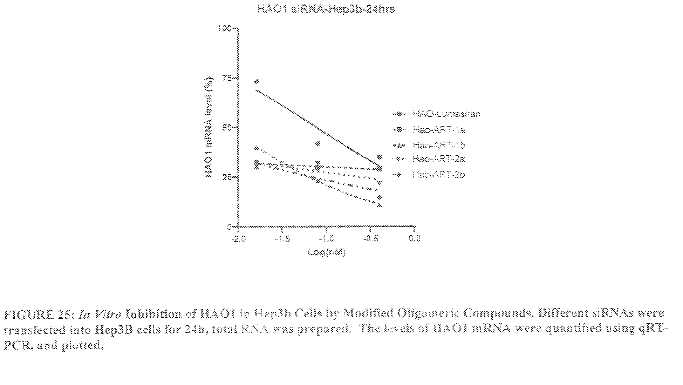

siRNA was transfected at 0-0.4 nM into Hep3b cells with RNAiMAX (Invitrogen, Waltham, MA) and the cells cultured for 24 hr. siRNA activity was determined by a HAO1 gene expression assay from ThermoFisher Scientific (catalog #Hs00213909_m1; Waltham, MA, USA) and the amount of HAO1 mRNA left after siRNA knockdown was assessed (Table 54 and Figure 25).

**Table 54: HAO1 siRNA Inhibition in Hep3b Cells After 24 Hrs**

| | HAO1 mRNA level % | | | |
|---|---|---|---|---|
| siRNA | 0 nM | 0.016 nM | 0.06 nM | 0.4 nM |
| Hao-Lumasiran | 100 | 73.2 | 48.9 | 35 |
| Hao-ART-1a | 100 | 32.3 | 29.5 | 29 |
| Hao-ART-1b | 100 | 40.3 | 23.9 | 11.3 |
| Hao-ART-2a | 100 | 30.4 | 27 | 21.6 |
| Hao-ART-2b | 100 | 29.6 | 29.6 | 14.5 |

The ARNATAR designed siRNAs showed better inhibition rates than the benchmark siRNA.

### EXAMPLE 16: ADDITIONAL CHEMICAL MODIFICATIONS TO EVALUATE THE EFFECTS ON siRNA ACTIVITY BY 5'-PHOSPHATE ON THE ANTISENSE STRAND AND PHOSPHOROTHIOATE ON THE SENSE STRAND

Additional chemical modification patterns were designed for LMNA siRNA (Table 55) and assessed to determine whether the new motifs would be beneficial.

Prior siRNAs evaluated above contain 5'-phosphate on the antisense strand, which is required for Ago2 binding and function. Since siRNAs can be phosphorylated in cells (Weitzer S, Martinez J. Nature, 2007, 447(7141):222-226), the 5'-phosphate may not be necessarily added during siRNA synthesis. Thus, the activity of siRNAs with or without 5' phosphate was evaluated; LMNA-si94 is previously disclosed LMNA-si78 with a 5'-phosphate on the antisense strand and the addition of a GalNAc conjugate on the sense strand. LMNA-si95 is LMNA-si94 with a 5'-OH instead of a 5'-phosphate on the antisense strand.

Additionally, the effects of the position and number of phosphorothioate (5' or 3', or both 5' and 3') around DNA or RNA nucleotides in the sense strand was evaluated; LMNA-si96 to LMNA-si100 was designed based on previously disclosed LMNA-si78 or LMNA-si90. LMNA-si96 is LMNA-si90 with a GalNAc conjugate. siRNA compounds LMNA-si97, LMNA-si98, LMNA-si99 and LMNA-si100 comprise the antisense strand of LMNA-si78 and a sense strand with a newly designed motif moving the phosphorothioate to various locations and a GalNAc conjugate.

The GalNAc conjugate used in this study is known herein as GalNAc2 (AM Chemicals, US Patent 10,087,208).

**Table 55: LMNA Oligomeric Compounds with Modifications to Both Strands**

| siRNA Name | Strand Name | Sense or Antisense | Sequence + Chemistry (5' to 3') | Sequence | SEQ ID NO: |
|---|---|---|---|---|---|
| LMNA -si94 | ATXL154 GA | Sense | mG*fC*mGmUrC*mArC*mCfAfAm AmArA*mGrC*mGmCfAmA*T*T- GalNAc2 | GCGUCACCAAA AAGCGCAATT | 23 |
| | ATXL187 | Antisense | mU*T*mGfCmGfCmUmUfUmUmUf GmGfUmGfAmCmGfC*mU*mU | UTGCGCUUUUU GGUGACGCUU | 60 |
| LMNA -si95 | ATXL154 GA | sense | mG*fC*mGmUrC*mArC*mCfAfAm AmArA*mGrC*mGmCfAmA*T*T- GalNAc2 | GCGUCACCAAA AAGCGCAATT | 23 |
| | ATXL187 OH | antisense | (OH)mU*T*mGfCmGfCmUmUfUm UmUfGmGfUmGfAmCmGfC*mU* mU | UTGCGCUUUUU GGUGACGCUU | 60 |
| LMNA -si96 | ATXL206 | sense | mG*fC*mGmUC*mAC*mCfAfAmA mAA*mGC*mGmCfAmA*T*T- GalNAc2 | GCGUCACCAAA AAGCGCAATT | 27 |
| | ATXL187 | antisense | mU*T*mGfCmGfCmUmUfUmUmUf GmGfUmGfAmCmGfC*mU*mU | UTGCGCUUUUU GGUGACGCUU | 60 |
| LMNA -si97 | ATXL207 | sense | mG*fC*mGmU*rC*mA*rC*mCfAfA mAmA*rA*mG*rC*mGmCfAmA*T *T-GalNAc2 | GCGUCACCAAA AAGCGCAATT | 28 |
| | ATXL187 | antisense | mU*T*mGfCmGfCmUmUfUmUmUf GmGfUmGfAmCmGfC*mU*mU | UTGCGCUUUUU GGUGACGCUU | 60 |
| LMNA -si98 | ATXL208 | sense | mG*fC*mGmUfCmAfCmCfAfAmA mAfAmGfCmGmCfAmA*T*T- GalNAc2 | GCGUCACCAAA AAGCGCAATT | 29 |
| | ATXL187 | antisense | mU*T*mGfCmGfCmUmUfUmUmUf GmGfUmGfAmCmGfC*mU*mU | UTGCGCUUUUU GGUGACGCUU | 60 |
| LMNA -si99 | ATXL209 | sense | mG*fC*mGmU*C*mA*C*mCfAfA mAmA*A*mG*C*mGmCfAmA*T* T-GalNAc2 | GCGUCACCAAA AAGCGCAATT | 31 |
| | ATXL187 | antisense | mU*T*mGfCmGfCmUmUfUmUmUf GmGfUmGfAmCmGfC*mU*mU | UTGCGCUUUUU GGUGACGCUU | 60 |
| LMNA -si100 | ATXL210 | sense | mG*fC*mGmU*CmA*CmCfAfAmA mA*AmG*CmGmCfAmA*T*T- GalNAc2 | GCGUCACCAAA AAGCGCAATT | 32 |
| | ATXL187 | antisense | mU*T*mGfCmGfCmUmUfUmUmUf GmGfUmGfAmCmGfC*mU*mU | UTGCGCUUUUU GGUGACGCUU | 60 |

### In Vitro Assay

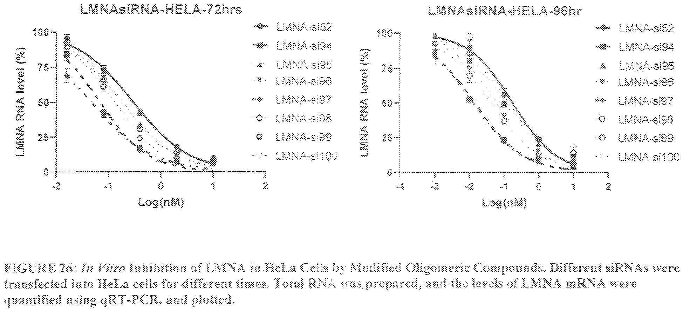

siRNA was transfected at 0-10 nM into HeLa cells with RNAiMAX (Invitrogen, Waltham, MA) and the cells further cultured for 72 hr and 96 hr. siRNA activity was determined by qRT-PCR using the primer probe set in Table 2 and the IC50 values of the siRNAs targeting LMNA were calculated (Table 56) and the percent inhibition of LMNA RNA levels graphed (Figure 26).

**Table 56: LMNA siRNA Inhibition in HeLa Cells**

| | HeLa Cells (72 hrs) | | HeLa Cells (96 hrs) | |
|---|---|---|---|---|
| siRNA | IC50 (nM) | Fold | IC50 (nM) | Fold |
| LMNA-si52 | 0.3 | 1 | 0.18 | 1 |
| LMNA-si94 | 0.07 | 4.31 | 0.014 | 12.54 |
| LMNA-si95 | 0.18 | 1.63 | 0.11 | 1.64 |
| LMNA-si96 | 0.19 | 1.56 | 0.06 | 2.89 |
| LMNA-si97 | 0.05 | 6.33 | 0.01 | 13.67 |
| LMNA-si98 | 0.18 | 1.64 | 0.14 | 1.3 |
| LMNA-si99 | 0.13 | 2.27 | 0.04 | 4.15 |
| LMNA-si100 | 0.19 | 1.6 | 0.18 | 1.02 |

The results indicate that siRNA with an antisense strand with 5'-phosphate (LMNA-si94) showed better activity than siRNA with an antisense strand with 5'-OH (LMNA-si95), and that siRNA can tolerate phosphorothioate in the sense strand at both 5' and 3' of DNA or RNA nucleotide. In addition, phosphorothioate in the sense strand at 3' of a DNA nucleotide (LMNA-si96) appears slightly better than at 5' of a DNA nucleotide (LMNA-si100). The results also show that ARNATAR designed compounds were more potent than benchmark LMNA-si52 *in vitro.* (Table 56 and Figure 26).

### In Vivo Assay

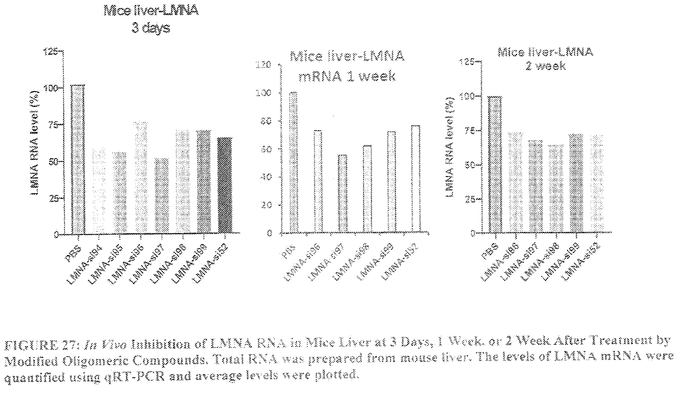

Several siRNA compounds found to be potent in the *in vitro* inhibition assay above was assessed *in vivo;* 6- to 8-week old male Balb/c mice were injected subcutaneously with 3 mg/kg of siRNA, with 3 mice per group. Groups of mice were assessed at 3-days, 1-week or 2-weeks. Phosphate buffered saline (PBS) was injected as a control. Post dosing 3-days, 1-week or 2-week, the mice were sacrificed and organs harvested for analysis (Table 57 and Figure 27). No groups of mice treated with LMNA-si94 or LMNA-si95 were taken into the 1-week or 2-week time points.

siRNA activity was determined by measuring the levels of target mRNA through qRT-PCR using the LMNA primer probe set listed in Table 2. qRT-PCR was performed using AgPath-ID^{™} One-Step RT-PCR Reagents in QS3 real-time PCR system (ThermoFisher Scientific, Waltham, MA, USA). The target RNA levels detected in qRT-PCR assay were normalized to GAPDH mRNA levels detected in the aliquots of RNA samples using qRT-PCR.

**Table 57: Percent LMNA mRNA in Mouse Liver at Day 3, 1 Week or 2 Week**

| | LMNA Knockdown Levels (% Inhibition) | | |
|---|---|---|---|
| siRNA | 3-Day | 1-Week | 2-Week |
| LMNA-si52 | 65.5 | 72.5 | 71.3 |
| LMNA-si94 | 59.5 | n/a | n/a |
| LMNA-si95 | 56.0 | n/a | n/a |
| LMNA-si96 | 76.5 | 69.6 | 73.3 |
| LMNA-si97 | 52.0 | 53.0 | 67.9 |
| LMNA-si98 | 70.5 | 59.2 | 64.3 |
| LMNA-si99 | 70.5 | 68.6 | 72.2 |

The *in vivo* results show that, when dosed at 3 mg/kg, the motif designs for LMNA-si94, LMNA-si95 and LMNA-si97 were more potent than the benchmark compound LMNA-si52 at the 3-day period. At the 1- and 2-week time periods, LMNA-si97 and LMNA-si98 were more potent inhibitors than LMNA-si52. No mice treated with LMNA-si94 or LMNA-si95 were carried through more than 3-days post-treatment.

### EXAMPLE 17: ADDITIONAL MODIFICATIONS TO INCREASE siRNA DURABILITY

Sense and antisense strands used to design LMNA siRNA in previous examples were mixed and matched in different combinations (Table 58) and assessed to determine whether the new siRNA compounds would be potent. The sense strands from LMNA-si97 (ATXL207) and LMNA-si98 (ATXL208), which showed durable inhibition activity at the 2-week time point in the previous example, was paired with different antisense strands from siRNA previously shown to be potent LMNA inhibitor (LMNA-si74) or stable (LMNA-si79). Each antisense strand has a 5'-phosphate and each sense strand has a GalNAc2 conjugate.

**Table 58: LMNA Oligomeric Compounds with Modifications to Both Strands**

| siRNA Name | Strand Name | Sense or Antisense | Sequence + Chemistry (5' to 3') | Sequence | SEQ ID NO: |
|---|---|---|---|---|---|
| LMNA -si103 | ATXL208 | sense | mG*fC*mGmUfCmAfCmCfAfAmA mAfAmGfCmGmCfAmA*T*T- GalNAc2 | GCGUCACCAAA AAGCGCAATT | 29 |
| | ATXL168 | antisense | mU*T*mGfCmGfCT*mUfUmUmUf GmGfUmGfAmCmGC*mU*mU | UTGCGCTUUUU GGUGACGCUU | 67 |
| LMNA -si104 | ATXL208 | sense | mG*fC*mGmUfCmAfCmCfAfAmA mAfAmGfCmGmCfAmA*T*T- GalNAc2 | GCGUCACCAAA AAGCGCAATT | 29 |
| | ATXL188 | antisense | mU*fU*mGfCmGfCmUmUfUmUm UfGmGfUmGfAmCmGfCmU*mU | UUGCGCUUUUU GGUGACGCUU | 58 |
| LMNA -si105 | ATXL207 | sense | mG*fC*mGmU*rC*mA*rC*mCfAf AmAmA*rA*mG*rC*mGmCfAmA *T*T-GalNAc2 | GCGUCACCAAA AAGCGCAATT | 28 |
| | ATXL168 | antisense | mU*T*mGfCmGfCT*mUfUmUmUf GmGfUmGfAmCmGC*mU*mU | UTGCGCTUUUU GGUGACGCUU | 67 |
| LMNA -si106 | ATXL207 | sense | mG*fC*mGmU*rC*mA*rC*mCfAf AmAmA*rA*mG*rC*mGmCfAmA *T*T-GalNAc2 | GCGUCACCAAA AAGCGCAATT | 28 |
| | ATXL188 | antisense | mU*fU*mGfCmGfCmUmUfUmUm UfGmGfUmGfAmCmGfCmU*mU | UUGCGCUUUU GGUGACGCUU | 58 |

### In Vitro Assay

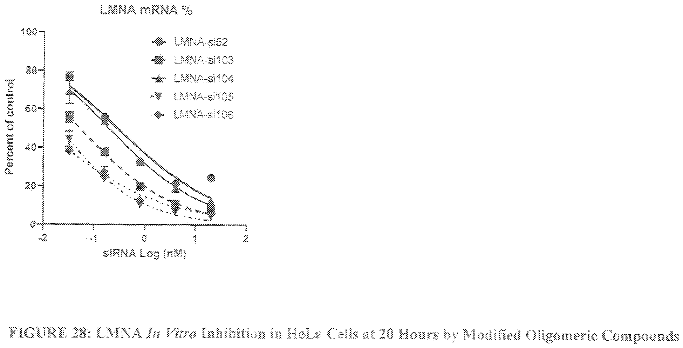

siRNA was transfected at 0-10 nM into HeLa cells with RNAiMAX (Invitrogen, Waltham, MA) and the cells further cultured for 20 hr. siRNA activity was determined by qRT-PCR using the primer probe set in Table 2 and the IC50 values of the siRNAs targeting LMNA were calculated (Table 59) and the percent inhibition of LMNA RNA levels graphed (Figure 28).

**Table 59: LMNA siRNA Inhibition in HeLa Cells**

| | HeLa Cell (20 hrs) | |
|---|---|---|
| siRNA | IC50 (nM) | Fold |
| LMNA-si52 | 0.287 | 1.0 |
| LMNA-si103 | 0.052 | 5.5 |
| LMNA-si104 | 0.194 | 1.5 |
| LMNA-si105 | 0.021 | 14.0 |
| LMNA-si106 | 0.009 | 30.9 |

siRNAs with LMNA-si97 sense strand that contains several RNA nucleotides showed better activity, while the siRNA (LNMA-si103) with a fully 2'-OMe/F modified sense strand and a DNA modified antisense strand also improved activity compared with reference siRNA (LMNA-si52). However, the siRNA (LMNA-si104) that is fully 2'-OMe/F modified in both sense and antisense had slightly better activity compared with the reference siRNA.

### EXAMPLE 18: ADDITIONAL MODIFICATIONS TO INCREASE siRNA DURABILITY

Sense and antisense strands used to design LMNA siRNA in previous examples were mixed and matched in different combinations (LMNA-si107 and LMNA-si108) or a non-GalNAc version of LMNA-103 (LMNA-si109) as shown in Table 60, and assessed to determine whether the new siRNA compounds would be potent. Each antisense strand has a 5'-phosphate. The sense strand of LMNA-si107 and LMNA-si108 has a GalNAc2 conjugate.

**Table 60: LMNA Oligomeric Compounds with Modifications to Both Strands**

| siRNA Name | Strand Name | Sense or Antisense | Sequence + Chemistry (5' to 3') | Sequence | SEQ ID NO: |
|---|---|---|---|---|---|
| LMNA -si107 | ATXL206 | sense | mG*fC*mGmUC*mAC*mCfAfAmA mAA*mGC*mGmCfAmA*T*T- GalNAc2 | GCGUCACCAAA AAGCGCAATT | 27 |
| | ATXL168 | antisense | mU*T*mGfCmGfCT*mUfUmUmUf GmGfUmGfAmCmGC*mU*mU | UTGCGCTUUUU GGUGACGCUU | 67 |
| LMNA -si108 | ATXL154 GA | sense | mG*fC*mGmUrC*mArC*mCfAfAm AmArA*mGrC*mGmCfAmA*T*T - GalNAc2 | GCGUCACCAAA AAGCGCAATT | 23 |
| | ATXL168 | antisense | mU*T*mGfCmGfCT*mUfUmUmUf GmGfUmGfAmCmGC*mU*mU | UTGCGCTUUUU GGUGACGCUU | 67 |
| LMNA -si109 | ATXL232 | sense | mG*fC*mGmUfCmAfCmCfAfAmA mAfAmGfCmGmCfAmA*T*T | GCGUCACCAAA AAGCGCAATT | 30 |
| | ATXL168 | antisense | mU*T*mGfCmGfCT*mUfUmUmUf GmGfUmGfAmCmGC*mU*mU | UTGCGCTUUUU GGUGACGCUU | 67 |

### In Vitro Assays

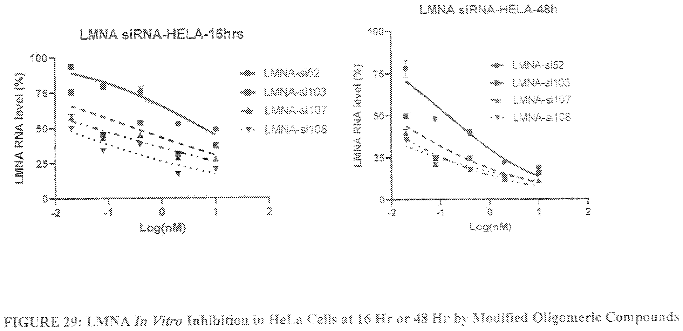

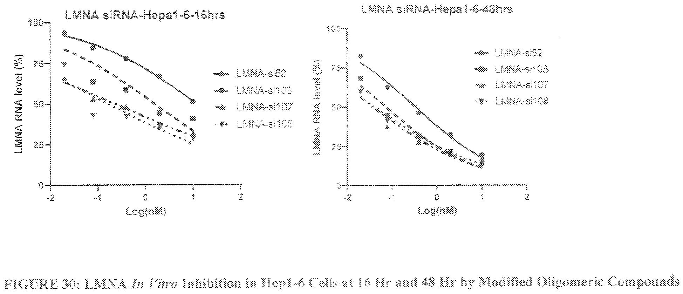

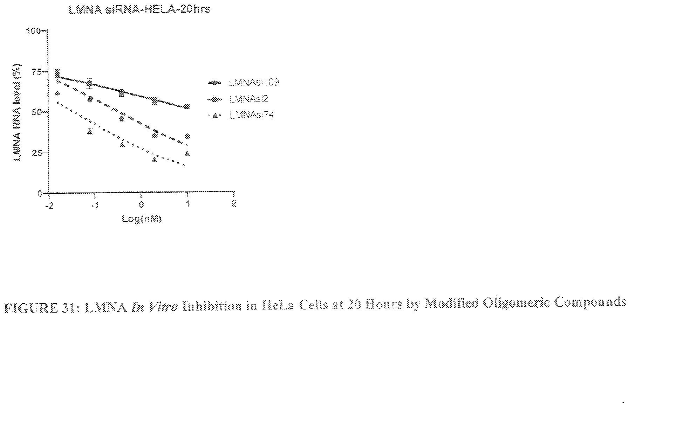

siRNA was transfected at 0-10 nM into HeLa cells or Hepa1-6 cells with RNAiMAX (Invitrogen, Waltham, MA) and the cells further cultured for various times as indicated in the tables. siRNA activity was determined by qRT-PCR using the primer probe set in Table 2 and the IC50 values of the siRNAs targeting LMNA were calculated (Tables 61-63) and the percent inhibition of LMNA RNA levels graphed (Figures 29-31).

**Table 61: LMNA siRNA Inhibition in HeLa Cells**

| | HeLa Cells (16 hrs) | | HeLa Cells (48 hrs) | |
|---|---|---|---|---|
| siRNA | IC50 (nM) | Fold | IC50 (nM) | Fold |
| LMNA-si52 | 5.76 | 1 | 0.15 | 1 |
| LMNA-si103 | 0.31 | 18.4 | 0.009 | 15.8 |
| LMNA-si107 | 0.06 | 100.3 | 0.003 | 44.6 |
| LMNA-si108 | 0.013 | 434.1 | 0.0006 | 234.8 |

**Table 62: LMNA siRNA Inhibition in Hepa1-6 Cells**

| | Hepa1-6 Cells (16 hrs) | | Hepa1-6 Cells (48 hrs) | |
|---|---|---|---|---|
| siRNA | IC50 (nM) | Fold | IC50 (nM) | Fold |
| LMNA-si52 | 11.83 | 1 | 0.343 | 1 |
| LMNA-si103 | 1.58 | 7.5 | 0.075 | 4.6 |
| LMNA-si107 | 0.23 | 50.6 | 0.033 | 10.5 |
| LMNA-si108 | 0.17 | 71.2 | 0.043 | 8 |

**Table 63: LMNA siRNA Inhibition in HeLa Cells**

| | HeLa Cell (20 hrs) | |
|---|---|---|
| siRNA | IC50 (nM) | Fold |
| LMNAsi2 | 15.43 | 1 |
| LMNAsi109 | 0.33 | 47 |
| LMNAsi74 | 0.04 | 438 |

The results indicate that the new siRNA combinations generally showed better activity than the reference siRNAs LMNA-si2 or LMNA-si52, while the two siRNAs containing RNA nucleotides in sense strand (LMNA-si107, LMNA-si108) have even better activity.

### EXAMPLE 19: CHARACTERIZATION OF THE DURATION OF siRNA ACTIVITY IN VIVO

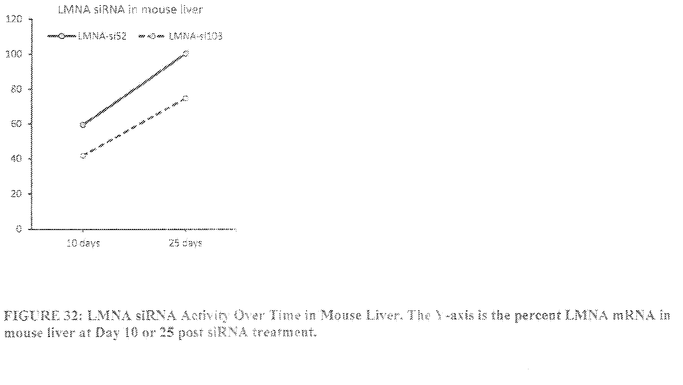

In addition to activity, another important factor for a siRNA therapeutic is the duration of action in vivo. To evaluate the duration of action of siRNAs with newly modified patterns, siRNAs LMNA-si103 and reference siRNA LMNA-si52 were dosed subcutaneously at 5 mg/kg into 7-week BalB/C mice, and sacrificed at 10- or 25-days after siRNA injection. LMNA mRNA levels in total liver were analyzed using qRT-PCR with the primer probe set as described in Table 2. The results are shown in Table 64. Substantially better knockdown was observed for LMNA-103 than for reference siRNA LMNA-si52 in vivo. In addition, LMNA-si103 and reference LMNA-si52 have similar recovery slopes (Figure 32), suggesting that the newly modified siRNA has a better activity and a comparable duration with the reference siRNA in animals.

**Table 64: Percent LMNA mRNA in Mouse Liver at Day 10 or 25**

| | LMNA Knockdown Levels (% Inhibition) | |
|---|---|---|
| | 10 days | 25 days |
| PBS | 100 | 100 |
| LMNA-si52 | 59.42 | 100.45 |
| LMNA-si103 | 41.74 | 74.80 |

### EXAMPLE 20: COMPARISON OF ARNATAR siRNA PLATFORM DESIGN WITH THIRD PARTY siRNA PLATFORM DESIGN

As disclosed in Example 1, LMNA-si2 benchmark was designed based on the chemical modification design of a commercially available therapeutic, Lumasiran. Benchmark LMNA-si52 is LMNA-si2 with the addition of a GalNAc conjugate (Example 8). However, to minimize variables in the above experiments, LMNA-si2 (and LMNA-si52) differed from Lumasiran in that it was designed to have a LMNA sequence, a structure with overhangs at both ends, and 21nt length.

In this study, the chemical modification platform ESC Plus (ESC+) (Hu et al., Therapeutic siRNA: State of the Art, Signal Transduction and Targeted Therapy, 2020, 5:101) was used to design benchmark siRNAs for three targets: LMNA, NCL and ApoC3. As described by Hu et al., these new benchmark siRNAs have 21nt sense strands, 23nt antisense strands, without 3' overhangs in the sense strands, and 2 natural nucleotides overhanging at the 3' end of antisense strands (forming 23nt base-pairing with mRNA target). Also, the 5' ends of the antisense strands have no phosphates and position 7 in the antisense strands are "GNA" modified nucleotides when commercially available. To form a better benchmark siRNA, the antisense siRNAs share the same 21nt sequence from the 5' end to ensure similar seed sequences. The benchmark siRNAs are LMNA-si111, NCL-ALN, ApoC3-AL as shown in Table 65.

ARNATAR designed 21nt siRNAs targeting NCL and ApoC3 were designed using the LMNA-74 chemical motif as shown in Table 65. The ARNATAR designed siRNAs have a 5'-phosphate on the antisense strand.

The siRNAs were made without GalNAc conjugates in order to only compare the chemical modification motifs.

**Table 65: Oligomeric Compounds with Different Designs for NCL, LMNA, and APOC3 mRNAs**

| siRNA Name | Strand Name | Sense or Antisense | Sequence + Chemistry* (5' to 3') | Sequence | SEQ ID NO: |
|---|---|---|---|---|---|
| LMNA-74 | ATXL-154 | Sense | mG*fC*mGmUrC*mArC*mCfAf AmAmArA*mGrC*mGmCfAmA *T*T | GCGUCACC AAAAAGCG CAATT | 22 |
| | ATXL-168 | Antisense | (5p)mU*T*mGfCmGfCT*mUfU mUmUfGmGfUmGfAmCmGC*m U*mU | UTGCGCTUU UUGGUGAC GCUU | 67 |
| LMNA-si111 | ATXL-239 | Sense | mC*mA*mGmCmGmUfCmAfCf CfAmAmAmAmAmGmCmGmC mAmA | CAGCGUCA CCAAAAAG CGCAA | 118 |
| | ATXL238 | Antisense | mU*fU*mGmCmGfCgnaTfUfUm UmUmGmGfUmGfAmCmGmCm UmG*mC*mC | UUGCGCTU UUUGGUGA CGCUGCC | 119 |
| NCL-74 | NCL-74s | Sense | mG*fG*mAmCrA*mUrU*mCfCf AmAmGrA*mCrA*mGmUfAmU *T*T | GGACAUUC CAAGACAG UAUTT | 120 |
| | NCL-74a | Antisense | (5p)mA*T*mAfCmUfGT*mCfU mUmGfGmAfAmUfGmUmCC*m U*mU | ATACUGTCU UGGAAUGU CCUU | 121 |
| NCL-ALN | NCL-Als1 | Sense | mG*mA*mGmGmAmCfAmUfUf CfCmAmAmGmAmCmAmGmU mAmU | GAGGACAU UCCAAGAC AGUAU | 122 |
| | NCL-Ala | Antisense | mA*fU*mAmCmUfGgnaTfCfUm UmGmGmAfAmUfGmUmCmCm UmC*mA*mG | AUACUGTC UUGGAAUG UCCUCAG | 123 |
| ApoC3-74 | Apoc3-74s | Sense | mC*fU*mCmUrG*mArG*mUfUf CmUmGrG*mGrA*mUmUfUmG *T*T | CUCUGAGU UCUGGGAU UUGTT | 76 |
| | Apoc3-74a | Antisense | (5p)mC*A*mAfAmUfCC*mCfA mGmAfAmCfUmCfAmGmAG*m U*mU | CAAAUCCC AGAACUCA GAGUU | 86 |
| ApoC3-AL | Apoc3-ALs | Sense | mU*mU*mCmUmCmUfGmAfGf UfUmCmUmGmGmGmAmUmU mUmG | UUCUCUGA GUUCUGGG AUUUG | 124 |
| | Apoc3-ALa | Antisense | mC*fA*mAmAmUfCgnaCfCfAm GmAmAmCfUmCfAmGmAmGm AmA*mC*mU | CAAAUCCC AGAACUCA GAGAACU | 125 |

| | | | | | |
|---|---|---|---|---|---|
| * the oligomeric compounds of this Table of SEQ ID NOs: 118, 119, 122, 123, 124 and 125 do not have the motifs claimed | | | | | |

### In Vitro Assays

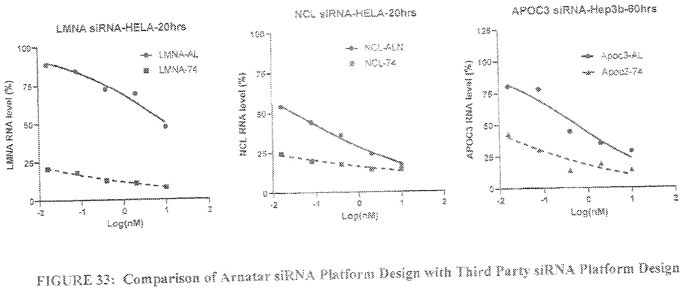

siRNA was transfected at 0-10 nM into HeLa cells with RNAiMAX (Invitrogen, Waltham, MA) for LMNA and NCL siRNAs and the cells further cultured for 20 hr. For ApoC3 siRNA, it was transfected into Hep3B cells at 0-10 nM using RNAiMax and cells were incubated for 60 hr. siRNA activity was determined by qRT-PCR using the primer probe sets as listed in Table 2, 30, 19. The IC50 of the siRNAs targeting corresponding mRNAs are shown in Table 66 and the percent inhibition of LMNA RNA levels graphed (Figure 33).

**Table 66. The activity of siRNAs targeting different mRNAs in HeLa or Hep3B cells**

| | Cell type | Treated time (hour) | siRNAs | IC50 (nM) | Fold |
|---|---|---|---|---|---|
| LMNA mRNA | HeLa | 20 | LMNA-74 | ~0.0000069 | >1000 |
| | | | LMNA-si111 | 10.46 | 1 |
| NCL mRNA | HeLa | 20 | NCL-74 | ~0.00000061 | >1000 |
| | | | NCL-ALN | 0.033 | 1 |
| ApoC3 mRNA | Hep3B | 60 | ApoC3-74 | 0.004 | 140 |
| | | | ApoC3-AL | 0.56 | 1 |

The results showed that the newly designed siRNAs dramatically improved activity compared with the ESC+ design. Also, the ARNATAR design motif generally conveyed increased activity irrespective of mRNA target or siRNA sequence.

### EXAMPLE 21: COMPARISON OF siRNA WITH VARYING LENGTHS TARGETING LMNA, NCL AND AGT mRNAs

Previously tested siRNA compounds were mainly 21nt long, with two overhangs at both ends. Since the length of siRNAs generated in cells by Dicer cleavage may vary (e.g., from about 21nt to 23nt), the effects of varying siRNA length on siRNA activity were evaluated. To this end, siRNAs 21-mer or 23-mer lengths were designed with ARNATAR motifs to target NCL (NCL-74 and NCL-23nt) and LMNA (LMNA-si110) as shown in Table 67. The ARNATAR design motifs include, among other design elements, 2nt overhangs at both strands and 5'-phosphate on the antisense strand. 23mer siRNAs from previous Examples with non-ARNATAR chemical modifications were used as benchmarks (LMNA-si111, NCL-ALN). The siRNAs were made without GalNAc conjugates in order to only compare the chemical modification motifs.

**Table 67: Modified LMNA Oligomeric Compounds of Varying Lengths**

| siRNA Name | Strand Name | Sense or Antisense | Sequence + Chemistry* (5' to 3') | Sequence | SEQ ID NO: |
|---|---|---|---|---|---|
| LMNA -si110 | ATXL237 | sense | mC*fA*mGmCrG*mUrC*mAfCfC mAmArA*mArA*mGmCfGmCmA fA*T*T | CAGCGUCACC AAAAAGCGCA ATT | 126 |
| | ATXL236 | antisense | (5p)mU*T*mGfCmGfCT*mUfUm UmUfGmGfUmGfAmCmGC*mUm G*C*C | UTGCGCTUUU UGGUGACGCU GCC | 127 |
| LMNA -si111 | ATXL239 | sense | mC*mA*mGmCmGmUfCmAfCfCf AmAmAmAmAmGmCmGmCmA mA | CAGCGUCACC AAAAAGCGCA A | 118 |
| | ATXL238 | antisense | mU*fU*mGmCmGfCgnaTfUfUmU mUmGmGfUmGfAmCmGmCmUm G*mC*mC | UUGCGCTUUU UGGUGACGCU GCC | 119 |
| NCL-ALN | NCL-Als1 | Sense | mG*mA*mGmGmAmCfAmUfUfC fCmAmAmGmAmCmAmGmUmA mU | GAGGACAUUC CAAGACAGUA U | 122 |
| | NCL-ALa | Antisense | mA*fU*mAmCmUfGgnaTfCfUmU mGmGmAfAmUfGmUmCmCmUm C*mA*mG | AUACUGTCUU GGAAUGUCCU CAG | 123 |
| NCL-74 | NCL-74s | Sense | mG*fG*mAmCrA*mUrU*mCfCfA mAmGrA*mCrA*mGmUfAmU*T* T | GGACAUUCCA AGACAGUAUT T | 120 |
| | NCL-74a | Antisense | (5p)mA*T*mAfCmUfGT*mCfUm UmGfGmAfAmUfGmUmCC*mU* mU | ATACUGTCUU GGAAUGUCCU U | 121 |
| NCL-23nt | NCL-23nt-s | Sense | mG*fA*mGmGrA*mCrA*mUfUfC mCmArA*mGrA*mCmAfGmUmA fU*T*T | GAGGACAUUC CAAGACAGUA UTT | 128 |
| | NCL-23nt-a | Antisense | (5p)mA*T*mAfCmUfGT*mCfUm UmGfGmAfAmUfGmUmCfCmUC *mA*mG | ATACUGTCUU GGAAUGUCCU CAG | 129 |

| | | | | | |
|---|---|---|---|---|---|
| * the oligomeric compounds of this Table of SEQ ID NOs: 118, 119, 122, 123, 126-129 do not have the motifs claimed | | | | | |

### In Vitro Assays - Comparison of LMNA-si110 and LMNA-si74 to Benchmark LMNA-si111

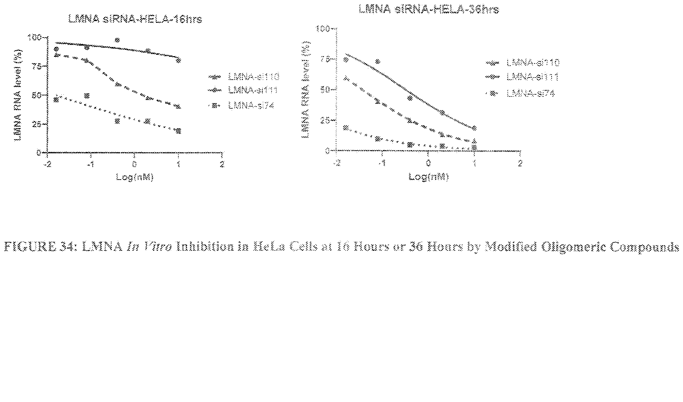

LMNA siRNA was transfected at 0-10 nM into HeLa cells with RNAiMAX (Invitrogen, Waltham, MA) and the cells further cultured for 16 and 36 hr. siRNA activity was determined by qRT-PCR using the primer probe set in Table 2 and the IC50 values of the siRNAs targeting LMNA were calculated (Table 68) and the percent inhibition of LMNA RNA levels graphed (Figure 34).

**Table 68: LMNA siRNA Inhibition in HeLa Cells**

| | HeLa Cell (16 hrs) | | HeLa Cell (36 hrs) | |
|---|---|---|---|---|
| siRNA | IC50 (nM) | Fold | IC50 (nM) | Fold |
| LMNA-si110 | 1.61 | 22 | 0.0367 | 9 |
| LMNA-si111 | 35.6 | 1 | 0.331 | 1 |
| LMNA-si74 | 0.0447 | 796 | 0.0004 | 824 |

### In Vitro Assays - Comparison of NCL-74 and NCL-23nt to NCL-ALN

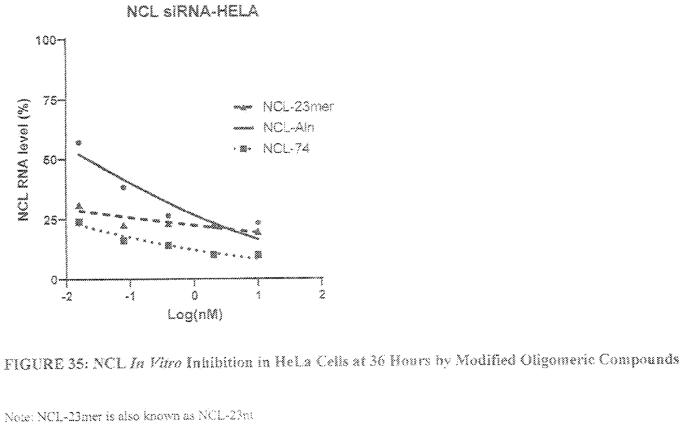

NCL siRNA was transfected at 0-10 nM into HeLa cells with RNAiMAX (Invitrogen, Waltham, MA) and the cells further cultured for 36 hr. siRNA activity was determined by qRT-PCR using the primer probe sets as listed in Table 30. The results are shown in Figure 35. Since NCL mRNA reduction was too great to determine the IC50, the percentage mRNA levels at 0.08 nM siRNA concentration are listed in Table 69.

**Table 69: NCL siRNA Inhibition in HeLa Cells at 36 Hrs**

| siRNA | % NCL mRNA at 0.08 nM siRNA |
|---|---|
| NCL-23nt (23 nt) | 23% |
| NCL-ALN (Benchmark) | 38% |
| NCL74 (21 nt) | 16% |

The results showed that the 23nt siRNAs with newly designed chemistry also showed better activity compared with benchmark siRNA of the same length, although the 21nt siRNA appear to have more activity compared with 23mer siRNAs.

### In Vitro Assay - Comparison of 21nt to 23nt siRNAs Targeting AGT

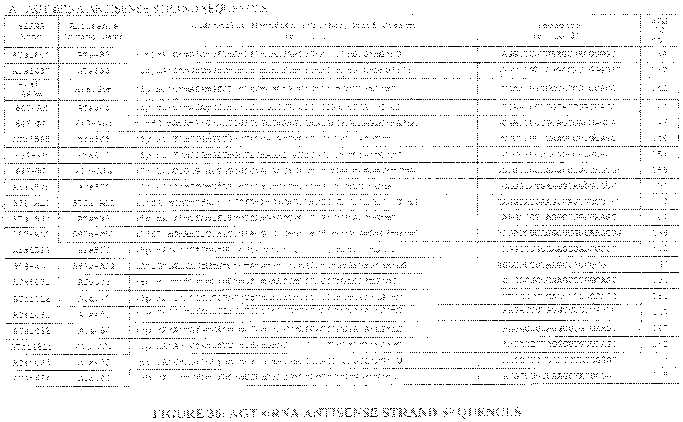

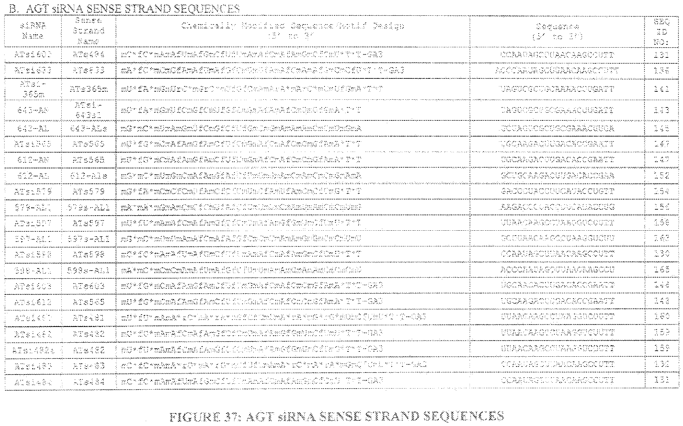

To further determine if the better activity of 21-mer siRNA over 23-mer siRNA also applies to siRNA sequences targeting other mRNA transcripts, 21-mer and 23-mer siRNAs targeting human angiotensinogen (AGT) mRNA were designed as shown in Table 70 and Figures 36-37). A GalNAc (as described in Sharma et al., 2018, Bioconjugate Chem, 29:2478-2488 and known herein as GA3 or GalNAc3) is conjugated to the sense strand. The ARNATAR designed siRNAs have a 5'-phosphate on the antisense strand.

**Table 70: Oligomeric Compounds with Different Length Targeting AGT mRNA**

| siRNA | Strand Name | Sense or Antisense | Sequence + Chemistry* (5' to 3') | Sequence | SEQ ID NO: |
|---|---|---|---|---|---|
| AT-si600 | ATs484 | Sense | mC*fC*mAmAfUmAfGmCfUf UmAmAfCmAfAmGmCfCmU* T*T-GA3 | CCAAUAGCUUAA CAAGCCUTT | 131 |
| | ATa483 | Antisense | (5p)mA*G*mGfCmUfUmGmUf UmAmAfGmCfUmAfUmUmGf G*mG*mU | AGGCUUGUUAAG CUAUUGGGU | 134 |
| AT-si633 | ATs633 | Sense | mA*fC*mCmCfAmAfUmAfGf CmUmUfAmAfCmAmAfGmC mCfU*T*T-GA3 | ACCCAAUAGCUU AACAAGCCUTT | 136 |
| | ATa633 | Antisense | (5p)mA*G*mGfCmUfUmGmUf UmAmAfGmCfUmAfUmUmGf GmGmU*T*T | AGGCUUGUUAAG CUAUUGGGUTT | 137 |

| | | | | | |
|---|---|---|---|---|---|
| * the oligomeric compounds of this Table of SEQ ID NOs: 136 and 137 do not have the motifs claimed | | | | | |

### In Vitro Assays

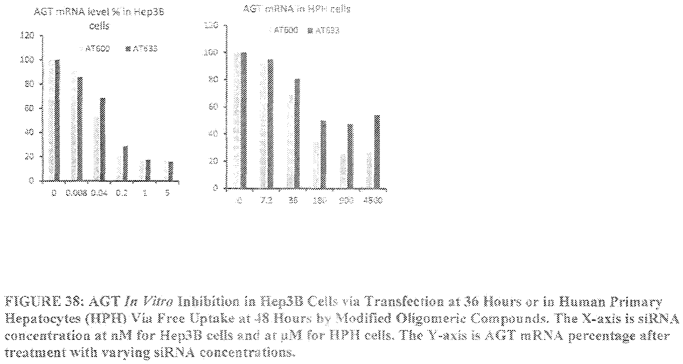

AGT siRNA was transfected at 0-10 nM into Hep3B cells with RNAiMAX (Invitrogen, Waltham, MA) and the cells further cultured for 36 hr. In addition, the siRNAs were incubated with human primary hepatocytes without transfection for 48 hr. siRNA activity was determined by qRT-PCR using the primer probe sets as listed in Table 71. The IC50 of the siRNAs targeting AGT are shown in Table 72 and the percent inhibition of AGT mRNA levels graphed (Figure 38).

**Table 71: Sequences of Primer Probe Set for AGT**

| Name | Sequence | SEQ ID NO: |
|---|---|---|
| hsAGT-F | CTGATCCAGCCTCACTATGC | 138 |
| hsAGT-R | AGGTCATAAGATCCTTGCAGC | 139 |
| hsAGT-P | AGGGTCTCACTTTCCAGCAAAACTCC | 140 |

**Table 72: siRNA Activity Targeting AGT in Hep3B cells**

| | | Hep3B Cells (36 hr) | | Human primary hepatocytes | |
|---|---|---|---|---|---|
| siRNA | siRNA Length | IC50 (nM) | Fold | IC50 (µM) | Fold |
| ATsi600 | 21mer | 0.057 | 1.62 | 0.137 | 11.8 |
| ATsi633 | 23mer | 0.093 | 1 | 1.617 | 1 |

The results with this target also show that the 21nt siRNA has better activity than the 23nt siRNA.

### EXAMPLE 22: NEW CHEMICAL MODIFICATION MOTIFS SHOWED BETTER ACTIVITY THAN REFERENCE DESIGN FOR DIFFERENT siRNAs TARGETING AGT

The above results suggest that the newly optimized ARNATAR chemistry design increased the activity of siRNAs for different targets or sequences. To further determine if this observation is generalizable, siRNAs were designed to target 5 different regions of human AGT, a therapeutic target for the treatment of resistant hypertension. The siRNA sequences and chemistry are listed in Table 73. For comparison, the siRNAs were designed with ARNATAR motifs, or with a third party's structure and modification design (ESC or ESC+ as described in Hu et al., Therapeutic siRNA: State of the Art, Signal Transduction and Targeted Therapy, 2020, 5:101). The ARNATAR designed siRNAs have a 5'-phosphate on the antisense strand. The siRNAs were made without GalNAc conjugates in order to only compare the chemical modification motifs.

**Table 73: Oligomeric Compounds Targeting Different Regions of AGT mRNA.**

| siRNA | Strand Name | Sense or Antisense | Sequence + Chemistry* | Sequence | SEQ ID NO: |
|---|---|---|---|---|---|
| ATsi-365m | ATs365m | sense | mU*fA*mGmUrC*mGrC*mUfGfC mAmArA*mArC*mUmUfGmA*T* T | UAGUCGCUGCAA AACUUGATT | 141 |
| | ATa365m | Antisense | (5p)mU*C*mAfAmGfUT*mUfUm GmCfAmGfCmGfAmCmUA*mG* mC | UCAAGUTUUGCA GCGACUAGC | 142 |
| 643-AN | ATsi-643s1 | sense | mU*fA*mGmUfCmGfCmUfGfCm GmAfAmAfCmUmUfGmA*T*T | UAGUCGCUGCGA AACUUGATT | 143 |
| | ATa643 | Antisense | (5p)mU*C*mAfAmGfUmUmUfCm GmCfAmGfCmGfAmCmUfA*mG* mC | UCAAGUUUCGCA GCGACUAGC | 144 |
| 643-AL | 643-Als | sense | mG*mC*mUmAmGmUfCmGfCfUf GmCmGmAmAmAmCmUmUmGm A | GCUAGUCGCUGC GAAACUUGA | 145 |
| | 643-Ala | Antisense | mU*fC*mAmAmGfUgnaTfUfCmG mCmAmGfCmGfAmCmUmAmGm C*mA*mC | UCAAGUUUCGCA GCGACUAGCAC | 146 |
| ATsi565 | ATs565 | sense | mU*fG*mCmAfAmGfAmCfUfUm GmAfCmAfCmCmGfAmA*T*T | UGCAAGACUUGA CACCGAATT | 147 |
| | ATa565 | Antisense | (5p)mU*T*mCfGmGfUG*mUfCm AmAfGmUfCmUfUmGmCA*mG* mC | UTCGGUGUCAAG UCUUGCAGC | 149 |
| 612-AN | ATs565 | sense | mU*fG*mCmAfAmGfAmCfUfUm GmAfCmAfCmCmGfAmA*T*T | UGCAAGACUUGA CACCGAATT | 147 |
| | ATa612 | Antisense | (5p)mU*T*mCfGmGfUmGmUfCm AmAfGmUfCmUfUmGmCfA*mG* mC | UTCGGUGUCAAG UCUUGCAGC | 151 |
| 612-AL | 612-Als | sense | mG*mC*mUmGmCmAfAmGfAfCf UmUmGmAmCmAmCmCmGmAm A | GCUGCAAGACUU GACACCGAA | 152 |
| | 612-Ala⁺⁺ | Antisense | mU*fU*mCmGmGgnaTmGfUfCm AmAmGmUfCmUfUmGmCmAmG mC*mG*mA | UUCGGUGUCAAG UCUUGCAGCGA | 153 |
| ATsi579 | ATs579 | sense | mG*fA*mCmCfCmUfAmCfCfUm UmCfAmUfAmCmCfUmG*T*T | GACCCUACCUUC AUACCUGTT | 154 |
| | ATa579 | Antisense | (5p)mC*A*mGfGmUfAT*mGfAm AmGfGmUfAmGfGmGmUC*mU* mU | CAGGUATGAAGG UAGGGUCUU | 155 |
| 579-AL1 | 579s-AL1 | sense | mA*mA*mGmAmCmCfCmUfAfCf CmUmUmCmAmUmAmCmCmUm G | AAGACCCUACCU UCAUACCUG | 156 |
| | 579a-AL | Antisense | mC*fA*mGmGmUfAgnaTfGfAmA mGmGmUfAmGfGmGmUmCmUm U*mU*mG | CAGGUAUGAAGG UAGGGUCUUUG | 157 |
| ATsi597 | ATs597 | sense | mU*fU*mAmAfCmAfAmGfCfCm UmAfAmGfGmUmCfUmU*T*T | UUAACAAGCCUA AGGUCUUTT | 158 |
| | ATa597 | Antisense | (5p)mA*A*mGfAmCfCT*mUfAm GmGfCmUfUmGfUmUmAA*mG* mC | AAGACCTUAGGC UUGUUAAGC | 161 |
| 597-AL1 | 597s-AL1 | sense | mG*mC*mUmUmAmAfCmAfAfGf CmCmUmAmAmGmGmUmCmUm U | GCUUAACAAGCC UAAGGUCUU | 163 |
| | 597a-AL | Antisense | mA*fA*mGmAmCfCgnaTfUfAmG mGmCmUfUmGfUmUmAmAmGm C*mU*mG | AAGACCUUAGGC UUGUUAAGCUG | 164 |
| ATsi598 | ATs598 | sense | mC*fC*mAmAfUmAfGmCfUfUm AmAfCmAfAmGmCfCmU*T*T | CCAAUAGCUUAA CAAGCCUTT | 130 |
| | ATa598 | Antisense | (5p)mA*G*mGfCmUfUG*mUfUm AmAfGmCfUmAfUmUmGG*mG* mU | AGGCUUGUUAAG CUAUUGGU | 133 |
| 598-AL1 | 598s-AL1 | sense | mA*mC*mCmCmAmAfUmAfGfCf UmUmAmAmCmAmAmGmCmCm U | ACCCAAUAGCUU AACAAGCCU | 165 |
| | 598a-AL⁺⁺⁺ | Antisense | mA*fG*mGmCmUfUmGfUfUmAm AmGmCfUmAfUmUmGmGmGmU *mA*mG | AGGCUUGUUAAG CUAUUGGUAG | 166 |

| | | | | | |
|---|---|---|---|---|---|
| ⁺⁺Since gnaG was not available, 612-Ala differs from the normal ESC+ chemistry in placement of the gna modification, however, gna at position 6 has also been widely used in drug discovery (see PCT/US2019/032150). ⁺⁺⁺Since gnaG is not available, 598a-AL does not contain gna at position 7, similar to the ESC chemistry. * the oligomeric compounds of this Table of SEQ ID NOs: 145, 146, 152, 153, 156, 157, 163, 164, 165 and 166 do not have the motif claimed. | | | | | |

### In Vitro Assays - Comparison of ARNATAR Motifs to Third Party Motifs

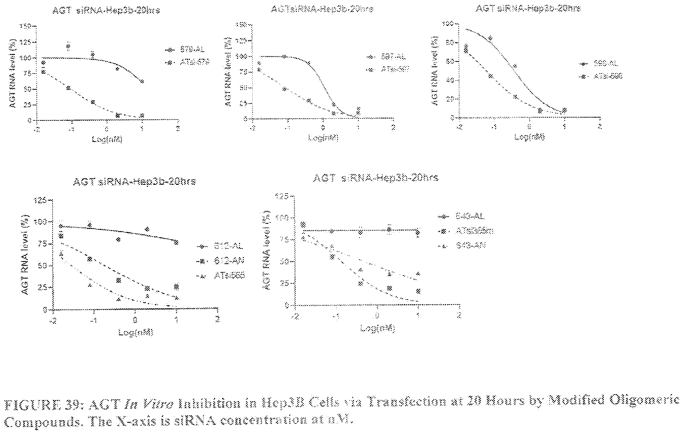

AGT siRNAs were transfected at 0-10 nM into Hep3B cells with RNAiMAX (Invitrogen, Waltham, MA) and the cells further cultured for 24 hr. siRNA activity was determined by qRT-PCR using the primer probe sets as listed in Table 71. The IC50 of the siRNAs targeting AGT are shown in Table 74 and the percent inhibition of AGT mRNA levels graphed (Figure 39).

**Table 74: siRNA Activity Targeting AGT in Hep3B cells**

| siRNAs | IC50 (nM) |
|---|---|
| 579AL | 15.06 |
| ATsi-579 | 0.0947 |
| 597AL | 1.105 |
| ATsi-597 | 0.087 |
| 598-AL | 0.3776 |
| ATsi-598 | 0.0604 |
| 612-AL | 1233 |
| 612-AN | 0.1821 |
| ATsi-565 | 0.0284 |
| 643-AL | N/A |
| ATsi-365m | 0.1346 |
| 643-AN | 0.4656 |

The results indicate that for all these different sequences, the optimized ARNATAR designs are more potent than the third party design.

### EXAMPLE 23: COMPARISON OF siRNA WITH DIFFERENT CHEMICAL MOTIFS TARGETING THE SAME AGT REGIONS

As shown in prior experiments above, altering chemical modification can affect siRNA activity. We thus evaluated the activities of altering chemical modifications of siRNAs targeting the same sequences of AGT mRNA (see Table 75 and Figures 36-37).

**Table 75. Oligomeric Compounds Targeting Same Region of AGT with Different Chemical Motif.**

| siRNA | Strand Name | Sense or Antisense | Sequence + Chemistry | Sequence | SEQ ID NO: |
|---|---|---|---|---|---|
| ATsi603 | ATs603 | sense | mU*fG*mCmAfAmGfAmCf UfUmGmAfCmAfCmCmGf AmA*T*T-GA3 | UGCAAGACUU GACACCGAAT T | 148 |
| | ATa603 | Antisense | (5p)mU*T*mCfGmGfUG*m UfCmAmAfGmUfCmUfUm GmCA*mG*mC | UTCGGUGUCA AGUCUUGCAG C | 150 |
| ATsi612 | ATs565 | sense | mU*fG*mCmAfAmGfAmCf UfUmGmAfCmAfCmCmGf AmA*T*T-GA3 | UGCAAGACUU GACACCGAAT T | 148 |
| | ATa612 | Antisense | (5p)mU*T*mCfGmGfUmGm UfCmAmAfGmUfCmUfUm GmCfA*mG*mC | UTCGGUGUCA AGUCUUGCAG C | 151 |
| ATsi481 | ATs481 | sense | mU*fU*mAmA*rC*mA*rA* mGfCfCmUmA*rA*mG*rG* mUmCfUmU*T*T-GA3 | UUAACAAGCC UAAGGUCUUT T | 160 |
| | ATa481 | Antisense | (5p)mA*A*mGfAmCfCmUm UfAmGmGfCmUfUmGfUm UmAfA*mG*mC | AAGACCUUAG GCUUGUUAAG C | 167 |
| ATsi482 | Ats482 | sense | mU*fU*mAmAfCmAfAmGf CfCmUmAfAmGfGmUmCf UmU*T*T-GA3 | UUAACAAGCC UAAGGUCUUT T | 159 |
| | ATa482 | Antisense | (5p)mA*A*mGfAmCfCmUm UfAmGmGfCmUfUmGfUm UmAfA*mG*mC | AAGACCUUAG GCUUGUUAAG C | 167 |
| ATsi482a | ATs482 | sense | mU*fU*mAmAfCmAfAmGf CfCmUmAfAmGfGmUmCf UmU*T*T-GA3 | UUAACAAGCC UAAGGUCUUT T | 159 |
| | ATa-482a | Antisense | (5p)mA*A*mGfAmCfCT*m UfAmGmGfCmUfUmGfUm UmAfA*mG*mC | AAGACCTUAG GCUUGUUAAG C | 162 |
| ATsi483 | ATs483 | sense | mC*fC*mAmA*rU*mA*rG* mCfUfUmAmA*rC*mA*rA* mGmCfCmU*T*T-GA3 | CCAAUAGCUU AACAAGCCUT T | 132 |
| | ATa483 | Antisense | (5p)mA*G*mGfCmUfUmG mUfUmAmAfGmCfUmAfU mUmGfG*mG*mU | AGGCUUGUUA AGCUAUUGGG U | 134 |
| ATsi484 | ATs484 | sense | mC*fC*mAmAfUmAfGmCf UfUmAmAfCmAfAmGmCf CmU*T*T-GA3 | CCAAUAGCUU AACAAGCCUT T | 131 |
| | ATa484 | Antisense | (5p)mA*G*mGfCmUfUG*m UfUmAmAfGmCfUmAfUm UmGG*mG*mU | AGGCUUGUUA AGCUAUUGGG U | 135 |

### In Vitro Assays - Comparison of siRNAs with Different Chemical Motifs Targeting the Same AGT Sequences

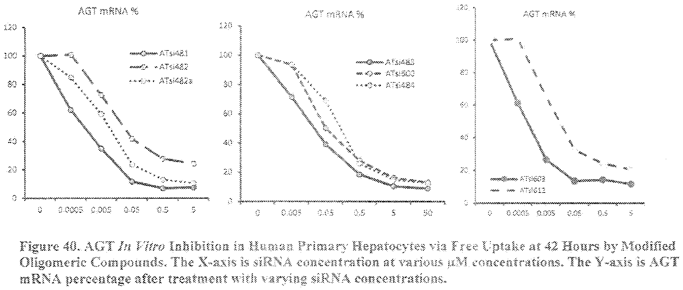

The siRNAs were incubated at 0-5 µM (ATsi481, ATsi482, ATsi482a, ATsi483, ATsi484) or 0-50 µM (ATsi603, ATsi612) final concentration by free uptake with human primary hepatocytes. Cells were incubated for 42 hr, and total RNA was prepared. siRNA activity was evaluated using qRT-PCR with AGT primer probe sets (Table 71). The IC50 of the siRNAs targeting AGT mRNA are shown in Table 76 and the percent inhibition of AGT mRNA levels graphed (Figure 40).

**Table 76: siRNA Activity Targeting AGT in Human Primary Hepatocytes**

| siRNA | IC50 (µM) |
|---|---|
| ATsi481 | 0.0015 |
| ATsi482 | 0.0353 |
| ATsi482a | 0.0085 |
| ATsi603 | 0.0009 |
| ATsi612 | 0.0269 |
| ATsi483 | 0.026 |
| ATsi600 | 0.096 |
| ATsi484 | 0.154 |

### SEQUENCE LISTING

**Table 77: Sequence Listing**

| SEQ ID NO: | SEQUENCE | DESCRIPTION |
|---|---|---|
| 1 | GCGTCACCAAAAAGCGCAA | LMNA DNA Target Seq, sense strand |
| 2 | GCGUCACCAAAAAGCGCAA | LMNA RNA Target Seq |
| 3 | CGGGTGGATGCTGAGAAC | Human LMNA Primer hsLMNA-S |
| 4 | TGCTTCCCATTGTCAATCTCC | Human LMNA Primer hsLMNA-A |
| 5 | AGTGAGGAGCTGCGTGAGACCAA | Human LMNA Probe hsLMNA-P |
| 6 | GGACCAGGTGGAACAGTATAAG | Mouse LMNA Primer msLMNA-S |
| 7 | TCAATGCGGATTCGAGACTG | Mouse LMNA Primer msLMNA-A |
| 8 | CAGCTTGGCGGAGTATGTCTTTTCTAGC | Mouse LMNA Probe msLMNA-P |
| 9 | AGGGTTACATGAAGCACGC | Human ApoC3 Primer ApoC3-F |
| 10 | AGAGAACTTGTCCTTAACGGTG | Human ApoC3 Primer ApoC3-R |
| 11 | AGGGAACTGAAGCCATCGGTCAC | Human ApoC3 Probe ApoC3-P |
| 12 | GCTTGGCTTCTTCTGGACTCA | Human NCL Primer hsNCL-F |
| 13 | TCGCGAGCTTCACCATGA | Human NCL Primer hsNCL-R |
| 14 | CGCCACTTGTCCGCTTCACACTCC | Human NCL Probe hsNCL-P |
| 168 | GCGUCACCAAAAAGCGCAATT | LMNA sense strands ATXL019, ATXL021, ATXL026, ATXL023, ATXL030, ATXL031, ATXL154, ATXL155, ATXL171, ATXL204, ATXL206, ATXL232 |
| 169 | UUGCGCUUUUUGGUGACGCTT | LMNA antisense strands ATXL018, ATXL020 |
| 170 | GCGUCACCAAAAAGCGCAAUU | LMNA sense strand ATXL022, ATXL024, ATXL025, ATXL027-ATXL029, ATXL032, ATXL048-ATXL053, ATXL079-ATXL085 |
| 171 | UUGCGCUUUUUGGUGACGCUU | LMNA antisense strand ATXL041, ATXL188 |
| 172 | UTGCGCUUUUUGGUGACGCUU | LMNA antisense strand ATXL042, ATXL187 |
| 173 | UTGCGCTUUUUGGUGACGCUU | LMNA antisense strand ATXL043, ATXL046, ATXL047, ATXL078, ATXL081, ATXL152, ATXL152, ATXL168-ATXL170, ATXL186 |
| 174 | UUGCGCTUUUUGGUGACGCUU | LMNA antisense strand ATXL044, ATXL045, ATXL077 |
| 175 | CUCUGAGUUCUGGGAUUUGTT | ApoC3 sense strand ATXL059, ATXL116, ApoC3-74s |
| 176 | CAAAUCCCAGAACUCAGAGTT | ApoC3 antisense strand ATXL058 |
| 177 | CUCUGAGUUCUGGGAUUUG | ApoC3 sense strand ATXL060, ATXL061 |
| 178 | CUCUGAGUUCUGGGAUUUGUU | ApoC3 sense strand ATXL068, ATXL069 |
| 179 | CAAAUCCCAGAACUCAGAGUU | ApoC3 antisense strand ATXL067, ATXL072, ApoC3-74a |
| 180 | GGAUAGUUACUGACCGGGATT | NCL sense strand ATXL125 |
| 181 | UCCCGGUCAGUAACUAUCCTT | NCL antisense strand ATXL124 |
| 182 | GGAUAGUUACUGACCGGGAUU | NCL sense strand ATXL128, ATXL129 |
| 183 | UCCCGGTCAGUAACUAUCCUU | NCL antisense strand ATXL126, ATXL127 |
| 184 | CAAAUCCCCAGAACUCAGAGUU | ApoC3 antisense strand ATXL066 |
| 185 | UTGCGCTUUUUGGTGACGCUU | LMNA antisense strand ATXL074 and ATXL075 |
| 186 | UTGCGCTUTUUGGTGACGCUU | LMNA antisense strand ATXL076 |
| 98 | GGAUAGUUACUGACCGGGA | NCL Target Seq |
| 99 | AAACACTGTGAGGATGGGATC | hsNFkB formard primer hsNFkB-F |
| 100 | TCTGTCATTCGTGCTTCCAG | hsNFkB reverse primer hsNFkB-R |
| 101 | TGTCACATGAAGTATACCCAGGTTTGCG | hsNFkB probe hsNFkB-P |
| 102 | GTGTCAGAGCCCTTGTAACTG | msNfkB Forward Primer NfkB-F |
| 103 | ACATTTGCCCAGTTCCGTAG | msNfkB Reverse Primer NfkB-R |
| 104 | TCGTCTGCCATGGTGAAGATGCG | msNfkB Probe NfkB-P |
| 105 | AAACCGCTATGAAGTTCCTCTC | msIL-6 Forward Primer IL6-F |
| 106 | GTGGTATCCTCTGTGAAGTCTC | msIL-6 Reverse Primer IL6-R |
| 107 | TTGTCACCAGCATCAGTCCCAAGAA | msIL-6 Probe IL6-P |
| 108 | AGACCCTCACACTCAGATCA | msTNF Forward Primer TNF-F |
| 109 | TGTCTTTGAGATCCATGCCG | msTNF Reverse Primer TNF-R |
| 110 | CCTGTAGCCCACGTCGTAGCAAA | msTNF Probe TNF-P |
| 187 | GACUUUCAUCCUGGAAAUAUA | HaoAl-S1 sense strand |
| 188 | UAUAUUUCCAGGAUGAAAGUCCA | HaoAl-A1 antisense strand |
| 189 | GACUUUCAUCCUGGAAAUATA | Hao-ART-S97 sense strand |
| 190 | UAUAUUUCCAGGAUGAAAGUC | Hao-ART-A2 antisense strand |
| 191 | UAUAUUTCCAGGAUGAAAGUCCA | Hao-ART-A74-1 antisense strand |
| 192 | UAUAUUTCCAGGAUGAAAGUC | Hao-ART-A74-2 antisense strand |
| 193 | CAGCGUCACCAAAAAGCGCAA | LMNA sense strand ATXL239 |
| 194 | UUGCGCTUUUUGGUGACGCUGCC | LMNA antisense strand ATXL238 |
| 195 | GGACAUUCCAAGACAGUAUTT | NCL sense strand NCL-74s |
| 196 | ATACUGTCUUGGAAUGUCCUU | NCL antisense strand NCL-74a |
| 197 | GAGGACAUUCCAAGACAGUAU | NCL sense strand NCL-Als1 |
| 198 | AUACUGTCUUGGAAUGUCCUCAG | NCL antisense strand NCL-ALa |
| 199 | UUCUCUGAGUUCUGGGAUUUG | ApoC3 sense strand Apoc3-ALs |
| 200 | CAAAUCCCAGAACUCAGAGAACU | ApoC3 antisense strand Apoc3-ALa |
| 201 | CAGCGUCACCAAAAAGCGCAATT | LMNA sense strand ATXL237 |
| 202 | UTGCGCTUUUUGGUGACGCUGCC | LMNA antisense strand ATXL236 |
| 203 | GAGGACAUUCCAAGACAGUAUTT | NCL sense strand NCL-23nt-s |
| 204 | ATACUGTCUUGGAAUGUCCUCAG | NCL sense strand NCL-23nt-a |
| 205 | CCAAUAGCUUAACAAGCCUTT | AGT sense strand ATs600, ATs598, ATs483, ATs484 |
| 206 | AGGCUUGUUAAGCUAUUGGGU | AGT antisense strand ATa600, ATa598, ATa483, ATa484 |
| 207 | ACCCAAUAGCUUAACAAGCCUTT | AGT sense strand ATs633 |
| 208 | AGGCUUGUUAAGCUAUUGGGUTT | AGT antisense strand ATa633 |
| 138 | CTGATCCAGCCTCACTATGC | hsAGT forward primer hsAGT-F |
| 139 | AGGTCATAAGATCCTTGCAGC | hsAGT reverse primer hsAGT-R |
| 140 | AGGGTCTCACTTTCCAGCAAAACTCC | hsAGT probe hsAGT-P |
| 209 | UAGUCGCUGCAAAACUUGATT | AGT sense strand, ATs365m |
| 210 | UCAAGUTUUGCAGCGACUAGC | AGT antisense strand ATa365m |
| 211 | UAGUCGCUGCGAAACUUGATT | AGT sense strand ATsi-643s1 |
| 212 | UCAAGUUUCGCAGCGACUAGC | AGT antisense strand ATa643 |
| 213 | GCUAGUCGCUGCGAAACUUGA | AGT sense strand 643-Als |
| 214 | UCAAGUUUCGCAGCGACUAGCAC | AGT antisense strand 643-ALa |
| 215 | UGCAAGACUUGACACCGAATT | AGT sense strand ATs565, ATs612, ATs603 |
| 216 | UTCGGUGUCAAGUCUUGCAGC | AGT antisense strands ATa565, ATa612, ATa603 |
| 217 | GCUGCAAGACUUGACACCGAA | AGT sense strand 612-Als |
| 218 | UUCGGUGUCAAGUCUUGCAGCGA | AGT antisense strand 612-Ala |
| 219 | GACCCUACCUUCAUACCUGTT | AGT sense strand, ATs579 |
| 220 | CAGGUATGAAGGUAGGGUCUU | AGT antisense strand ATa579 |
| 221 | AAGACCCUACCUUCAUACCUG | AGT sense strand 579s-AL1 |
| 222 | CAGGUAUGAAGGUAGGGUCUUUG | AGT antisense strand 579a-AL |
| 223 | UUAACAAGCCUAAGGUCUUTT | AGT sense strand ATs597 |
| 224 | AAGACCTUAGGCUUGUUAAGC | AGT antisense strand ATa597, ATa482a |
| 225 | GCUUAACAAGCCUAAGGUCUU | AGT sense strand 597s-AL1 |
| 226 | AAGACCUUAGGCUUGUUAAGCUG | AGT antisense strand 597a-AL |
| 227 | ACCCAAUAGCUUAACAAGCCU | AGT sense strand 598s-AL1 |
| 228 | AGGCUUGUUAAGCUAUUGGGUAG | AGT antisense strand 598a-AL |
| 229 | AAGACCUUAGGCUUGUUAAGC | AGT antisense strand ATa481, ATa482 |

## Claims

1. An oligomeric compound that inhibits the expression of a target nucleic acid, comprising a sense strand having 21 linked nucleotides, wherein the sense strand sequence is represented by Formula (X):
5' MFMMNMNMFFNMNMNMMNMDD 3'; and
an antisense strand having 21 linked nucleotides, wherein the antisense strand sequence is represented by Formula (VIII):
5' L-MNMNMFNMFMMNMFMFMMNMM 3'; and
wherein
D is a deoxyribonucleoside,
M is a 2'-OMe modified nucleoside,
N is a modified or unmodified nucleoside,
F is a 2'-F modified nucleoside, and
L is a 5' phosphate, 5' vinyl phosphonate or 5' OH, and
wherein no single modification type modifies more than two consecutive nucleotides.

2. The oligomeric compound of claim 1
wherein a duplex is formed by the sense and antisense strands,
wherein the duplex region is 19 nucleotide pairs in length,
wherein each strand has a 2 nucleotide overhang at the 3' end.

3. An oligomeric compound of claim 1 or 2, wherein the sense strand is represented by Formula (XI):
5' MFMMNMNMFFMMNMNMMFMDD 3',
wherein
D is a deoxyribonucleoside,
M is a 2'-OMe modified nucleoside,
N is a modified or unmodified nucleoside, and
F is a 2'-F modified nucleoside, and
wherein no single modification type modifies more than two consecutive nucleotides.

4. An oligomeric compound of any preceding claim, wherein the antisense strand is represented by Formula (XII):
5' L-MDMFMFNMFMMFMFMFMMNMM 3',
wherein
D is a deoxyribonucleoside,
M is a 2'-OMe modified nucleoside,
N is a modified or unmodified nucleoside,
F is a 2'-F modified nucleoside, and
L is a 5' phosphate, 5' vinyl phosphonate or 5' OH, and
wherein no single modification type modifies more than two consecutive nucleotides.

5. An oligomeric compound of any preceding claim, comprising:
a) the sense strand represented by Formula (XI):
5' MFMMNMNMFFMMNMNMMFMDD 3', and
b) the antisense strand represented by Formula (XII):
5' L-MDMFMFNMFMMFMFMFMMNMM 3', and
wherein a duplex is formed by the sense and antisense strands,
wherein
D is a deoxyribonucleoside,
M is a 2'-OMe modified nucleoside,
N is a modified or unmodified nucleoside,
F is a 2'-F modified nucleoside, and
L is a 5' phosphate, 5' vinyl phosphonate or 5' OH,
wherein the duplex region is 19 nucleotide pairs in length,
wherein each strand has a 2 nucleotide overhang at the 3' end, and
wherein no single modification type modifies more than two consecutive nucleotides.

6. The oligomeric compound of any preceding claim, wherein the N can be a ribonucleoside (R), deoxyribonucleoside (D), 2'-OMe, 2'-MOE, 2'-F, unlocked nucleic acid (UNA) or locked nucleic acid (LNA).

7. The oligomeric compound of any preceding claim, wherein the oligomeric compound comprises at least one phosphorothioate internucleotide (PS) linkage.

8. The oligomeric compound of any preceding claim, wherein the phosphorothioate internucleotide (PS) linkage is adjacent to a deoxyribonucleoside (D) or ribonucleoside (R).

9. The oligomeric compound of any preceding claim, wherein the oligomeric compound is a siRNA.

10. The oligomeric compound of any preceding claim, further comprising a conjugate, wherein the conjugate is preferably N-Acetylgalactosamine (GalNAc).

11. The oligomeric compound of any preceding claim, wherein the compound inhibits expression of a target nucleic acid by at least about 70%, 75%, 80%, 85%, 90%, 95%, 98% or 99%.

12. A pharmaceutical composition comprising the oligomeric compound of any preceding claim, alone or in combination with a pharmaceutically acceptable carrier or excipient.

13. A process for making an oligomeric compound of any of the preceding claims comprising the steps of:
a. synthesizing a sense strand oligonucleotide on a solid support using phosphoramidite chemistry
b. synthesizing an antisense oligonucleotide on a solid support using phosphoramidite chemistry; and
c. annealing the two oligonucleotides so synthesized
thereby making the oligomeric compound.

## Patentansprüche

1. Oligomere Verbindung, die die Expression einer Zielnukleinsäure hemmt, umfassend einen Sense-Strang mit 21 verbundenen Nukleotiden, wobei die Sense-Strangsequenz durch die Formel (X) dargestellt wird:
5' MFMMNMNMFFNMNMNMMNMDD 3'; und
einen Antisense-Strang mit 21 verbundenen Nukleotiden, wobei die Antisense-Strangsequenz durch die Formel (VIII) dargestellt wird:
5' L-MNMNMFNMFMMNMFMFMMNMM 3'; und
wobei
D ein Desoxyribonukleosid ist,
M ein 2'-OMe-modifiziertes Nukleosid ist,
N ein modifiziertes oder unmodifiziertes Nukleosid ist,
F ein 2'-F-modifiziertes Nukleosid ist und
L ein 5'-Phosphat, 5'-Vinylphosphonat oder 5'-OH ist, und
wobei kein einzelner Modifikationstyp mehr als zwei aufeinanderfolgende Nukleotide modifiziert.

2. Die oligomere Verbindung nach Anspruch 1,
wobei ein Duplex durch den Sense- und den Antisense-Strang gebildet wird,
wobei der Doppelstrangbereich eine Länge von 19 Nukleotidpaaren aufweist,
wobei jeder Strang am 3'-Ende einen Überhang von 2 Nukleotiden aufweist.

3. Oligomere Verbindung nach Anspruch 1 oder 2, wobei der Sense-Strang durch die Formel (XI) dargestellt wird:
5' MFMMNMNMFFMMNMNMMFMDD 3',
wobei
D ein Desoxyribonukleosid ist,
M ein 2'-OMe-modifiziertes Nukleosid ist,
N ein modifiziertes oder unmodifiziertes Nukleosid ist und
F ein 2'-F-modifiziertes Nukleosid ist, und
wobei kein einzelner Modifikationstyp mehr als zwei aufeinanderfolgende Nukleotide modifiziert.

4. Oligomere Verbindung nach einem der vorstehenden Ansprüche, wobei der Antisense-Strang durch die Formel (XII) dargestellt ist:
5' L-MDMFMFNMFMMFMFMFMMNMM 3',
wobei
D ein Desoxyribonukleosid ist,
M ein 2'-OMe-modifiziertes Nukleosid ist,
N ein modifiziertes oder unmodifiziertes Nukleosid ist,
F ein 2'-F-modifiziertes Nukleosid ist und
L ein 5'-Phosphat, 5'-Vinylphosphonat oder 5'-OH ist, und
wobei kein einzelner Modifikationstyp mehr als zwei aufeinanderfolgende Nukleotide modifiziert.

5. Oligomere Verbindung nach einem der vorstehenden Ansprüche, umfassend:
a) den durch die Formel (XI) dargestellten Sense-Strang:
5' MFMMNMNMFFMMNMNMMFMDD 3', und
b) den Antisense-Strang, dargestellt durch die Formel (XII):
5' L-MDMFMFNMFMMFMFMFMMNMM 3', und
wobei durch den Sense- und den Antisense-Strang ein Doppelstrang gebildet wird,
wobei
D ein Desoxyribonukleosid ist,
M ein 2'-OMe-modifiziertes Nukleosid ist,
N ein modifiziertes oder unmodifiziertes Nukleosid ist,
F ein 2'-F-modifiziertes Nukleosid ist und
L ein 5'-Phosphat, 5'-Vinylphosphonat oder 5'-OH ist,
wobei der Duplexbereich eine Länge von 19 Nukleotidpaaren aufweist,
wobei jeder Strang einen Überhang von 2 Nukleotiden am 3'-Ende aufweist und
wobei kein einzelner Modifikationstyp mehr als zwei aufeinanderfolgende Nukleotide modifiziert.

6. Oligomere Verbindung nach einem der vorstehenden Ansprüche, wobei das N ein Ribonukleosid (R), Desoxyribonukleosid (D), 2'-OMe, 2'-MOE, 2'-F, eine ungesperrte ("unlocked") Nukleinsäure (UNA) oder gesperrte ("locked") Nukleinsäure (LNA) sein kann.

7. Oligomere Verbindung nach einem der vorstehenden Ansprüche, wobei die oligomere Verbindung mindestens eine Phosphorothioat- Internukleotid (PS)-Verbindung umfasst.

8. Oligomere Verbindung nach einem der vorstehenden Ansprüche, wobei die Phosphorothioat-Internukleotid (PS)-Verknüpfung benachbart zu einem Desoxyribonukleosid (D) oder Ribonukleosid (R) ist.

9. Oligomere Verbindung nach einem der vorstehenden Ansprüche, wobei die oligomere Verbindung eine siRNA ist.

10. Oligomere Verbindung nach einem der vorstehenden Ansprüche, die ferner ein Konjugat umfasst, wobei das Konjugat vorzugsweise N-Acetylgalactosamin (GalNAc) ist.

11. Oligomere Verbindung nach einem der vorstehenden Ansprüche, wobei die Verbindung die Expression einer Zielnukleinsäure um mindestens etwa 70%, 75%, 80%, 85%, 90%, 95%, 98% oder 99% hemmt.

12. Pharmazeutische Zusammensetzung, die die oligomere Verbindung aus einem der vorstehenden Ansprüche allein oder in Kombination mit einem pharmazeutisch verträglichen Träger oder Hilfsstoff umfasst.

13. Verfahren zur Herstellung einer oligomeren Verbindung nach einem der vorstehenden Ansprüche, umfassend die folgenden Schritte:
a. Synthese eines Sense-Strang-Oligonukleotids auf einem festen Träger unter Verwendung von Phosphoramidit-Chemie
b. Synthese eines Antisense-Oligonukleotids auf einem festen Träger unter Verwendung von Phosphoramidit-Chemie und
c. Anlagerung der beiden so synthetisierten Oligonukleotide
wodurch die oligomere Verbindung hergestellt wird.

## Revendications

1. Composé oligomérique qui inhibe l'expression d'un acide nucléique cible, comprenant un brin sens comportant 21 nucléotides liés, dans lequel la séquence du brin sens est représentée par la formule (X) :
5' MFMMNMNMFFNMNMNMMNMDD 3'; et
un brin antisens comportant 21 nucléotides liés, dans lequel la séquence du brin antisens est représentée par la formule (VIII) :
5' L-MNMNMFNMFMMNMFMFMMNMM 3'; et
dans lequel
D est un désoxyribonucléoside,
M est un nucléoside modifié en 2'-OMe,
N est un nucléoside modifié ou non modifié,
F est un nucléoside modifié en 2'-F, et
L est un phosphate 5', un phosphonate de vinyle 5' ou un OH 5', et
dans lequel aucun type de modification ne modifie plus de deux nucléotides consécutifs.

2. Le composé oligomère selon la revendication 1,
dans lequel un duplex est formé par les brins sens et antisens,
dans lequel la région duplex a une longueur de 19 paires de nucléotides,
dans lequel chaque brin présente un excès de 2 nucléotides à l'extrémité 3'.

3. Composé oligomère selon la revendication 1 ou 2, dans lequel le brin sens est représenté par la formule (XI) :
5' MFMMNMNMFFMMNMNMMFMDD 3',
dans lequel
D est un désoxyribonucléoside,
M est un nucléoside modifié en 2'-OMe,
N est un nucléoside modifié ou non modifié, et
F est un nucléoside modifié en 2'-F, et
dans lequel aucun type de modification ne modifie plus de deux nucléotides consécutifs.

4. Composé oligomère selon l'une quelconque des revendications précédentes, dans lequel le brin antisens est représenté par la formule (XII) :
5' L-MDMFMFNMFMMFMFMFMMNMM 3',
dans lequel
D est un désoxyribonucléoside,
M est un nucléoside modifié en 2'-OMe,
N est un nucléoside modifié ou non modifié,
F est un nucléoside modifié en 2'-F, et
L est un phosphate 5', un phosphonate de vinyle 5' ou un OH 5', et
dans lequel aucun type de modification ne modifie plus de deux nucléotides consécutifs.

5. Composé oligomère selon l'une quelconque des revendications précédentes, comprenant :
a) le brin sens représenté par la formule (XI) :
5' MFMMNMNMFFMMNMNMMFMDD 3', et
b) le brin antisens représenté par la formule (XII) :
5' L-MDMFMFNMFMMFMFMFMMNMM 3', et
dans lequel un duplex est formé par les brins sens et antisens,
dans lequel
D est un désoxyribonucléoside,
M est un nucléoside modifié en 2'-OMe,
N est un nucléoside modifié ou non modifié,
F est un nucléoside modifié en 2'-F, et
L est un phosphate 5', un phosphonate de vinyle 5' ou un OH 5',
dans lequel la région duplex a une longueur de 19 paires de nucléotides,
dans lequel chaque brin présente un excès de 2 nucléotides à l'extrémité 3', et
dans lequel aucun type de modification ne modifie plus de deux nucléotides consécutifs.

6. Le composé oligomère de l'une quelconque des revendications précédentes, dans lequel le N peut être un ribonucléoside (R), un désoxyribonucléoside (D), 2'-OMe, 2'-MOE, 2'-F, un acide nucléique non verrouillé (« unlocked ») (UNA) ou un acide nucléique verrouillé (« locked ») (LNA).

7. Le composé oligomère de toute revendication précédente, dans lequel le composé oligomère comprend au moins une liaison internucléotidique phosphorothioate (PS).

8. Composé oligomère selon l'une quelconque des revendications précédentes, dans lequel la liaison phosphorothioate internucléotidique (PS) est adjacente à un désoxyribonucléoside (D) ou à un ribonucléoside (R).

9. Le composé oligomère de l'une quelconque des revendications précédentes, dans lequel le composé oligomère est un siRNA.

10. Composé oligomère selon l'une quelconque des revendications précédentes, comprenant en outre un conjugué, dans lequel le conjugué est de préférence la N-acétylgalactosamine (GalNAc).

11. Le composé oligomère de l'une quelconque des revendications précédentes, dans lequel le composé inhibe l'expression d'un acide nucléique cible d'au moins environ 70%, 75%, 80%, 85%, 90%, 95%, 98% ou 99%.

12. Composition pharmaceutique comprenant le composé oligomère de l'une quelconque des revendications précédentes, seul ou en combinaison avec un support ou un excipient pharmaceutiquement acceptable.

13. Procédé de fabrication d'un composé oligomère selon l'une quelconque des revendications précédentes, comprenant les étapes suivantes :
a. la synthèse d'un oligonucléotide de brin sens sur un support solide à l'aide d'une chimie phosphoramidite
b. la synthèse d'un oligonucléotide antisens sur un support solide à l'aide d'une chimie phosphoramidite et
c. réhybridation des deux oligonucléotides ainsi synthétisés
et obtenir ainsi le composé oligomère.
